(19) Europäisches Patentamt
European Patent Office
Office européen des brevets

(11) EP 3 981 796 A1

(12) EUROPEAN PATENT APPLICATION
published in accordance with Art. 153(4) EPC

(43) Date of publication:
13.04.2022 Bulletin 2022/15

(21) Application number: 20817959.8

(22) Date of filing: 03.06.2020

(51) International Patent Classification (IPC):
C08B 15/02 (2006.01)     A61Q 1/00 (2006.01)
A61Q 3/00 (2006.01)      A61Q 11/00 (2006.01)
A61Q 15/00 (2006.01)     A61Q 19/00 (2006.01)
A61Q 5/00 (2006.01)      A61K 8/06 (2006.01)
A61K 8/73 (2006.01)      A61K 8/81 (2006.01)
A61K 8/85 (2006.01)      C08B 37/08 (2006.01)

(52) Cooperative Patent Classification (CPC):
A61K 8/06; A61K 8/73; A61K 8/81; A61K 8/85;
A61Q 1/00; A61Q 3/00; A61Q 5/00; A61Q 11/00;
A61Q 15/00; A61Q 19/00; C08B 15/02

(86) International application number:
PCT/JP2020/021982

(87) International publication number:
WO 2020/246510 (10.12.2020 Gazette 2020/50)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 06.06.2019  JP 2019106122
06.09.2019  JP 2019162889

(71) Applicant: TOPPAN INC.
Tokyo 110-0016 (JP)

(72) Inventor: HAYASHI, Yumi
Tokyo 110-0016 (JP)

(74) Representative: Hoffmann Eitle
Patent- und Rechtsanwälte PartmbB
Arabellastraße 30
81925 München (DE)

(54) COMPOSITE PARTICLE AND METHOD FOR PRODUCING SAME, PERSONAL CARE PRODUCT, PARTICLES FOR PERSONAL CARE AND METHOD FOR PRODUCING SAME, PERSONAL CARE GOODS, AND COMPOSITION FOR PERSONAL CARE

(57) A composite particle (1) according to the first aspect of the present invention includes a core particle (2) having a pore (4) and fine fibers (3) inseparably bonded to a surface of the core particle (2). The fine fibers (3) are at least either cellulose nanofibers or chitin nanofibers.

FIG.1A

EP 3 981 796 A1

**Description**

[Technical Field]

The present invention relates to composite particles, a method of producing the composite particles, a personal care product containing the composite particles, personal care particles, a method of producing the personal care particles, a personal care article, and a personal care composition.

[Background Art]

Personal care products intended for skin care, makeup, hair care, oral care, nail care, and the like contain various functional materials. For example, makeup products such as foundations contain oil-based raw materials such as fats, oils, and waxes, functional materials such as ultraviolet absorbers, colorants, fungicides, antioxidants, preservatives, moisturizers, vitamins, amino acids, hormones, and natural extracts, microparticles for good skin compatibility, and the like. In skin care products, functional materials are added to a dispersion solvent containing water, alcohol, oil, and the like combined at an arbitrary ratio. These personal care products are used to satisfy various required characteristics such as moisturizing properties, color development, ultraviolet light blocking, antibacterial effect, and high smoothness and good skin compatibility in application to the skin or the like.

Examples of personal care products in practical use include personal care products composed of various microparticles and microcapsules. Microparticles are typically micro-sized particles made of various polymers such as polyethylene and polypropylene, and are widely used for personal care, and for example, used in foundations for a better feeling in use and a soft-focus effect, or used as a keratin removing agent in facial cleansers.

As microparticles used for foundations, the use of porous or hollow microparticles has been considered in order to allow the foundations to have a better feeling in use, have oil absorption properties, achieve impregnation with an active ingredient, and exhibit a high soft-focus effect.

Furthermore, attempts have been made to impart and exhibit further functionality of microparticles by forming a microcapsule structure in which the surface of a microparticle serving as a core substance is coated with a film. Specifically, by microencapsulating a core substance containing a functional material such as a pharmaceutical product, a perfume, an enzyme, a pigment, or a dye, it is possible to protect the functional material and control the release behavior. A functional material may be further added to the wall film that coats the core substance.

However, microparticles with a micro size have a large specific surface area, and thus the microparticles generally tend to aggregate, resulting in low dispersion stability.

Therefore, there has been a strong demand for new microparticles that have good dispersion stability, exhibit various functions such as moisturizing properties, a skin pore correction effect, and a soft-focus effect, have good skin compatibility, and are applicable to personal care products.

Meanwhile, in recent years, attempts have been actively made to utilize, as a new material, fine fibers obtained by finely grinding cellulose fibers in wood or chitin and/or chitosan constituting the shells of crustaceans such as crabs until at least one dimension of the structure has a nanometer-order size.

For example, Patent Literature 1 discloses repeated mechanical treatment using a blender or a grinder applied to wood cellulose provides fine cellulose fibers, that is, cellulose nanofibers (hereinafter also referred to as CNF). The CNF obtained by this method has been reported to have a minor axis diameter of 10 to 50 nm and a major axis diameter of 1 $\mu$m to 10 mm. The CNF has a strength 5 times or more that of steel, with 1/5 the weight of steel, and has a very large specific surface area of 250 $m^2$/g or more; thus, the CNF is expected to be used as a resin reinforcement filler or an adsorbent.

Furthermore, attempts have been actively made to produce CNF by chemically treating cellulose fibers in wood in advance to facilitate fine grinding, followed by fine grinding by low-energy mechanical treatment such as treatment using a household blender. The chemical treatment method is not particularly limited, but is preferably a method in which an anionic functional group is introduced into the cellulose fibers to facilitate fine grinding. When an anionic functional group is introduced into the cellulose fibers, a solvent is more likely to enter a cellulose microfibril structure due to the osmotic pressure effect, leading to a significant reduction in energy required to finely grind the cellulose raw material.

The method of introducing an anionic functional group is not particularly limited, and for example, Non Patent Literature 1 discloses a method in which the surface of fine fibers of cellulose is subjected to selective phosphoric acid esterification.

Patent Literature 2 discloses a method in which cellulose is carboxymethylated by reacting the cellulose with monochloroacetic acid or sodium monochloroacetate in a high-concentration alkaline aqueous solution. Alternatively, a carboxyl group may be introduced to the cellulose by directly reacting the cellulose with a carboxylic acid anhydride compound such as maleic acid or phthalic acid gasified in an autoclave.

A method has been reported in which 2,2,6,6-tetramethylpiperidinyl-1-oxy radical (TEMPO), which is a relatively stable N-oxyl compound, is used as a catalyst to selectively oxidize the surface of fine fibers of cellulose (see, for example, Patent Literature 3). An oxidation reaction using TEMPO as a catalyst (TEMPO oxidation reaction) enables an environmentally friendly chemical modification that proceeds in an aqueous system at normal temperature under normal pressure. When the TEMPO oxidation reaction is applied to cellulose in wood, the reaction does not proceed inside crystals of the cellulose, and an alcoholic primary carbon of a cellulose molecular chain on the crystal surface can be selectively converted into a carboxyl group.

Due to the osmotic pressure effect caused by ionization of carboxyl groups selectively introduced to the crystal surface by TEMPO oxidation, it is possible to obtain cellulose single nanofibers (hereinafter also referred to as CSNF, TEMPO-oxidized cellulose nanofibers, or TEMPO-oxidized CNF) which are cellulose microfibrils dispersed in a solvent. CSNF exhibits high dispersion stability derived from the carboxyl groups on the surface. CSNF derived from wood that is obtained from wood by a TEMPO oxidation reaction is a structure having a high aspect ratio with a minor axis diameter of approximately 3 nm and a major axis diameter of several tens of nanometers to several micrometers. CSNF water dispersion liquids and objects made of CSNF have been reported to have high transparency. Patent Literature 4 reports that a laminate film obtained by application and drying of a CSNF dispersion liquid has gas barrier properties.

Furthermore, the addition of further functionality to CNF or CSNF has been taken into consideration. For example, further functionality can be added using the carboxyl groups on the surface of CSNF. Patent Literature 5 discloses a composite in which metal nanoparticles are supported on CSNF (metal nanoparticle-supported CSNF) obtained by reducing and depositing a metal while metal ions are adsorbed on the carboxyl groups on the surface of CSNF. Patent Literature 5 discloses an example using metal nanoparticle-supported CSNF as a catalyst, and reports that catalytic activity is improved by enabling the metal nanoparticles having a large specific surface area to be dispersed and stabilized.

Patent Literature 6 discloses that chitin nanofibers are obtained by grinding chitin and/or chitosan collected from crab shells or the like into ultrafine fibers. Chitin nanofibers are a recyclable resource and have antibacterial properties and biodegradability, and are expected to be used as additives to food products or cosmetics and as reinforcing fibers such as films, and used for agricultural resources and medical care.

Chitin and/or chitosan molecules are closely bonded to each other by a strong hydrogen bond, and thus it is not easy to prepare completely separated nanofibers from chitin and/or chitosan. According to the chitin nanofibers and the method of producing the chitin nanofibers described in Patent Literature 6, a dispersion liquid containing separated chitin nanofibers can be obtained by a simple process.

Thus, various studies have been conducted to develop high-functional materials by adding new functionality to naturally occurring fine fibers such as cellulose nanofibers and chitin nanofibers.

For practical use of such fine fibers, there is a problem that a fine fiber dispersion liquid obtained from the fine fibers has a low solid concentration of approximately 0.1 to 5%. For example, transporting a dispersion of fine fibers means transporting a large amount of solvent, which causes considerably higher transportation costs, resulting in significant deterioration in business efficiency.

However, if the solvent of the fine fiber dispersion liquid is removed simply by heat drying or the like, the fine fibers become aggregated and keratinized or formed into a film, and this makes it difficult to effectively utilize the characteristic, i.e., the large specific surface area, of the fine fibers, thus making it difficult to stably exhibit the function. Furthermore, due to the low solid concentration of the fine fibers, the process of removing the solvent by drying requires a large amount of energy, which may cause deterioration in business efficiency.

Thus, handling fine fibers such as cellulose nanofibers and chitin nanofibers in the form of a dispersion liquid causes deterioration in business efficiency. Therefore, there has been a strong demand for, as composite particles including fine fibers bonded to their surface, composite particles that allow a new handling state effectively utilizing the characteristic, i.e., the large specific surface area, of the fine fibers and that are easily washed or separated from a solvent.

[Citation List]

[Patent Literature]

[PTL 1] JP 2010-216021 A
[PTL 2] WO 2014/088072 A
[PTL 3] JP 2008-001728 A
[PTL 4] WO 2013/042654 A
[PTL 5] WO 2010/095574 A
[PTL 6] JP 2010-180309 A

[Non Patent Literature]

[NPL 1] Noguchi Y, Homma I, Matsubara Y. Complete nanofibrillation of cellulose prepared by phosphorylation. Cellulose. 2017; 24: 1295.10.1007/s10570-017-1191-3

[Summary of the Invention]

[Technical Problem]

As described above, there has been a strong demand for new microparticles that have good skin compatibility and are applicable to personal care products; however, porous microparticles produced by the conventional method cannot maintain their shape and have a poor feeling in use. Furthermore, it is difficult to form porous microparticles having both oil absorbency and moisturizing properties by the conventional method.

A first object of the present invention is to provide, as composite particles including fine fibers bonded to their surface, composite particles that maintain their spherical shape even as porous particles or hollow particles having a high porosity, have good dispersion stability and good skin compatibility, and exhibit good oil absorbency and good moisturizing properties.

A second object of the present invention is to provide personal care particles that have good dispersion stability, exhibit various functions such as moisturizing properties and color developability, and have good skin compatibility.

[Solution to Problem]

In order to achieve the first object, the present invention provides the following first, second, and third aspects.

[First aspect]

A composite particle including a core particle having a pore, and fine fibers made of at least either cellulose nanofibers or chitin nanofibers, the fine fibers being inseparably bonded to a surface of the core particle.

[Second aspect]

A method of producing a composite particle, the method including: (1) fibrillating one or both of a cellulose raw material and a chitin/chitosan raw material in a solvent to obtain a dispersion liquid of fine fibers that are at least either cellulose nanofibers or chitin nanofibers; (2) dispersing a droplet containing a pore forming agent and a core material in the dispersion liquid, and coating a surface of the droplet with the fine fibers, the pore forming agent forming a pore when removed, the core material forming a core particle when solidified; (3) solidifying the core material in the droplet to obtain a dispersion liquid of a core particle that contains the pore forming agent and whose surface is coated with the fine fibers; and (4) collecting the core particle from the obtained dispersion liquid of the core particle, and removing the pore forming agent from the collected core particle to form a pore in the core particle.

[Third aspect]

A method of producing a composite particle, the method including: (11) fibrillating one or both of a cellulose raw material and a chitin/chitosan raw material in a solvent to obtain a dispersion liquid of fine fibers that are at least either cellulose nanofibers or chitin nanofibers; (12) producing a W/O emulsion liquid in which a droplet containing a pore forming agent that forms a pore when removed is dispersed in a core material that forms a core particle when solidified, dispersing a droplet containing the W/O emulsion liquid in the dispersion liquid of the fine fibers, and coating a surface of the droplet containing the W/O emulsion liquid with the fine fibers to produce a W/O/W emulsion liquid; (13) solidifying the core material in a droplet of the W/O/W emulsion liquid to obtain a dispersion liquid of a core particle that contains the droplet composed of the pore forming agent and whose surface is coated with the fine fibers; and (14) collecting the core particle from the obtained dispersion liquid of the core particle, and removing the pore forming agent from the collected core particle to form a pore in the core particle.

The composite particle of the first aspect can be contained in a personal care product.

In order to achieve the second object, the present invention provides the following fourth, fifth, and sixth aspects.

[Fourth aspect]

A personal care particle including a core particle made of a material containing a polymer, fine fibers made of at least either cellulose nanofibers or chitin nanofibers, the fine fibers being inseparably bonded to a surface of the core particle, and a functional material that is supported by the fine fibers and exhibits a personal care function.

[Fifth aspect]

A method of producing a personal care particle, the method including: (21) fibrillating one or both of a cellulose raw material and a chitin/chitosan raw material in a solvent to obtain a dispersion liquid of fine fibers that are at least either cellulose nanofibers or chitin nanofibers; (22) causing the fine fibers in the dispersion liquid of the fine fibers to support a functional material that exhibits a personal care function, thereby obtaining a dispersion liquid of the fine fibers supporting the functional material; (23) dispersing a droplet containing a core material in the dispersion liquid of the fine fibers supporting the functional material, and coating a surface of the droplet with the fine fibers supporting the functional material; and (24) solidifying the core material in the droplet coated with the fine fibers supporting the functional material.

[Sixth aspect]

A method of producing a personal care particle, the method including: (31) fibrillating one or both of a cellulose raw material and a chitin/chitosan raw material in a solvent to obtain a dispersion liquid of fine fibers that are at least either cellulose nanofibers or chitin nanofibers; (32) dispersing a droplet containing a core material in the dispersion liquid of the fine fibers, and coating a surface of the droplet with the fine fibers; (33) solidifying the core material in the droplet to obtain a dispersion liquid of a particle in which a surface of a core particle is coated with the fine fibers; and (34) causing, in the dispersion liquid of the particle, the fine fibers on the surface of the particle coated with the fine fibers to support a functional material that exhibits a personal care function.
The personal care particle of the fourth aspect can be contained in a personal care article and a personal care composition.

[Advantageous Effects of the Invention]

According to the composite particle of the first aspect of the present invention, as a composite particle including fine fibers bonded to its surface, the composite particle is expected to maintain its spherical shape even as a porous particle or a hollow particle having a high porosity, have good dispersion stability and good skin compatibility, and exhibit good oil absorbency and good moisturizing properties.
By the production method of the second and third aspects of the present invention, the composite particle of the first aspect of the present invention is produced.
According to the personal care particle of the fourth aspect of the present invention, since the core particle is coated with the fine fibers that have high safety, high hydrophilicity, and a large specific surface area, the personal care particle is expected to have high smoothness, good skin compatibility, and good moisturizing properties.
Furthermore, the fourth aspect of the present invention provides a personal care particle that due to a functional material being supported by fine fibers having a large specific surface area, maintains dispersion stability of the functional material, and effectively exhibits a function of the functional material such as ultraviolet light blocking (absorption and/or scattering), color development, or an antibacterial effect.
Furthermore, since cellulose nanofibers and chitin nanofibers are respectively made of cellulose and chitin/chitosan which are biodegradable polymers, the use of a biodegradable polymer as a polymer contained in a core particle provides a personal care particle that reduces the environmental load.
By the production method of the fifth and sixth aspects of the present invention, the personal care particle of the fourth aspect of the present invention is produced.

[Brief Description of the Drawings]

    Fig. 1A is a schematic diagram showing a composite particle of a first embodiment (one embodiment of the first aspect) of the present invention.
    Fig. 1B is a schematic diagram showing a composite particle of the first embodiment (one embodiment of the first aspect) of the present invention.
    Fig. 1C is a schematic diagram showing a composite particle of the first embodiment (one embodiment of the first aspect) of the present invention.
    Fig. 2 is a diagram showing a method of producing the composite particles of the first embodiment (production method I).

Fig. 3 is a diagram showing a method of producing the composite particles of the first embodiment (production method II).

Fig. 4 is a schematic diagram showing a personal care particle of a second embodiment (one embodiment of the fourth aspect) of the present invention.

Fig. 5 is a diagram showing a method of producing the personal care particles of the second embodiment (production method III).

Fig. 6 is a diagram showing a method of producing the personal care particles of the second embodiment (production method IV).

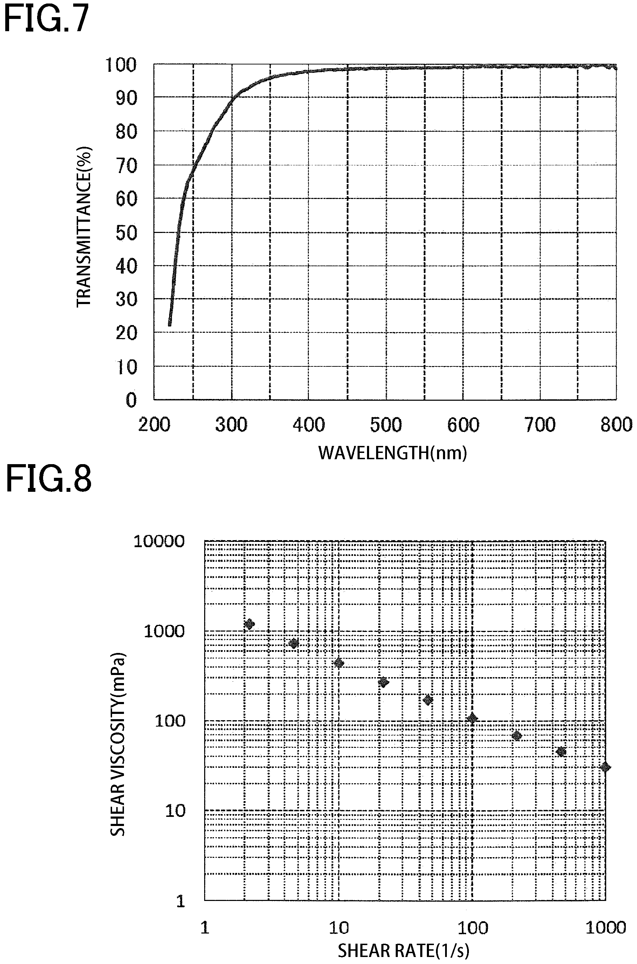

Fig. 7 is a graph showing the results of transmission spectrum measurement of a cellulose nanofiber water dispersion liquid obtained in Example 1.

Fig. 8 is a graph showing the results of steady-state viscoelasticity measurement using a rheometer, of the cellulose nanofiber water dispersion liquid obtained in Example 1.

Fig. 9 shows microscope images (SEM images) of personal care particles supporting silver fine particles obtained in Example 21.

Fig. 10 shows microscope images (SEM images) of personal care particles supporting flat plate-like silver fine particles obtained in Example 22.

Fig. 11 shows microscope images (SEM images) of personal care particles supporting gold fine particles obtained in Example 29.

[Description of the Embodiments]

Embodiments of the present invention will be described below with reference to the drawings. In the drawings described below, the corresponding portions are given the same reference numerals, and redundant description is omitted as appropriate. The embodiments are merely examples of configurations for embodying the technical idea of the present invention, and do not specify the materials, shapes, structures, arrangements, dimensions, or the like of the components. The technical idea of the present invention may be modified in various manners within the technical scope defined by the claims.

[First embodiment]

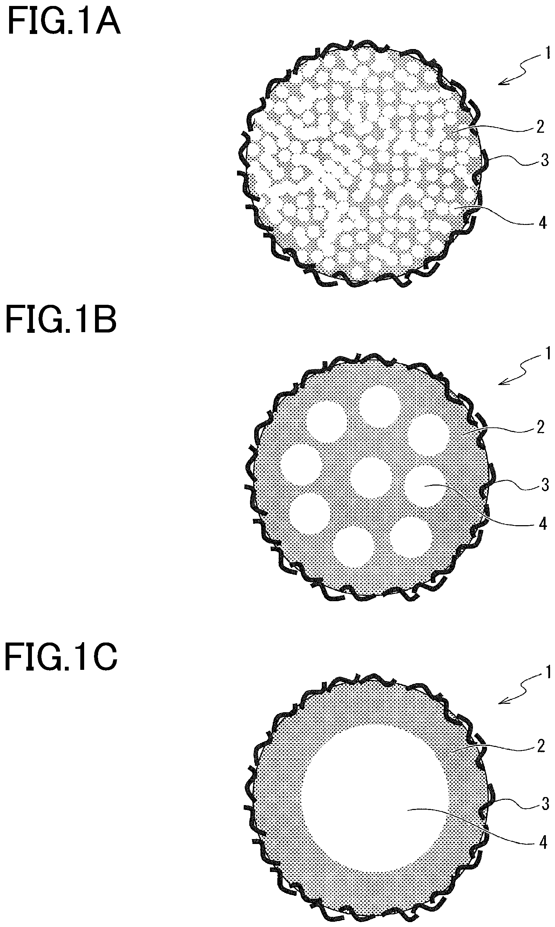

As shown in Figs. 1A, 1B, and 1C, a composite particle 1 of the present embodiment includes a core particle 2 having a pore 4, and fine fibers 3 made of at least either cellulose nanofibers or chitin nanofibers, the fine fibers 3 being inseparably bonded to a surface of the core particle 2. That is, the fine fibers 3 may be either cellulose nanofibers or chitin nanofibers, or may contain both cellulose nanofibers and chitin nanofibers.

In the composite particle 1 shown in Fig. 1A, the core particle 2 has a large number of small pores 4. That is, Fig. 1A shows the composite particle 1 in which the core particle 2 is a porous particle. The core particle 2 has a portion in which a pore 4 is independently present and a portion in which a plurality of pores 4 are connected to each other. The pores 4 that are present in an outer peripheral portion of the core particle 2 include pores that are open to the surface of the core particle 2 and pores that are not open to the surface of the core particle 2.

In the composite particle 1 shown in Fig. 1B, the core particle 2 has a plurality of relatively large pores 4. That is, Fig. 1B shows the composite particle 1 in which the core particle 2 is a multi-hollow particle.

In the composite particle 1 shown in Fig. 1C, the core particle 2 has a single large pore 4. That is, Fig. 1C shows the composite particle 1 in which the core particle 2 is a single-hollow particle.

Thus, the size, shape, and number of pores 4 of the core particle 2 are not particularly limited, and the core particle 2 may include a single pore 4 or a plurality of pores 4. The pore 4 may be independently present, or a plurality of pores 4 may be connected to each other, and the pore 4 may or may not open to the surface of the core particle 2.

In the present embodiment, the fine fibers 3 are formed as a coating layer that coats the surface of the core particle 2. That is, in the composite particle of the first aspect, the fine fibers are preferably formed as a coating layer that coats the surface of the core particle.

The method of producing the composite particles 1 is not particularly limited, and may be a known method, and a chemical preparation method or a physicochemical preparation method can be used.

Examples of the chemical preparation method include polymerization granulation methods in which particles are formed from a polymerizable monomer in a polymerization process (an emulsion polymerization method, a suspension polymerization method, a seed polymerization method, a radiation polymerization method, etc.).

Examples of the physicochemical preparation method include dispersion granulation methods in which particles are formed from a polymer solution in the form of microdroplets (a spray drying method, an in-liquid curing method, a solvent evaporation method, a phase separation method, a solvent dispersion cooling method, etc.).

For example, first, droplets containing a pore forming agent (a material that forms pores when removed in a later step) and a core material (core particle precursor, core precursor) that forms core particles when solidified are dispersed in a dispersion liquid of fine fibers to form an O/W Pickering emulsion.

Next, the core material in the droplets is solidified. This provides core particles that contain the pore forming agent and whose surface is coated with the fine fibers that are inseparably bonded thereto. Next, during or after solidification of the core material, the pore forming agent is removed. As a result, it is possible to obtain the composite particles 1 in which the fine fibers 3 are inseparably bonded to the surface of the core particles 2 having the pores 4.

In this method, the use of fine fibers makes it possible to form stable droplets without using an additive such as a surfactant, and obtain composite particles that maintain their spherical shape even with a high porosity, have high dispersibility, high smoothness, and good skin compatibility.

The O/W emulsion is also referred to as an oil-in-water emulsion, in which oil is dispersed as oil droplets (oil particles) in water as a continuous phase.

The core material is not particularly limited as long as the core material is a material that can form core particles when solidified. The core material may be, for example, a polymerizable monomer, a molten polymer, or a dissolved polymer. The method of solidifying the core material is not particularly limited, and the core material can be solidified by polymerizing a polymerizable monomer, solidifying a molten polymer, or removing a solvent from a dissolved polymer.

The pore forming agent may be a non-polymerizable solvent, a linear polymer, solid fine particles, or the like. Examples of the non-polymerizable solvent include hydrophobic solvents such as n-hexane, n-heptane, n-dodecane, and toluene, hydrophilic solvents such as ethanol and methanol, and water. Examples of the linear polymer include polymethyl methacrylate, polyethylene oxide, polypropylene oxide, and polymethylsiloxane. Examples of the solid fine particles include calcium carbonate.

The method of removing the pore forming agent is not particularly limited, and may be a method selected according to the material used as a pore forming agent, such as a method in which the pore forming agent is dried under normal pressure or reduced pressure at normal temperature or during heating, or a method in which the pore forming agent is dissolved in a good solvent such as toluene.

For example, when a non-polymerizable solvent is used as a pore forming agent, a core material is mixed and dissolved in the non-polymerizable solvent to obtain a solution, and droplets containing the solution (the core material and the non-polymerizable solvent) are dispersed in a dispersion liquid of fine fibers, and the surface of the droplets is coated with the fine fibers. Next, the core material in the droplets is solidified. The non-polymerizable solvent dissolved in the core material is removed when the non-polymerizable solvent leaves the droplets and enters the dispersion liquid during solidification of the core material. As a result, it is possible to obtain composite particles in which pores are formed in portions where the non-polymerizable solvent has been present.

Composite particles having pores can also be obtained by mixing an excessive amount of oil-soluble surfactant in a core material. When the core material contains an excessive amount of oil-soluble surfactant above the critical micelle concentration, micelles are formed. When droplets contain micelles, a hydrophilic solvent as a continuous phase is absorbed in the droplets and functions as a pore forming agent.

Furthermore, porous or hollow composite particles can be obtained by dispersing a hydrophilic solvent (pore forming agent) such as water in a core material using a stabilizer such as an oil-soluble surfactant or fine fibers to prepare a W/O emulsion in advance, dispersing the obtained W/O emulsion in a dispersion liquid of the fine fibers to prepare a W/O/W emulsion, and then solidifying the core material and removing the hydrophilic solvent such as water.

A W/O emulsion is also referred to as a water-in-oil emulsion, in which water is dispersed as water droplets (water particles) in oil as a continuous phase. A W/O/W emulsion is a double emulsion in which oil droplets (oil particles) containing dispersed water droplets (water particles) are dispersed in water, and is also referred to as a water-in-oil-in-water emulsion.

When a core material containing a linear polymer is used, after production of particles coated with fine fibers, the linear polymer can be dissolved by using a good solvent such as toluene.

Personal care products are products such as cosmetics and toiletries for maintenance of cleanliness of human skin, grooming, and beautification. Personal care products are, for example, products related to hair care, oral care, odor care, nail care, body care, skin care, and makeup. Examples of such products include toothpastes, perfumes, nail lacquers, skin lotions, milky lotions, cleansing agents, eye shadows, eyeliners, foundations, blushes, hair gels, hair sprays, shampoos, conditioners, hair tonics, lotions, creams, and soaps.

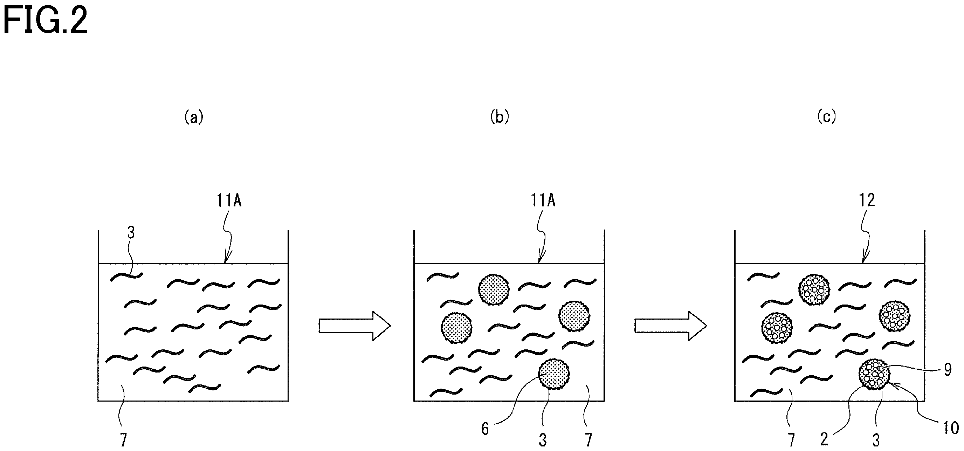

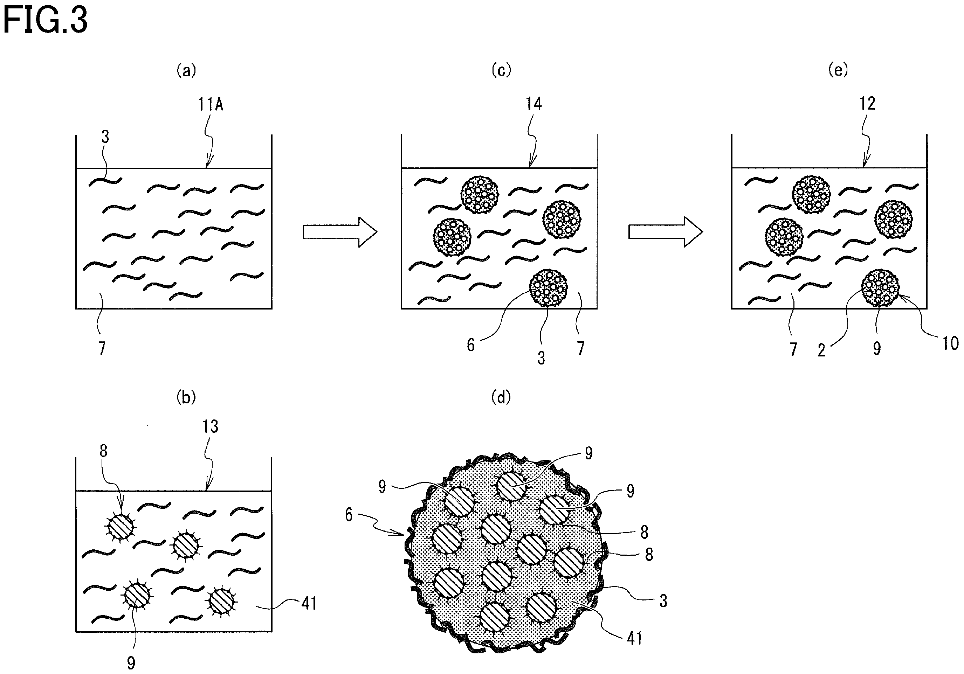

The composite particles 1 of the present embodiment can be produced by production methods shown in Figs. 2 and 3.

The production method shown in Fig. 2 corresponds to the production method of the second aspect (production method I), and includes a first step, a second-i step, a third-i step, and a fourth step.

As shown in Fig. 2 (a), the first step is a step of fibrillating a cellulose raw material or a chitin/chitosan raw material in a hydrophilic solvent 7 to obtain a dispersion liquid (a dispersion liquid in which the fine fibers 3 are dispersed in the hydrophilic solvent 7) 11A of the fine fibers 3 that are either cellulose nanofibers or chitin nanofibers.

The second-i step is a step of dispersing droplets 6 containing a pore forming agent and a core material in the dispersion liquid 11A of the fine fibers 3, and coating the surface of the droplets 6 with the fine fibers 3. In this step, as shown in Fig. 2 (b), an O/W Pickering emulsion is obtained in which the dispersion liquid 11A of the fine fibers 3 serves as a continuous phase and the droplets 6 containing the pore forming agent and the core material serve as a dispersed phase.

As shown in Fig. 2 (c), the third-i step is a step of solidifying the core material in the droplets 6 to obtain a dispersion liquid 12 of particles 10 in which the surface of the core particles 2 containing the pore forming agent (e.g., droplets 9) is coated with the fine fibers 3.

The fourth step is a step of collecting the particles 10 from the dispersion liquid 12 in the state shown in Fig. 2 (c), and then removing the pore forming agent (e.g., the droplets 9) contained in the solidified core particles 2. In this step, the composite particles (particles in which the surface of the core particles 2 having the pores 4 is coated with the fine fibers 3) 1 are obtained.

That is, in the production method I, the fine fibers 3 are adsorbed on the interface of the droplets 6 that contain the pore forming agent and the core material and are dispersed in the hydrophilic solvent 7, thereby stabilizing the O/W Pickering emulsion. Then, while the stabilized state is maintained, the core material in the droplets 6 of the emulsion is solidified to form core particles, and the pore forming agent (the non-polymerizable solvent contained in the droplets 6, the hydrophilic solvent 7 absorbed by the droplets 6 due to the effect of an excessive amount of oil-soluble surfactant contained in the droplets 6, and the like) is removed from the core particles to obtain the composite particles 1.

When the droplets 6 contain a hydrophobic non-polymerizable solvent, the non-polymerizable solvent is deposited as oil droplets in the core particles 2 during solidification of the core material; thus, by removing the oil droplets, the core particles 2 having the pores 4 can be obtained.

When the droplets 6 contain a large amount of oil-soluble surfactant above the critical micelle concentration, the hydrophilic solvent 7 is absorbed in the droplets 6 and functions as a pore forming agent. When the core material is solidified, the core particles 2 containing the hydrophilic solvent 7 are obtained, and by removing the hydrophilic solvent 7, the core particles 2 having the pores 4 can be obtained.

The production method shown in Fig. 3 corresponds to the production method of the third aspect (production method II), and includes the first step, a second-ii step, the third-i step, and the fourth step.

As shown in Fig. 3 (a), the first step is a step of fibrillating a cellulose raw material or a chitin/chitosan raw material in the hydrophilic solvent 7 to obtain the dispersion liquid 11A of the fine fibers 3 that are either cellulose nanofibers or chitin nanofibers.

The second-ii step is a step of dispersing a pore forming agent such as a hydrophilic solvent in a core material using a stabilizer to prepare a W/O emulsion liquid as hydrophilic droplets in advance, dispersing the emulsion liquid as hydrophobic droplets in the dispersion liquid 11A of the fine fibers 3, and coating the surface of the hydrophobic droplets with the fine fibers 3. Thus, in the second-ii step, a W/O/W emulsion liquid is obtained in which the droplets composed of the W/O emulsion liquid serve as a dispersed phase and the hydrophilic solvent 7 serves as a continuous phase.

In the second-ii step, first, as shown in Fig. 3 (b), a pore forming agent such as a hydrophilic solvent is dispersed in a core material (a polymerizable monomer, a dissolved polymer, or a molten polymer) 41 using a stabilizer 8 to produce a W/O emulsion liquid 13 of the droplets 9 composed of a hydrophilic pore forming agent.

Next, the W/O emulsion liquid 13 is added to the dispersion liquid 11A of the fine fibers 3, and as shown in Fig. 3 (c), the droplets 6 containing the W/O emulsion liquid are dispersed in the W/O emulsion liquid 13, and the surface of the droplets 6 containing the W/O emulsion liquid is coated with the fine fibers 3 to produce a W/O/W emulsion liquid 14. Fig. 3 (d) is an enlarged view of a droplet 6.

As shown in Fig. 3 (e), the third-i step is a step of solidifying the core material in the droplets 6 to obtain the dispersion liquid 12 of the particles 10 in which the surface of the core particles 2 is coated with the fine fibers 3. The droplets 9 composed of the pore forming agent are contained in the core particles 2.

The fourth step is a step of collecting the particles 10 from the dispersion liquid 12 in the state shown in Fig. 3 (e), and removing the pore forming agent (the hydrophilic solvent dispersed as the droplets 9 in the droplets 6) contained in the core particles 2 to form the pores 4 in the core particles 2. In this step, the composite particles (particles in which the surface of the core particles 2 having the pores 4 is coated with the fine fibers 3) 1 are obtained.

That is, in the production method II, the fine fibers 3 are adsorbed on the interface of "the droplets 6 composed of the liquid in which the droplets (droplets of the hydrophilic pore forming agent) 9 are dispersed in the core material 41" dispersed in the hydrophilic solvent 7, thereby stabilizing the W/O/W Pickering emulsion. Then, while the stabilized state is maintained, the core material 41 contained in the droplets 6 in the emulsion is solidified. Subsequently, free fine fibers 3 and the hydrophilic solvent 7 as a continuous phase are separated by filtration or the like from the dispersion liquid 12 of the particles 10 in which the surface of the core particles 2 is coated with the fine fibers 3, and then the hydrophilic solvent (pore forming agent) dispersed as the droplets 9 in the core particles 2 is removed to obtain the composite particles 1.

The term "inseparable" as used herein means that the core particles and the fine fibers are not separated from each other and the coating of the core particles with the fine fibers is maintained even after repetition of the operation of centrifuging a dispersion liquid containing the particles or composite particles coated with the fine fibers to remove the supernatant, redispersing the particles in a solvent, and collecting and washing the particles or composite particles coated with the fine fibers, or the operation of washing the particles or composite particles coated with the fine fibers with a solvent by filtration washing using a membrane filter. The coating state can be confirmed by observing the surface of the composite particles with a scanning electron microscope.

The mechanism of bonding between the fine fibers and the core particles during production of the particles or composite particles coated with the fine fibers by the above production methods is not clear, but the following presumption can be made. First, particles or composite particles coated with fine fibers are produced using, as a template, an O/W emulsion stabilized by the fine fibers. Then, while the fine fibers are in contact with the core material in the droplets of the emulsion, the core material is solidified to form core particles. Thus, presumably, the fine fibers are physically fixed to the surface of the core particles, resulting in the core particles and the fine fibers being inseparable from each other.

Although not particularly limited, when particles or composite particles coated with fine fibers are produced using, as a template, an O/W emulsion stabilized by the fine fibers, due to the stabilized O/W emulsion, it is possible to obtain particles or composite particles coated with the fine fibers having a spherical shape derived from the O/W emulsion. Specifically, it is preferable to form a coating layer that is composed of the fine fibers and has a relatively uniform thickness on the surface of the core particles having a spherical shape.

In the composite particles of the embodiment, the surface of the core particles is coated with the fine fibers having high strength; thus, the composite particles can maintain the spherical shape even with a high porosity (pore ratio or hollow ratio), and achieve high light diffusing properties, smooth feeling in use, good moisturizing properties, and high dispersibility.

The particle size of the composite particles can be confirmed by observation with an optical microscope. The average particle size of the composite particles can be calculated, for example, by measuring the diameter of 100 randomly selected composite particles, and taking the average value of the diameter of the particles. The average particle size is not particularly limited, but is preferably 0.1 $\mu$m or more and 1000 $\mu$m or less.

From the viewpoint of dispersion stability, the fine fibers are preferably formed as a coating layer on the surface of the core particles. The coating layer preferably coats the entire surface of the core particles, but may not necessarily coat the entire surface.

The thickness of the coating layer composed of the fine fibers is not particularly limited, but is preferably 3 nm or more and 1000 nm or less.

The average thickness of the coating layer can be calculated by cutting the composite particles fixed with an embedding resin using a microtome, observing the composite particles with a scanning electron microscope, measuring the thickness of the coating layer in the cross-sectional image of the composite particles at 100 random locations in the image, and taking the average value of the thickness at the 100 locations.

The composite particles are preferably uniformly coated with a coating layer having a relatively uniform thickness. When the particles are coated with a coating layer having a uniform thickness, the particles have high dispersion stability. Specifically, the coefficient of variation of the thickness of the coating layer is preferably 0.5 or less, and more preferably 0.4 or less.

The fine fibers constituting the composite particles of the present embodiment are cellulose nanofibers and/or chitin nanofibers.

Cellulose nanofibers are fibers that are made of cellulose or a cellulose derivative and have a number average minor axis diameter of 1 nm or more and 1000 nm or less. Chitin nanofibers are fibers that are made of chitin and/or chitosan or a chitin derivative and/or a chitosan derivative and have a number average minor axis diameter of 1 nm or more and 1000 nm or less.

Cellulose nanofibers (CNF) are fine fibers that can be obtained by grinding a cellulose raw material obtained from wood or the like into ultrafine fibers, and CNF is safe and biodegradable.

Chitin nanofibers (chitin NF) are fine fibers that can be obtained by grinding a chitin and/or chitosan (hereinafter referred to as chitin/chitosan) raw material collected from crab shells or the like into ultrafine fibers, and chitin NF is safe and biodegradable and has antibacterial properties. In particular, chitin nanofibers produced by the method described in Patent Literature 6 are made of chitin and/or chitosan not chemically modified, and require no safety confirmation, thus significantly facilitating the use of the chitin nanofibers particularly for applications requiring incorporation into the body, such as food products, medical care products, drugs, and personal care products.

The fine fibers preferably have an ionic functional group on the crystal surface. When the fine fibers have an ionic functional group, aggregation of the composite particles can be prevented, thus achieving personal care articles having good skin compatibility. Furthermore, the ionic functional group can be used to support a functional material on the surface of the composite particles. For example, the fine fibers having an anionic functional group can easily support a cationic functional material, and the fine fibers having a cationic functional group can easily support an anionic functional material.

The type of ionic functional group is not particularly limited, but is preferably an anionic functional group. In the case where the fine fibers have an anionic functional group, for example, when metal fine particles are supported by the fine fibers, by coordinating metal ions to the anionic functional group of the fine fibers, and reducing and depositing the metal ions, the metal fine particles can be efficiently supported by the fine fibers. Furthermore, the fine fibers function as a dispersion stabilizer, thus enabling control of the shape and particle size of metal fine particles to be generated. Furthermore, it is possible to prevent removal of the metal fine particles from the fine fibers in the composite particles.

In general, when metal fine particles are dried, the metal fine particles are aggregated; thus, optical characteristics due to localized surface plasmon resonance (LSPR) (described later) cannot be exhibited. However, when the composite particles are in the form of a dry powder, the metal fine particles do not become aggregated; thus, the composite particles as a dry powder can exhibit the optical characteristics. When the metal fine particles are silver fine particles, the metal fine particles can impart antibacterial properties.

Examples of the anionic functional group include, but are not particularly limited to, a carboxyl group, a phosphate group, and a sulfo group. Of these, a carboxyl group and a phosphate group are preferable, and a carboxyl group is more preferable in terms of ease of selective introduction to the crystal surface of cellulose.

The content of ionic functional group is preferably 0.1 mmol or more and 5.0 mmol or less per 1 g of fine fiber raw material and/or fine fibers. If the content of ionic functional group is less than 0.1 mmol, the composite particles 1 may have poor dispersion stability, and if the content of ionic functional group exceeds 5.0 mmol, it may be difficult to stably produce the composite particles 1.

The fine fibers preferably have a fiber form derived from a microfibril structure. Specifically, the fine fibers preferably have a fibrous form, and have a number average minor axis diameter of 1 nm or more and 1000 nm or less, and a number average major axis diameter of 50 nm or more. The number average major axis diameter is preferably 5 times or more the number average minor axis diameter. The degree of crystallinity of the fine fibers is preferably 50% or more. The crystal structure of the cellulose nanofibers is preferably cellulose type I. The crystal structure of the chitin nanofibers may be alpha chitin or beta chitin, but is preferably alpha chitin.

As shown in Figs. 1A to 1C, the core particles 2 constituting the composite particles 1 of the present embodiment have the pores 4. The shape, size, and number of pores 4 can be controlled by adjusting the type and amount of pore forming agent to be used. The core particles 2 of the composite particles 1 may be porous particles or hollow particles.

Porous particles are particles having a very large number of pores. When the core particles constituting the composite particles are porous particles, the core particles have a large specific surface area; thus, personal care products containing the composite particles can absorb oil on the skin and provide a dry touch.

When the core particles of the composite particles are porous particles, the pore diameter (the diameter of the pores) is not particularly limited, but is preferably 2 nm or more and 30 $\mu$m or less. In order to obtain the porous composite particles 1 having oil absorption properties, the pore diameter is preferably 2 nm or more and 50 nm or less. If the pore diameter is less than 2 nm, the amount of water absorption and the amount of oil absorption of the composite particles 1 are insufficient. If the pore diameter exceeds 50 nm, the composite particles 1 are less likely to hold a functional component.

The specific surface area of the composite particles is preferably 2 $m^2/g$ or more and 1000 $m^2/g$ or less, more preferably 3 $m^2/g$ or more and 400 $m^2/g$ or less, and still more preferably 5 $m^2/g$ or more and 300 $m^2/g$ or less. If the specific surface area is less than 2 $m^2/g$, the composite particles cannot exhibit sufficient water absorbency or sufficient oil absorbency. If the specific surface area exceeds 1000 $m^2/g$, the composite particles cannot maintain the spherical shape, thus failing to achieve a smooth feeling in use.

The pore diameter and the specific surface area can be measured by a gas adsorption method. For example, the measurement can be performed by the BET method (nitrogen adsorption method) in accordance with JIS Z 8830. The BET nitrogen adsorption isotherm of a dry powder of the composite particles 1 is measured by using an automatic specific surface area/pore distribution measuring device, and the specific surface area is calculated from the amount of nitrogen adsorption by the BET multipoint method. The pore diameter (average pore diameter) and the pore volume are calculated by the BJH method.

The amount of oil absorption of the composite particles of the present embodiment is preferably 30 ml/100 g or more and 800 ml/100 g or less, more preferably 50 ml/100 g or more and 500 ml/100 g or less, and still more preferably 70 ml/100 g or more and 300 ml/100 g or less. If the amount of oil absorption is less than 30 ml, powder cosmetics such as powder foundations containing the composite particles may not allow the makeup to last long enough. On the other hand, if the amount of oil absorption exceeds 800 ml/100 g, the composite particles may absorb other components.

The amount of water absorption of the composite particles of the present embodiment is preferably 30 ml/100 g or more and 800 ml/100 g or less, more preferably 50 ml/100 g or more and 500 ml/100 g or less, and still more preferably 70 ml/100 g or more and 300 ml/100 g or less. If the amount of water absorption is less than 30 ml/100 g, foundations or the like containing the composite particles are likely to not sufficiently absorb sweat. If the amount of water absorption exceeds 800 ml/100 g, the composite particles absorb moisture in the surroundings, resulting in a poor moisturizing effect.

The amount of oil absorption can be evaluated by the method in accordance with JIS K 5101-13-1. A powder of the composite particles 1 is placed on a measuring plate, and 4 or 5 droplets at a time of purified linseed oil are gradually added to the powder through a burette. Each time the purified linseed oil is added, the purified linseed oil is kneaded into the sample with a palette knife. This action is repeated, and the purified linseed oil is continuously dropped until a lump of purified linseed oil and sample is formed. After that, a single droplet at a time of purified linseed oil is added, and kneading is repeated so that the purified linseed oil is completely mixed with the sample. The process is ended when the paste has a smooth consistency. The amount of water absorption can be evaluated by the above method using pure water instead of the linseed oil.

Hollow particles are particles having a closed cavity. In the case where the core particles constituting the composite particles are hollow particles, the composite particles can have a high soft-focus effect regardless of the refractive index of the surroundings, and even when the composite particles are dispersed in an oil phase such as a liquid foundation, no oil component enters the composite particles; thus, the composite particles can maintain the high soft-focus effect.

The number of pores (voids) of the hollow particles is not particularly limited, and the hollow particles may be single-hollow particles having a single pore, or may be multi-hollow particles having a large number of pores. The porosity of the composite particles as hollow particles is not particularly limited, but is preferably 10% or more and 90% or less, more preferably 20% or more and 80% or less, and still more preferably 40% or more and 60% or less. If the porosity is less than 10%, it is difficult for the composite particles to exhibit a sufficient soft-focus effect. On the other hand, if the porosity exceeds 90%, it is difficult to obtain the composite particles 1 having a spherical shape.

The porosity can be calculated by observing the composite particles with a transmission electron microscope. A dry powder of the composite particles 1 is observed using a transmission electron microscope, and the average particle size and the average inner diameter of 100 or more particles are calculated. The average porosity can be obtained by the formula "(average inner diameter)$^3$/(average particle size)$^3 \times 100$".

When a non-polymerizable solvent having high hydrophobicity is used as a pore forming agent, the non-polymerizable solvent is moved toward the center during solidification of the core material, and thus hollow composite particles are more likely to be obtained. On the other hand, when a non-polymerizable solvent having relatively high hydrophilicity is used as a pore forming agent, porous composite particles are more likely to be obtained.

The composite particles of the present embodiment preferably have a spherical shape, and particularly preferably have a perfectly spherical shape. When the composite particles used for cosmetics such as foundations have a perfectly spherical shape, the cosmetics can have a smooth feeling in use. The sphericity index can be evaluated from the circularity obtained by using an image analysis particle size distribution analyzer. The circularity is preferably 0.6 or more, more preferably 0.7 or more, and still more preferably 0.9 or more. If the circularity is less than 0.6, it is difficult for the composite particles to achieve a smooth feeling in use. The average circularity can be calculated as the average value of the circularity of 1000 or more particles measured by using an image analysis particle size distribution analyzer. The circularity can be calculated by the formula "circularity = $4 \pi S/L^2$, where S represents the area of a composite particle in the image, and L represents the circumference of the composite particle in the image.

Since the composite particles of the present embodiment have pores, the composite particles exhibit good light diffusing properties. The light diffusing properties can be evaluated according to a light diffusion index measured by the following method.

A glass plate on which a dry powder is placed is inserted in a variable angle photometer GC5000 manufactured by Nippon Denshoku Industries Co., Ltd., and a spectral reflectance R1 and a spectral reflectance R2 are measured at a wavelength of 400 nm to 700 nm. The spectral reflectance R1 is a spectral reflectance when incident light at an angle of -45° is measured at an angle of 0°, and the spectral reflectance R2 is a spectral reflectance when incident light at an angle of -45° is measured at an angle of 45°. From the measurement results, the ratio of R1 to R2 at a wavelength of 600 nm is calculated as a light diffusion index.

The light diffusion index of the composite particles is preferably 0.6 or more, more preferably 0.7 or more, and still more preferably 0.9 or more. If the light diffusion index is less than 0.6, foundations or the like containing the composite particles may have an insufficient skin pore correction effect and soft-focus effect.

The core particles constituting the composite particles of the present embodiment preferably contain an organic compound that is solid at 25°C. Examples of such organic compounds include paraffins, polymers, fats, and oils.

The core particles are preferably made of a material containing one or more types of polymers. The polymer may be a known polymer, and may be a polymer obtained by polymerizing a polymerizable monomer by a known method.

Examples of the polymer include acrylic polymers, epoxy polymers, polyester polymers, amino polymers, silicone polymers, fluorine polymers, and urethane/isocyanate polymers.

Although not particularly limited, the polymer is preferably a biodegradable polymer. A biodegradable polymer refers to a polymer that is decomposed and eliminated in the global environment such as soil or seawater, or/and a polymer that is decomposed and eliminated in vivo. In general, polymers are decomposed in soil or seawater by enzymes of microorganisms, whereas polymers are decomposed in vivo by physicochemical hydrolysis without the need for enzymes. The decomposition of a polymer indicates that the polymer loses its morphology due to its low molecular weight or water solubility. The decomposition of a polymer is not particularly limited, but occurs by hydrolysis of the main chain, side chains, or crosslinking points, or oxidative decomposition of the main chain.

Biodegradable polymers include naturally occurring natural polymers and synthetic polymers.

Examples of natural polymers include polysaccharides produced by plants (e.g., cellulose, starch, and alginic acid), polysaccharides produced by animals (e.g., chitin, chitosan, and hyaluronic acid), proteins (e.g., collagen, gelatin, and albumin), and polyesters produced by microorganisms (poly(3-hydroxyalkanoate)), and polysaccharides (e.g., hyaluronic acid).

Examples of synthetic polymers include aliphatic polyesters, polyols, and polycarbonates.

Examples of fatty acid polyesters include glycol/dicarboxylic acid-polycondensed polyesters (polyethylene succinate, polybutylene succinate, etc.), polylactides (polyglycolic acid, polylactic acid, etc.), polylactones ($\beta$-caprolactone, $\varepsilon$-caprolactone, etc.), and others (polybutylene terephthalate, adipate, etc.).

Examples of polyols include polyvinyl alcohol.

Examples of polycarbonates include polyester carbonate.

Examples of other biodegradable synthetic polymers include polyacid anhydrides, polycyanoacrylates, polyorthoesters, and polyphosphazenes.

The material for forming the core particles may include an additional component other than the polymer.

Examples of the additional component include colorants, oil absorbing agents, light shielding agents (ultraviolet absorbers, ultraviolet scattering agents, etc.), antibacterial agents, antioxidants, antiperspirants, antifoaming agents, antistatic agents, binding agents, bleaching agents, chelating agents, deodorizing components, aromatics, perfumes, dandruff preventing active substances, skin softeners, insect repellents, preservatives, natural extracts, beauty components, pH adjusting agents, vitamins, amino acids, hormones, oil-based raw materials such as fats, oils, and waxes, surfactants, and inorganic particles (titanium oxide, silica, clay, etc.).

The additional component may be in the form of a solid, gas, or liquid. The content ratio of the additional component in the composite particles is not particularly limited, but is preferably in a range in which the composite particles can stably maintain their shape. The content ratio of the additional component is preferably 0.001 parts by mass or more and 80 parts by mass or less with respect to 100 parts by mass of the composite particles.

The composite particles of the present embodiment preferably contain a functional component in the pores. The composite particles containing a functional component can exhibit a function of the functional component. Examples of the method of causing the composite particles to contain a functional component include a method of impregnating the composite particles with a functional component, and a method of causing the composite particles to contain a functional component in the process of producing the composite particles.

The functional component is, for example, a substance that functions by affecting organisms such as animals, plants, and fungi. Examples of the functional component include, but are not particularly limited to, antifungal agents, perfumes, fertilizers (biological fertilizers, chemical fertilizers, organic fertilizers, etc.), pH adjusting agents, agricultural chemicals (insecticides, fungicides, herbicides, etc.), plant activators, plant life-prolonging agents, pest and animal repellents, soil penetrants, nutritional components (minerals, etc.), plant hormones, inorganic particles (titanium oxide, silica, clay, etc.), and antibacterial components.

The composite particles 1 of the present embodiment preferably contain, in the pores 4, particularly a functional component that exhibits a function as a personal care product. Examples of the functional component include oil-based raw materials such as fats, oils, and waxes, colorants, oil absorbing agents, light shielding agents (ultraviolet absorbers, ultraviolet scattering agents, etc.), antibacterial agents, antioxidants, antiperspirants, peeling agents, sebum inhibitors, blood circulation promoting components, skin-whitening components, aging care components, anti-inflammatory components, cooling sensation components, warming sensation components, deodorizing components, antifoaming agents, antistatic agents, binding agents, bleaching agents, chelating agents, aromatics, perfumes, dandruff preventing active substances, skin softeners, insect repellents, preservatives, vitamins, amino acids, hormones, and natural extracts. Of these, the composite particles 1 preferably contain at least one of ultraviolet absorbers, ultraviolet scattering agents, perfumes, deodorizing components, peeling agents, aging care components, skin-whitening components, anti-inflammatory components, and colorants.

Examples of ultraviolet absorbers include homomenthyl salicylate, 2-cyano-3,3-diphenylprop-2-enoic acid 2-ethylhexyl ester, diparamethoxycinnamate-mono-2-ethylhexanoate glyceryl, para-aminobenzoic acid and its esters, and 4-tert-butyl-4'-methoxydibenzoylmethane.

Examples of ultraviolet scattering agents include titanium oxide and zinc oxide.

Perfumes include natural perfumes extracted from animals, plants, and the like, synthetic perfumes chemically synthesized, and mixed perfumes which are a mixture of a plurality of types of perfumes. Of these, examples of natural perfumes include perfumes derived from flowering plants such as jasmine, rose, carnation, lilac, cyclamen, lily of the valley, violet, lavender, and fragrant olive, perfumes derived from citrus fruits such as orange, lemon, and lime, perfumes derived from spices such as cinnamon and nutmeg, and perfumes derived from wood essential oils such as cypress essential oil, hiba essential oil, and cedar essential oil.

Examples of synthetic perfumes include hydrocarbons such as limonene, pinene, and camphene, alcohols such as linalool, geraniol, menthol, citronellol, and benzyl alcohol, aldehydes such as citral, citronellal, nonadienal, benzaldehyde, and cinnamic aldehyde, ketones such as acetophenone, methylacetophenone, methyl nonyl ketone, irone, and menthone, phenols such as methyl anisole, and eugenol, lactones such as decalactone, nonyllactone, and undecalactone, and esters such as linalyl acetate, geranyl acetate, benzyl acetate, terpinyl acetate, citronellyl acetate, methyl benzoate, ethyl benzoate, isoamyl benzoate, benzyl benzoate, methyl cinnamate, and ethyl cinnamate.

Examples of deodorizing components include diphenols such as catechol, 4-methylcatechol, 5-methylcatechol, resorcinol, 2-methylresorcinol, 5-methylresorcinol, and hydroquinone; biphenyldiols such as 4,4'-biphenyldiol and 3,4'-biphenyldiol; catechins such as catechin, epicatechin, epigallocatechin, and epicatechin gallate; plant-derived polyphenols, for example, extracted from catechol derivatives such as dopa, dopamine, chlorogenic acid, caffeic acid, para-coumaric acid, and tyrosine or extracted from plants; and deodorizing components derived from coffee, apple, grape, teas (green tea, roasted tea, black tea, oolong tea, mate tea, etc.), persimmon, soybean, cacao, rosemary, aloe, and the like.

Examples of peeling agents include glycolic acid, salicylic acid, and lactic acid.

Examples of aging care components include retinol palmitate, fullerene, acetyl hexapeptide-8, palmitoyl pentapeptide-4, ubiquinone, and platinum.

Examples of skin-whitening components include ascorbyl phosphate Mg, arbutin, placenta extract, and chamomile flower extract.

Examples of anti-inflammatory components include licorice root extract, dipotassium glycyrrhizinate, stearyl glycyrrhizinate, allantoin, and coix seed extract.

Examples of colorants include organic synthetic colorants (dyes, lakes, organic pigments), natural colorants, inorganic pigments, metal fine particles, and polymer powders. Of these, the composite particles 1 preferably contain organic synthetic colorants, natural colorants, inorganic pigments, or metal fine particles.

In the composite particles 1 of the present embodiment, when a functional material is supported by the fine fibers that coat the surface of the core particles, the functional material has high dispersion stability; thus, the composite particles 1 can effectively exhibit a function of the functional material. The state in which a functional material is "supported" by fine fibers indicates that the functional material is physically or chemically and reversibly or irreversibly bonded to or adsorbed on the fine fibers. The functional material can be supported by a known method.

The functional material and the fine fibers are preferably "inseparable" from each other. The state in which the fine fibers and the functional material are "inseparable" from each other indicates that the fine fibers and the functional material are not separated from each other even after repetition of the operation of centrifuging a dispersion liquid containing the composite particles to remove the supernatant, redispersing the composite particles in a solvent, and collecting and washing the composite particles, or the operation of repeatedly washing the composite particles with a solvent by filtration washing using a membrane filter.

A functional material is a material that exhibits its function in personal care products, and may be a known functional material. Examples of the functional material include oil-based raw materials such as fats, oils, and waxes, colorants, oil absorbing agents, light shielding agents (ultraviolet absorbers, ultraviolet scattering agents, etc.), antibacterial agents, antioxidants, antiperspirants, peeling agents, sebum inhibitors, blood circulation promoting components, skin-whitening components, aging care components, anti-inflammatory components, cooling sensation components, warming sensation components, deodorizing components, antifoaming agents, antistatic agents, binding agents, bleaching agents, chelating agents, aromatics, perfumes, dandruff preventing active substances, skin softeners, insect repellents, preservatives, vitamins, amino acids, hormones, and natural extracts. Of these, the composite particles 1 preferably contain at least one of ultraviolet absorbers, ultraviolet scattering agents, perfumes, deodorizing components, and colorants.

The method of causing the fine fibers to support a functional material is not particularly limited, and for example, composite particles supporting a functional material can be produced by the following method. First, a functional material is supported by fine fibers, and then the obtained fine fibers supporting the functional material are used to prepare an O/W Pickering emulsion having droplets containing a non-polymerizable solvent or a surfactant and a core material. Next, the core material in the droplets of the emulsion is solidified, and then filtration washing is performed and the pore forming agent is removed.

Alternatively, composite particles supporting a functional material can be produced by the following method. First, fine fibers are used to produce an O/W Pickering emulsion having droplets containing a non-polymerizable solvent or a surfactant and a core material. Next, the core material in the droplets of the emulsion is solidified, and then filtration washing is performed and the pore forming agent is removed to produce composite particles. Subsequently, a functional material is supported by the fine fibers on the surface of the composite particles.

The method of causing the fine fibers to support a functional material is not particularly limited, and for example, a commercially available or previously produced functional material may be added and mixed in a dispersion liquid of the fine fibers or the composite particles to attach the functional material to the fine fibers.

It is preferable to produce a functional material in a dispersion liquid of the fine fibers or the composite particles. When a functional material is produced in a dispersion liquid of the fine fibers or the composite particles, the functional material is stably supported by the fine fibers, and thus aggregation of the functional material can be prevented.

<Method of producing composite particles>

As described above, the composite particles 1 of the embodiment can be produced by the methods shown in Figs. 2 and 3 (production method I and production method II).

The composite particles 1 obtained by the production method I and the production method II are obtained as a dry solid, and can be redispersed to form a dispersion. The particles 10 or the composite particles 1 coated with the fine fibers can be collected by filtration, centrifugation, or the like. When the collected particles are heat dried in an oven or the like or dried in vacuum, the particles can be obtained as a dry solid. In this case, the obtained dry solid of the composite particles 1 is not in the form of a film or an aggregate, and is obtained as a fine and smooth powder.

The reason for this is not clear, but it is known that when the solvent is removed from the dispersion liquid of the fine fibers 3, the fine fibers 3 are generally strongly aggregated or formed into a film. On the other hand, in the case of the dispersion liquid containing the particles 10 or the composite particles 1 coated with the fine fibers, the fine fibers 3 are fixed to the surface of the obtained spherical particles 10 or composite particles 1; thus, even when the hydrophilic solvent 7 is removed, the fine fibers 3 do not become aggregated and the particles 10 or the composite particles 1 coated with the fine fibers are only in point contact with each other. This is presumably the reason why the dry solid is obtained as a fine powder.

The particles 10 or the composite particles 1 coated with the fine fibers are not aggregated, and thus achieve powder cosmetics such as foundations that have smoothness and good skin compatibility. Furthermore, porous and hollow particles can achieve a lighter texture than solid particles, and have a good soft-focus effect due to their high light diffusing properties. A functional component can be contained in pores of the porous particles or hollows of the hollow particles, and when the functional material is supported by the fine fibers 3, aggregation of the functional material can be prevented, thus achieving uniform powder cosmetics.

The dry powder of the composite particles 1 can be a dry solid having advantages such as containing almost no solvent such as the hydrophilic solvent 7 and being redispersible in a solvent. Specifically, the dry powder can have a solid content ratio of 80% or more, 90% or more, or 95% or more. Since the solvent can be almost completely removed, advantageous effects are obtained from the viewpoint of reduction of transportation costs, prevention of decay, improvement of the addition ratio, and improvement of the kneading efficiency with resin.

Due to the high hydrophilicity of the fine fibers 3 on the surface of the composite particles 1, the dry powder of the composite particles 1 has an advantage such as absorbing moisture such as sweat or exhibiting high moisturizing properties.

In addition, the composite particles 1 obtained as a dry powder are easily redispersed in a solvent again, and even after redispersion, the composite particles 1 exhibit dispersion stability derived from the fine fibers 3 bonded to the surface of the composite particles 1. Thus, the dry powder of the composite particles 1 can be contained in beauty essences, milky lotions, creams, and the like.

Each step will be described below in detail.

As described above, the production method I includes the first step, the second-i step, the third-i step, and the fourth step, and the production method II includes the first step, the second-ii step, the third-i step, and the fourth step.

(First step)

The first step is a step of fibrillating a fine fiber raw material in a solvent to obtain a dispersion liquid of fine fibers. The fine fiber raw material is a cellulose raw material which is a raw material of cellulose nanofibers and/or a chitin/chitosan raw material which is a raw material of chitin nanofibers.

First, various fine fiber raw materials are dispersed in a solvent to prepare a suspension. The fine fiber raw material is preferably contained in the suspension at a concentration of 0.1% or more and less than 10%. If the concentration of the fine fiber raw material is less than 0.1%, productivity is deteriorated due to there being an excessive amount of solvent, which is not preferable. If the concentration of the fine fiber raw material is 10% or more, the suspension is rapidly thickened due to fibrillation of the fine fiber raw material, and this makes it difficult to perform uniform fibrillation treatment, which is not preferable.

The solvent used to prepare the suspension is preferably a hydrophilic solvent. The hydrophilic solvent is not particularly limited, but is preferably, water; an alcohol such as methanol, ethanol, or isopropanol; a cyclic ether such as tetrahydrofuran, or a mixture of these. The suspension preferably contains 50% or more water. If the ratio of water in the suspension is 50% or less, in the step of fibrillating a fine fiber raw material in a solvent to obtain a fine fiber dispersion liquid (described later), dispersion of the fine fibers is inhibited. A solvent other than water contained in the suspension is preferably any of the hydrophilic solvents described above.

If necessary, the pH of the suspension may be adjusted in order to improve the dispersibility of the fine fiber raw material and fine fibers to be generated. Examples of an alkaline aqueous solution used for pH adjustment include organic alkali such as a sodium hydroxide aqueous solution, a lithium hydroxide aqueous solution, a potassium hydroxide aqueous solution, an ammonia aqueous solution, a tetramethylammonium hydroxide aqueous solution, a tetraethylammonium hydroxide aqueous solution, a tetrabutylammonium hydroxide aqueous solution, and a benzyltrimethylammonium hydroxide aqueous solution. A sodium hydroxide aqueous solution is preferable in terms of cost and the like.

Next, the suspension is subject to physical fibrillation treatment to finely grind the fine fiber raw material. The physical fibrillation treatment is not particularly limited, and may be, for example, mechanical treatment using a high-pressure homogenizer, an ultrahigh-pressure homogenizer, a ball mill, a roll mill, a cutter mill, a planetary mill, a jet mill, an attritor, a grinder, a blender, a homomixer, an ultrasonic homogenizer, a nanogenizer, or aqueous counter collision. By the physical fibrillation treatment, cellulose in the suspension containing the cellulose raw material and the hydrophilic solvent 7 as a solvent is finely ground to form a dispersion liquid of the cellulose that is finely ground until at least one dimension of the structure has a nanometer-order size. The number average minor axis diameter and the number average major axis diameter of the resulting fine fibers 3 can be adjusted by changing the treatment time and the number of times of the physical fibrillation treatment.

In this manner, a dispersion of the fine fibers (fine fiber dispersion liquid) is obtained in which the fine fibers are finely ground until at least one dimension of the structure has a nanometer-order size. The obtained dispersion can be used as an O/W emulsion stabilizer (described later) as it is or after dilution, concentration, or the like.

The dispersion of the fine fibers may contain, if necessary, an additional component other than cellulose and the component used for pH adjustment, to the extent that the effects of the present invention are not impaired. The additional component is not particularly limited, and may be appropriately selected from known additives according to the application of the composite particles 1, or the like. Specific examples of the additional component include organometallic compounds such as alkoxysilanes, and hydrolysates thereof, inorganic layered compounds, inorganic needle-like minerals, antifoaming agents, inorganic particles, organic particles, lubricants, antioxidants, antistatic agents, ultraviolet absorbers, stabilizers, magnetic materials, orientation promoters, plasticizers, crosslinking agents, pharmaceutical products, agricultural chemicals, perfumes, adhesives, enzymes, colorants, deodorants, metals, metal oxides, inorganic oxides, preservatives, antibacterial agents, natural extracts, and surfactants.

Since fine fibers typically have a fiber form derived from a microfibril structure, the fine fibers used in the production method of the present embodiment preferably have a fiber form with a size in the following range. That is, the fine fibers are preferably in a fibrous form. Furthermore, the fibrous fine fibers may have a number average minor axis diameter of 1 nm or more and 1000 nm or less, and preferably 2 nm or more and 500 nm or less.

If the number average minor axis diameter is less than 1 nm, a highly crystalline and rigid fine fiber structure cannot be formed; thus, it is difficult to stabilize the emulsion and to form composite particles by a polymerization reaction using the emulsion as a template, solidification of the polymer, and the like. On the other hand, if the number average minor axis diameter exceeds 1000 nm, the size of the fine fibers is too large to stabilize the emulsion; thus, it is difficult to control the size and shape of the resulting composite particles.

The number average major axis diameter of the fine fibers is not particularly limited, but may be preferably 5 times or more the number average minor axis diameter. If the number average major axis diameter is less than 5 times the number average minor axis diameter, it may be impossible to sufficiently control the size and shape of the composite particles, which is not preferable.

The number average minor axis diameter of the fine fibers is obtained as the average value of the minor axis diameter (minimum diameter) of 100 fibers measured by observation with a transmission electron microscope and an atomic force microscope. Also, the number average major axis diameter of the fine fibers is obtained as the average value of the major axis diameter (maximum diameter) of 100 fibers measured by observation with a transmission electron microscope and an atomic force microscope.

The type and crystal structure of cellulose that can be used as a cellulose raw material are not particularly limited. Specific examples of a raw material composed of cellulose type I crystals include, in addition to wood-based natural cellulose, non-wood-based natural cellulose such as cotton linters, bamboo, hemp, bagasse, kenaf, bacterial cellulose, hoya cellulose, and valonia cellulose. Furthermore, the cellulose raw material may be regenerated cellulose represented by rayon fibers and cuprammonium rayon fibers composed of cellulose type II crystals.

In terms of material availability, wood-based natural cellulose is preferable as a raw material. The wood-based natural cellulose is not particularly limited, and may be wood-based natural cellulose typically used to produce cellulose nanofibers, such as softwood pulp, hardwood pulp, or waste paper pulp. Softwood pulp is preferable in terms of ease of purification and fine grinding.

A chitin/chitosan raw material is a linear structural polysaccharide for supporting and protecting animal bodies such as tendons of arthropods including crustaceans such as crabs and shrimps, insects, and spiders, and squids, and the chitin/chitosan raw material is crystalline (has a portion in which molecules are regularly arranged) and is partially bonded to protein. Chitin is a polysaccharide containing N-acetylglucosamine as a main constituent sugar.

When a chitin and/or chitosan raw material is isolated and purified, there is little purified chitin consisting of 100% N-acetylglucosamine, and most of the obtained chitin and/or chitosan partially contains glucosamine as a constituent. For example, the chitin and/or chitosan raw material may be one of the following two types: a raw material obtained by crushing the shells of king crabs in a dry state into approximately 5 mm pieces; and a raw material obtained by crushing the pens of spear squids in a wet state into approximately 1 cm pieces. It is preferable to purify the chitin/chitosan raw material in advance.

Furthermore, the fine fiber raw material is preferably chemically modified. More specifically, it is preferable to introduce an ionic functional group to the crystal surface of the fine fiber raw material. This is because when an ionic functional group is introduced to the crystal surface of the fine fiber raw material, the solvent is more likely to enter between the crystals of the fine fiber raw material due to the osmotic pressure effect, thereby facilitating fine grinding of the fine fiber raw material. When the fine fibers have an ionic functional group, the fine fibers have a high droplet stabilizing effect. Furthermore, when a functional material is adsorbed via the ionic functional group on the fine fibers, the functional material can be supported by the fine fibers.

The type of ionic functional group introduced to the crystal surface of the fine fiber raw material is not particularly limited. For example, when the fine fibers have an anionic functional group, the fine fibers and a cationic functional material can be supported. Furthermore, by coordinating metal ions to the fine fibers having an anionic functional group, and reducing and depositing the metal ions, metal fine particles can be supported as a functional material by the fine fibers. The type of anionic functional group and the method of introducing the anionic functional group are not particularly limited, but it is preferable to introduce a carboxyl group or a phosphate group. When cellulose is used as a fine fiber raw material, in terms of ease of selective introduction to the crystal surface of the cellulose, it is preferable to introduce a carboxyl group.

The method of introducing a carboxyl group to the crystal surface of the cellulose is not particularly limited. For example, the cellulose may be carboxymethylated by reacting the cellulose with monochloroacetic acid or sodium monochloroacetate in a highly concentrated alkaline aqueous solution. Alternatively, a carboxyl group may be introduced to the cellulose by directly reacting the cellulose with a carboxylic acid anhydride compound such as maleic acid or phthalic acid gasified in an autoclave.

Alternatively, a carboxyl group may be introduced by a method in which a co-oxidant is used in the presence of an N-oxyl compound such as TEMPO that has high selectivity for oxidation of alcoholic primary carbon, while maintaining the structure as much as possible under relatively mild aqueous conditions. Oxidation using an N-oxyl compound is more preferable for the selectivity of a site for introducing a carboxyl group and reduction of the environmental load.

Examples of the N-oxyl compound include TEMPO (2,2,6,6-tetramethylpiperidinyl-1-oxy radical), 2,2,6,6-tetramethyl-4-hydroxypiperidin-1-oxyl, 4-methoxy-2,2,6,6-tetramethylpiperidin-N-oxyl, 4-ethoxy-2,2,6,6-tetramethylpiperidin-N-oxyl, and 4-acetamide-2,2,6,6-tetramethylpiperidin-N-oxyl. Of these, TEMPO, which has high reactivity, is preferable. The amount of N-oxyl compound to be used is not particularly limited, and may be the amount required for use as a colorant. Typically, the amount of N-oxyl compound is 0.01 mass% or more and 5.0 mass% or less with respect to the solid content of the wood-based natural cellulose subjected to oxidation treatment.

The oxidation method using an N-oxyl compound may be, for example, a method in which wood-based natural cellulose is dispersed in water and subjected to oxidation treatment in the presence of the N-oxyl compound. In this method, it is preferable to use a co-oxidant in combination with the N-oxyl compound. In this case, in the reaction system, the N-oxyl compound is gradually oxidized by the co-oxidant to generate an oxammonium salt, by which the cellulose is oxidized. With this oxidation treatment, the oxidation reaction proceeds smoothly even under mild conditions, leading to improvement in the efficiency of introducing a carboxyl group. When the cellulose is subjected to oxidation treatment under mild conditions, the crystal structure of the cellulose is more likely to be maintained.

The co-oxidant may be any oxidant that can promote the oxidation reaction, such as halogen, hypohalous acid, halous acid, or perhalous acid, or a salt thereof, halogen oxide, nitrogen oxide, or peroxide. In terms of availability and reactivity, sodium hypochlorite is preferable. The amount of co-oxidant to be used is not particularly limited as long as the co-oxidant can promote the oxidation reaction. Typically, the amount of co-oxidant is approximately 1 to 200 mass% with respect to the solid content of the wood-based natural cellulose subjected to oxidation treatment.

In combination with the N-oxyl compound and the co-oxidant, at least one compound selected from the group consisting of bromide and iodide may be used. The use of such a compound allows the oxidation reaction to proceed smoothly, leading to improvement in the efficiency of introducing a carboxyl group. The compound is preferably sodium bromide or lithium bromide, and sodium bromide is more preferable in terms of cost and stability. The amount of compound to be used is not particularly limited as long as the compound can promote the oxidation reaction. Typically, the amount of compound is approximately 1 to 50 mass% with respect to the solid content of the wood-based natural cellulose subjected to oxidation treatment.

The reaction temperature of the oxidation reaction is preferably 4°C or more and 80°C or less, and more preferably 10°C or more and 70°C or less. If the reaction temperature is less than 4°C, the reactivity of the reagent is reduced, resulting in a longer reaction time. If the reaction temperature exceeds 80°C, side reactions are promoted which reduce the molecular weight of the sample and collapse the highly crystalline and rigid fiber structure of the cellulose nanofibers; thus, the cellulose nanofibers cannot be used as an O/W emulsion stabilizer.

The reaction time of the oxidation treatment is not particularly limited, and may be appropriately set in consideration of the reaction temperature, the desired amount of carboxyl group, and the like, but is typically 10 minutes or more and 5 hours or less.

The pH of the reaction system during the oxidation reaction is not particularly limited, but is preferably 9 or more and 11 or less. When the pH of the reaction system is 9 or more, the reaction can proceed efficiently. If the pH of the reaction system exceeds 11, side reactions proceed, and thus decomposition of the sample may be accelerated. In the oxidation treatment, as the oxidation proceeds, a carboxyl group is generated, resulting in a reduction of the pH in the system. Thus, during the oxidation treatment, it is preferable to maintain the pH of the reaction system in the range of 9 or more and 11 or less. Examples of the method of maintaining the pH of the reaction system in the range of 9 or more and 11 or less include a method of adding an alkaline aqueous solution according to the reduction of the pH.

Examples of the alkaline aqueous solution include organic alkali such as a sodium hydroxide aqueous solution, a lithium hydroxide aqueous solution, a potassium hydroxide aqueous solution, an ammonia aqueous solution, a tetramethylammonium hydroxide aqueous solution, a tetraethylammonium hydroxide aqueous solution, a tetrabutylammonium hydroxide aqueous solution, and a benzyltrimethylammonium hydroxide aqueous solution. A sodium hydroxide aqueous solution is preferable in terms of cost and the like.

The oxidation reaction using an N-oxyl compound can be stopped by adding alcohol to the reaction system. In this case, the pH of the reaction system is preferably maintained in the range described above. In order to quickly complete the reaction, the alcohol to be added is preferably a low-molecular-weight alcohol such as methanol, ethanol, or propanol. Ethanol is particularly preferable in terms of safety of by-products generated by the reaction, and the like.

The reaction liquid after the oxidation treatment may be directly subjected to the fine-grinding step; however, in order to remove a colorant such as an N-oxyl compound, impurities, and the like, it is preferable to collect oxidized cellulose contained in the reaction liquid and wash the oxidized cellulose with a cleaning liquid. The oxidized cellulose can be collected by a known method such as filtration using a glass filter or a nylon mesh with a pore diameter of 20 $\mu$m. The cleaning liquid used to wash the oxidized cellulose is preferably pure water.

When the obtained TEMPO-oxidized cellulose is subjected to fibrillation treatment, TEMPO-oxidized cellulose nanofibers (also referred to as TEMPO-oxidized CNF, cellulose single nanofibers, or CSNF) having a uniform fiber width of 3 nm are obtained. When CSNF is used as a raw material of the cellulose nanofibers of the composite particles 1, due to the uniform structure of the CSNF, the resulting O/W emulsion is more likely to have a uniform particle size.

As described above, the CSNF used in the present embodiment can be obtained by the step of oxidizing a cellulose raw material and the step of finely grinding the cellulose raw material to obtain a dispersion liquid of the cellulose. The content of carboxyl group introduced into the CSNF is preferably 0.1 mmol/g or more and 5.0 mmol/g or less, and more preferably 0.5 mmol/g or more and 2.0 mmol/g or less.

If the content of carboxyl group is less than 0.1 mmol/g, solvent entry due to the osmotic pressure effect does not occur between the cellulose microfibrils, and thus fine grinding and uniform dispersion of the cellulose are difficult. If the content of carboxyl group exceeds 5.0 mmol/g, side reactions caused by chemical treatment reduces the molecular weight of the cellulose microfibrils, thereby preventing formation of a highly crystalline and rigid fiber structure of the cellulose nanofibers; thus, the cellulose nanofibers cannot be used as an O/W emulsion stabilizer.

If necessary, a functional material may be supported by the fine fibers in the dispersion liquid of the fine fibers to obtain a dispersion liquid of the fine fibers supporting the functional material. The fine fibers supporting the functional material can be used to obtain composite particles.

The method of adding a functional material to the fine fibers is not particularly limited, and a known method may be used. For example, a functional material may be added and mixed in the dispersion liquid of the fine fibers to attach the functional material to the fine fibers. Alternatively, a functional material may be synthesized in the dispersion liquid of the fine fibers to cause the fine fibers to support the functional material.

As described above, when a functional material is supported by the fine fibers, it is preferable to introduce in advance an anionic functional group to the crystal surface of the fine fibers. When a cationic functional material is added and mixed in a dispersion liquid of the fine fibers having an anionic functional group, the functional material can be stably supported by the fine fibers. By adjusting the pH of the dispersion liquid, it is possible to control support or removal of the functional material.

The concentration of the fine fibers for supporting a functional material is not particularly limited, but is preferably 0.1 mass% or more and 20 mass% or less, and more preferably 0.5 mass% or more and 10 mass% or less. If the concentration of the fine fibers is less than 0.1%, it is difficult for the fine fibers to efficiently support the functional material. If the concentration of the fine fibers exceeds 20 mass%, the fine fibers have a very high viscosity and cannot be sufficiently stirred, and this makes it difficult to achieve a uniform reaction.

The solvent of the dispersion liquid of the fine fibers is not particularly limited, but is preferably a hydrophilic solvent. The hydrophilic solvent is not particularly limited, but is preferably, water; an alcohol such as methanol, ethanol, or isopropanol; or a cyclic ether such as tetrahydrofuran. In particular, the suspension preferably contains 50 mass% or more of water. If the ratio of water in the suspension is less than 50 mass%, the fine fibers may have poor dispersibility. A solvent other than water contained in the suspension is preferably any of the hydrophilic solvents described above.

If necessary, the pH of the suspension may be adjusted in order to improve the dispersibility of the fine fiber raw material and the fine fibers. Examples of an alkaline aqueous solution used for pH adjustment include organic alkali such as a sodium hydroxide aqueous solution, a lithium hydroxide aqueous solution, a potassium hydroxide aqueous solution, an ammonia aqueous solution, a tetramethylammonium hydroxide aqueous solution, a tetraethylammonium hydroxide aqueous solution, a tetrabutylammonium hydroxide aqueous solution, and a benzyltrimethylammonium hydroxide aqueous solution. A sodium hydroxide aqueous solution is preferable in terms of cost and the like.

The method in which metal fine particles are supported as a functional material by cellulose nanofibers having an anionic functional group is not particularly limited, and for example, the following method may be used.

A method including a cellulose nanofiber dispersion liquid preparing step of preparing a dispersion liquid containing at least one type of cellulose nanofibers, a metal salt/cellulose nanofiber-containing liquid preparing step of preparing a dispersion liquid containing at least one type of metal salt and at least one type of cellulose nanofibers, and a metal fine particle-supporting cellulose nanofiber dispersion liquid preparation step of reducing metal ions in the metal salt/cellulose nanofiber-containing liquid to cause the cellulose nanofibers to support metal fine particles.

If necessary, the fine fibers supporting the functional material may be washed by filtration, centrifugation, or the like to remove free functional materials.

(Second-i step)

The second-i step is a step of coating the surface of droplets containing a pore forming agent and a core material with the fine fibers in the dispersion liquid of the fine fibers to stabilize the droplets as an emulsion.

Specifically, in the second-i step, a core material-containing liquid containing a pore forming agent and a core material is added to the fine fiber dispersion liquid obtained in the first step, and dispersed as droplets in the fine fiber dispersion liquid, and the surface of the droplets is coated with the fine fibers to produce an O/W emulsion stabilized by the fine fibers. The O/W emulsion stabilized by the fine fibers is referred to as an emulsion liquid.

The method of producing an O/W emulsion is not particularly limited, and may be typical emulsification treatment, for example, various types of homogenizer treatment or mechanical stirring treatment. Specifically, the production method may be mechanical treatment using a high-pressure homogenizer, an ultrahigh-pressure homogenizer, a universal homogenizer, a ball mill, a roll mill, a cutter mill, a planetary mill, a jet mill, an attritor, a grinder, a blender, a homomixer, an ultrasonic homogenizer, a nanogenizer, aqueous counter collision, or a paint shaker. Two or more types of mechanical treatment may be used in combination.

For example, when an ultrasonic homogenizer is used, a core material-containing liquid is added to the dispersion liquid of the fine fibers to form a mixed solvent, and the tip of the ultrasonic homogenizer is inserted into the mixed solvent to perform ultrasonic treatment. The conditions for the ultrasonic homogenizer treatment are not particularly limited, but for example, the frequency is typically 20 kHz or more, and the output is typically 10 W/cm$^2$ or more. The treatment time is not particularly limited, but is typically approximately 10 seconds to 1 hour.

By the ultrasonic treatment, droplets containing a pore forming agent and a core material are dispersed in the dispersion liquid of the fine fibers and emulsified, and the fine fibers are selectively adsorbed at the liquid-liquid interface between the droplets and the fine fiber dispersion liquid; thus, the droplets are coated with the fine fibers to form a stable structure as an O/W emulsion. Such an emulsion that is stabilized by the adsorption of a solid material on the liquid-liquid interface is academically called a "Pickering emulsion".

Although, as described above, the mechanism of how the fine fibers form a Pickering emulsion is not clear, presumably, since cellulose has a hydrophilic site derived from a hydroxyl group and a hydrophobic site derived from a hydrocarbon group in its molecular structure and thus exhibits amphipathic properties, due to the amphipathic properties, the fine fibers are adsorbed on the liquid-liquid interface between the hydrophobic monomer and the hydrophilic solvent.

As described above, when the droplets dispersed in the O/W emulsion contain a hydrophobic non-polymerizable solvent as a pore forming agent, oil droplets containing the non-polymerizable solvent are formed in the core particles during solidification of the core material in the third-i step. By removing the non-polymerizable solvent, pores are formed in the core particles.

When the droplets dispersed in the O/W emulsion contain an excessive amount of surfactant, the hydrophilic solvent as a continuous phase is absorbed in the droplets and functions as a pore forming agent, and the core particles generated by solidification of the core material in the third-i step contain the hydrophilic solvent. By removing the hydrophilic solvent, pores are formed in the core particles.

The O/W emulsion structure can be confirmed by observation with an optical microscope. The particle size of the O/W emulsion is not particularly limited, but the average particle size is preferably 0.1 $\mu$m or more and 1000 $\mu$m or

less. The average particle size can be calculated by measuring the diameter of 100 randomly selected emulsions, and taking the average value of the diameter of the emulsions.

In the O/W emulsion structure, the thickness of the coating layer (fine fiber layer) formed on the surface layer of the droplets containing the pore forming agent and the core material is not particularly limited, but is preferably 3 nm or more and 1000 nm or less. Although not particularly limited, the particle size of the emulsion structure is approximately the same as the particle size of the composite particles obtained in the third-i step. The thickness of the coating layer can be measured, for example, by using a cryo-TEM.

The weight ratio between the fine fiber dispersion liquid and the core material used in the second-i step is not particularly limited, but the weight ratio of the polymerizable monomer is preferably 1 part by mass or more and 50 parts by mass or less with respect to 100 parts by mass of the fine fibers. If the weight ratio of the polymerizable monomer is 1 part by mass or less, the yield of composite particles is reduced, which is not preferable. If the weight ratio of the polymerizable monomer exceeds 50 parts by mass, it is difficult to uniformly coat the droplets containing the pore forming agent and the core material with the fine fibers, which is not preferable.

The core material-containing liquid to be used may be one that contains a core material and is able to form an O/W emulsion, and is preferably hydrophobic in order to stably form an O/W emulsion. The core material is a material that is solidified by a chemical or physicochemical change to form core particles. The core material is not particularly limited as long as the core material can stably form droplets containing a pore forming agent and a core material. The core material may be, for example, a polymerizable monomer (monomer), a molten polymer, or a dissolved polymer.

The type of polymerizable monomer used in the second-i step is not particularly limited as long as the polymerizable monomer is a monomer of a polymer, has a polymerizable functional group in its structure, is liquid at normal temperature, is immiscible with water, and can form a polymer (high-molecular-weight polymer) by a polymerization reaction. The polymerizable monomer has at least one polymerizable functional group. A polymerizable monomer having a single polymerizable functional group is also referred to as a monofunctional monomer. A polymerizable monomer having two or more polymerizable functional groups is also referred to as a polyfunctional monomer.

The type of polymerizable monomer is not particularly limited, and may be, for example, a (meth)acrylic monomer, a vinyl monomer, or the like. The polymerizable monomer may be a polymerizable monomer (e.g., $\varepsilon$-caprolactone, etc.) having a cyclic ether structure such as an epoxy group or an oxetane structure. The term "(meth)acrylate" refers to both "acrylate" and "methacrylate".

Examples of monofunctional (meth)acrylic monomers include 2-hydroxyethyl (meth)acrylate, 2-hydroxypropyl (meth)acrylate, 2-hydroxybutyl (meth)acrylate, n-butyl (meth)acrylate, isobutyl (meth)acrylate, t-butyl (meth)acrylate, glycidyl (meth)acrylate, acryloylmorpholine, N-vinylpyrrolidone, tetrahydrofurfuryl acrylate, cyclohexyl (meth)acrylate, 2-ethylhexyl (meth)acrylate, isobornyl (meth)acrylate, isodecyl (meth)acrylate, lauryl (meth)acrylate, tridecyl (meth)acrylate, cetyl (meth)acrylate, stearyl (meth)acrylate, benzyl (meth)acrylate, 2-ethoxyethyl (meth)acrylate, 3-methoxybutyl (meth)acrylate, ethyl carbitol (meth)acrylate, phosphoric acid (meth)acrylate, ethylene oxide-modified phosphoric acid (meth)acrylate, phenoxy (meth)acrylate, ethylene oxide-modified phenoxy (meth)acrylate, propylene oxide-modified phenoxy (meth)acrylate, nonyl phenol (meth)acrylate, ethylene oxide-modified nonyl phenol (meth)acrylate, propylene oxide-modified nonyl phenol (meth)acrylate, methoxy diethylene glycol (meth)acrylate, methoxy polyethylene glycol (meth)acrylate, methoxy propylene glycol (meth)acrylate, 2-(meth)acryloyl oxyethyl-2-hydroxypropyl phthalate, 2-hydroxy-3-phenoxy propyl (meth)acrylate, 2-(meth)acryloyl oxyethyl hydrogen phthalate, 2-(meth)acryloyl oxypropyl hydrogen phthalate, 2-(meth)acryloyl oxypropyl hexahydro hydrogen phthalate, 2-(meth)acryloyl oxypropyl tetrahydro hydrogen phthalate, dimethylaminoethyl (meth)acrylate, trifluoroethyl (meth)acrylate, tetrafluoropropyl (meth)acrylate, hexafluoropropyl (meth)acrylate, octafluoropropyl (meth)acrylate, octafluoropropyl (meth)acrylate, and adamantane derivative mono(meth)acrylates, such as adamantyl acrylate having a monovalent mono(meth)acrylate, that are derived from 2-adamantane and adamantane diol.

Examples of bifunctional (meth)acrylic monomers include di(meth)acrylates such as ethylene glycol di(meth)acrylate, diethylene glycol di(meth)acrylate, butanediol di(meth)acrylate, hexanediol di(meth)acrylate, nonanediol di(meth)acrylate, ethoxylated hexanediol di(meth)acrylate, propoxylated hexanediol di(meth)acrylate, diethylene glycol di(meth)acrylate, polyethylene glycol di(meth)acrylate, tripropylene glycol di(meth)acrylate, polypropylene glycol di(meth)acrylate, neopentyl glycol di(meth)acrylate, ethoxylated neopentyl glycol di(meth)acrylate, tripropylene glycol di(meth)acrylate, and hydroxypivalic acid neopentyl glycol di(meth)acrylate.

Examples of trifunctional or higher functional (meth)acrylic monomers include tri(meth)acrylates such as trimethylolpropane tri(meth)acrylate, ethoxylated trimethylolpropane tri(meth)acrylate, propoxylated trimethylolpropane tri(meth)acrylate, tris-2-hydroxyethyl isocyanurate tri(meth)acrylate, and glycerol tri(meth)acrylate; trifunctional (meth)acrylate compounds such as pentaerythritol tri(meth)acrylate, dipentaerythritol tri(meth)acrylate, and ditrimethylolpropane tri(meth)acrylate; polyfunctional (meth)acrylate compounds having three or more polymerizable functional groups, such as pentaerythritol tetra(meth)acrylate, ditrimethylolpropane tetra(meth)acrylate, dipentaerythritol tetra(meth)acrylate, dipentaerythritol penta(meth)acrylate, ditrimethylolpropane penta(meth)acrylate, dipentaerythritol hexa(meth)acrylate, and ditrimethylolpropane hexa(meth)acrylate; and polyfunctional (meth)acrylate compounds in which part of these (meth)acrylates is substituted with an alkyl group or ε-caprolactone.

Preferable examples of monofunctional vinyl monomers include liquids that are immiscible with water at normal temperature, such as vinyl ether monomers, vinyl ester monomers, and aromatic vinyl monomers, and particularly styrene and styrene monomers.

Examples of (meth)acrylates of the monofunctional vinyl monomers include methyl (meth)acrylate, ethyl (meth)acrylate, butyl (meth)acrylate, t-butyl (meth)acrylate, 2-ethylhexyl (meth)acrylate, lauryl (meth)acrylate, alkyl (meth)acrylate, tridecyl (meth)acrylate, stearyl (meth)acrylate, cyclohexyl (meth)acrylate, benzyl (meth)acrylate, isobornyl (meth)acrylate, glycidyl (meth)acrylate, tetrahydrofurfuryl (meth)acrylate, allyl (meth)acrylate, diethylaminoethyl (meth)acrylate, trifluoroethyl (meth)acrylate, heptafluorodecyl (meth)acrylate, dicyclopentenyl (meth)acrylate, dicyclopentenyloxyethyl (meth)acrylate, and tricyclodecanyl (meth)acrylate.

Examples of monofunctional aromatic vinyl monomers include styrene, α-methyl styrene, o-methylstyrene, m-methylstyrene, p-methylstyrene, ethyl styrene, isopropenyltoluene, isobutyltoluene, tert-butylstyrene, vinylnaphthalene, vinylbiphenyl, and 1,1-diphenylethylene.

Examples of polyfunctional vinyl monomers include a polyfunctional group having an unsaturated bond, such as divinylbenzene. A liquid that is immiscible with water at normal temperature is preferable.

Specific examples of polyfunctional vinyl monomers include (1) divinyls such as divinylbenzene, 1,2,4-trivinylbenzene, and 1,3,5-trivinylbenzene; (2) dimethacrylates such as ethylene glycol dimethacrylate, diethylene glycol dimethacrylate, triethylene glycol dimethacrylate, polyethylene glycol dimethacrylate, 1,3-propylene glycol dimethacrylate, 1,4-butylene glycol dimethacrylate, 1,6-hexamethylene glycol dimethacrylate, neopentyl glycol dimethacrylate, dipropylene glycol dimethacrylate, polypropylene glycol dimethacrylate, and 2,2-bis(4-methacryloxydiethoxyphenyl)propane; (3) trimethacrylates such as trimethylolpropane trimethacrylate and triethylolethane trimethacrylate; (4) diacrylates such as ethylene glycol diacrylate, diethylene glycol diacrylate, triethylene glycol diacrylate, polyethylene glycol diacrylate, 1,3-dipropylene glycol diacrylate, 1,4-dibutylene glycol diacrylate, 1,6-hexylene glycol diacrylate, neopentyl glycol diacrylate, dipropylene glycol diacrylate, polypropylene glycol diacrylate, 2,2-bis(4-acryloxypropoxyphenyl)propane, and 2,2-bis(4-acryloxydiethoxyphenyl)propane; (5) triacrylates such as trimethylolpropane triacrylate and triethylolethane triacrylate; (6) tetraacrylates such as tetramethylolmethane tetraacrylate; and (7) others such as tetramethylenebis(ethyl fumarate), hexamethylenebis(acrylamide), triallyl cyanurate, and triallyl isocyanurate.

Specific examples of polyfunctional styrene monomers include divinylbenzene, trivinylbenzene, divinyltoluene, divinylnaphthalene, divinylxylene, divinylbiphenyl, bis(vinylphenyl)methane, bis(vinylphenyl)ethane, bis(vinylphenyl)propane, and bis(vinylphenyl)butane.

Other than these, it is possible to use resins such as a polyether resin, a polyester resin, a polyurethane resin, an epoxy resin, an alkyd resin, a spiroacetal resin, a polybutadiene resin, and a polythiol polyene resin that have at least one or more polymerizable functional groups, and the materials of the resins are not particularly limited.

The above polymerizable monomers may be used alone or in combination of two or more.

Furthermore, a polymerization initiator may be added to the polymerizable monomer. Typical examples of polymerization initiators include radical initiators such as organic peroxides and azo polymerization initiators.

Examples of organic peroxides include peroxyketal, hydroperoxide, dialkyl peroxide, diacyl peroxide, peroxycarbonate, and peroxyester.

Examples of azo polymerization initiators include 2,2-azobis(isobutyronitrile) (AIBN), 2,2-azobis(2-methylbutyronitrile) (AMBN), 2,2-azobis(2,4-dimethylvaleronitrile) (ADVN), 1,1-azobis(1-cyclohexanecarbonitrile) (ACHN), dimethyl-2,2-azobisisobutyrate (MAIB), 4,4-azobis(4-cyanovaleric acid) (ACVA), 1,1-azobis(1-acetoxy-1-phenylethane), 2,2-azobis(2-methylbutyramide), 2,2-azobis(4-methoxy-2,4-dimethylvaleronitrile), 2,2-azobis(2-methylamidinopropane)dihydrochloride, 2,2-azobis[2-(2-imidazoline-2-yl)propane], 2,2-azobis[2-methyl-N-(2-hydroxyethyl)propionamide], 2,2-azobis(2,4,4-trimethylpentane), 2-cyano-2-propylazoformamide, 2,2-azobis(N-butyl-2-methylpropionamide), and 2,2-azobis(N-cyclohexyl-2-methylpropionamide).

In the case where a core material-containing liquid containing a polymerizable monomer and a polymerization initiator is used in the second-i step, the polymerization initiator is contained in the droplets of the O/W emulsion (droplets containing the pore forming agent and the core material) in the third-i step (described later), thus facilitating a polymerization reaction when the monomer in the droplets of the emulsion is polymerized in the third-i step (described later).

The weight ratio between the polymerizable monomer and the polymerization initiator that can be used in the second-i step is not particularly limited, but in general, the weight ratio of the polymerization initiator is preferably 0.1 parts by mass or more with respect to 100 parts by mass of the polymerizable monomer. If the weight ratio of the polymerizable monomer is less than 0.1 parts by mass, the polymerization reaction does not sufficiently proceed, resulting in a lower yield of composite particles, which is not preferable.

The polymer used to obtain a dissolved polymer is not particularly limited, but is preferably difficult to be dissolved in a hydrophilic solvent. If the polymer is dissolved in a hydrophilic solvent, a stable emulsion cannot be formed.

The following are examples of the polymer used to obtain a dissolved polymer.

Cellulose acetate, cellulose acetate derivatives such as cellulose acetate butyrate and cellulose acetate propionate, polysaccharides such as chitin and chitosan, and polylactic acids such as polylactic acid, and copolymers of lactic acid and other hydroxycarboxylic acids.

Dibasic acid polyesters such as polybutylene succinate, polyethylene succinate, and polybutylene adipate. Polycaprolactones such as polycaprolactone, and copolymers of caprolactone and hydroxycarboxylic acid.

Polyhydroxy butyrates such as polyhydroxy butyrate, and copolymers of polyhydroxy butyrate and hydroxycarboxylic acid.

Aliphatic polyesters such as polyhydroxybutyric acid, and copolymers of polyhydroxybutyric acid and other hydroxycarboxylic acids.

Polyamino acids, polyester-polycarbonates, natural resins such as rosin, and the like. These materials may be used alone or in combination of two or more.

By dissolving the polymer in a solvent, a dissolved polymer can be obtained. The solvent in which the polymer is dissolved preferably has low immiscibility with the dispersion liquid of the fine fibers. If the solvent has high solubility in the hydrophilic solvent, the solvent is easily dissolved and transferred from a dispersed phase (droplets) to a continuous phase (hydrophilic solvent), thus making emulsification difficult.

The solvent in which the polymer is dissolved preferably has a boiling point of 90°C or less. If the boiling point exceeds 90°C, the hydrophilic solvent of the dispersion liquid of the fine fibers is evaporated before the solvent; thus, it is difficult to obtain composite particles 1.

Specific examples of the solvent that can be used include benzene, toluene, xylene, ethyl acetate, butyl acetate, methyl ethyl ketone (MEK), methyl isobutyl ketone (MIBK), isophorone, cellosolve acetate, isophorone, Solvesso 100, trichlene, hexane, chloroform, dichloromethane, dichloroethane, isooctane, and nonane.

The weight ratio between the polymer to be dissolved and the solvent is not particularly limited as long as the polymer can be dissolved in the solvent. The weight of the solvent is preferably 0.005 parts by mass or more and 100 parts by mass or less, and more preferably 0.1 parts by mass or more and 80 parts by mass or less with respect to 100 parts by mass of the polymer.

The method in which a polymer is melted to obtain a molten polymer is preferably, for example, the following method. First, a polymer that is solid at normal temperature is melted into a liquid. Then, as described above, while the molten polymer is subjected to mechanical treatment using an ultrasonic homogenizer or the like, the molten polymer is added to the dispersion liquid of the fine fibers heated to a temperature at which the molten state of the polymer can be maintained, thereby stabilizing the molten polymer droplets as an O/W emulsion in the dispersion liquid.

The molten polymer preferably has low solubility in the hydrophilic solvent of the fine fibers. If the molten polymer has high solubility in the hydrophilic solvent, the polymer is easily dissolved and transferred from a dispersed phase (droplets) to a continuous phase (hydrophilic solvent), thus making emulsification difficult. The molten polymer preferably has a melting point of 90°C or less. If the melting point exceeds 90°C, water in the dispersion liquid of the fine fibers is evaporated, thus making emulsification difficult.

Specific examples of the polymer used for a molten polymer include pentaerythritol tetrastearate, pentaerythritol distearate, pentaerythritol tristearate, stearyl stearate, batyl stearate, stearyl stearate, myristyl myristate, cetyl palmitate, ethylene glycol distearate, behenyl alcohol, microcrystalline wax, paraffin wax, hydrocarbon wax, fatty acid alkyl ester, polyol fatty acid ester, a mixture of fatty acid ester and wax, a mixture of fatty acid esters, glycerol monopalmitate(/stearic acid monoglyceride), glycerol monodistearate(/glycerol stearate), glycerol monoacetate monostearate(/glycerol fatty acid ester), succinic acid aliphatic monoglyceride(/glycerol fatty acid ester), citric acid saturated aliphatic monoglyceride, sorbitan monostearate, sorbitan fatty acid ester, sorbitan tribehenate, propylene glycol monobehenate(/propylene glycol fatty acid ester), stearate of adipic acid pentaerythritol polymer, pentaerythritol tetrastearate, dipentaerythritol hexastearate, stearyl citrate, pentaerythritol fatty acid ester, glycerol fatty acid ester, ultra-light-colored rosin, rosin-containing diol, ultra-light-colored rosin metal salt, hydrogenated petroleum resin, rosin ester, hydrogenated rosin ester, special rosin ester, novolac, crystalline poly-$\alpha$-olefin, polyalkylene glycol, polyalkylene glycol ester, polyoxyalkylene ether, polylactic acids such as polylactic acid and copolymers of lactic acid and other hydroxycarboxylic acids, dibasic acid polyesters such as polybutylene succinate, polyethylene succinate, and polybutylene adipate, polycaprolactones such as polycaprolactone and copolymers of caprolactone and hydroxycarboxylic acid, polyhydroxy butyrates such as polyhydroxy butyrate and copolymers of polyhydroxy butyrate and hydroxycarboxylic acid, aliphatic polyesters such as polyhydroxybutyric acid and copolymers of polyhydroxybutyric acid and other hydroxycarboxylic acids, polyamino acids, polyester-polycarbonates, and natural resins such as rosin.

When a core material-containing liquid containing a pore forming agent is used, core particles having pores can be obtained. As described above, the pore forming agent may be a known pore forming agent, and for example, a non-polymerizable solvent can be used.

Examples of the non-polymerizable solvent include aromatic compounds, acetic ester compounds, and aliphatic hydrocarbons. Examples of aromatic compounds include toluene and xylene, examples of acetic ester compounds include ethyl acetate and butyl acetate, and examples of aliphatic hydrocarbons include n-hexane, n-heptane, n-octane, and n-dodecane.

Although not particularly limited, it is preferable to use a non-polymerizable solvent that dissolves the core material but does not dissolve the solidified core particles. From this viewpoint, as a non-polymerizable solvent, it is preferable to use acetic ester compounds or aliphatic hydrocarbons, and n-hexane or n-heptane is particularly preferable.

When the non-polymerizable solvent that dissolves the core material but does not dissolve the solidified core particles is used, composite particles including porous or hollow core particles can be obtained by obtaining droplets in which the non-polymerizable solvent is dissolved in the core material in the second-i step, solidifying the core material and depositing the non-polymerizable solvent as oil droplets in the generated core particles in the third-i step, and removing the hydrophilic solvent in the fourth step.

The weight ratio between the core material and the pore forming agent used in the second-i step is not particularly limited, but the content ratio of the pore forming agent to the total mass of the core material and the pore forming agent is, for example, 10 mass% or more and 90 mass% or less, preferably 20 mass% or more and 80 mass% or less, and more preferably 30 mass% or more and 80 mass% or less. If the content ratio of the pore forming agent is 10 mass% or less, the pores formed in the core particles are insufficient or the core particles have a low porosity (void ratio), and this may cause the composite particles to lack a sufficient soft-focus effect and good texture. On the other hand, if the content ratio of the pore forming agent exceeds 90 mass%, it is difficult to obtain spherical particles.

The core material-containing liquid preferably contains an oil-soluble surfactant for the purpose of obtaining core particles having pores. When the core material-containing liquid contains a large amount of oil-soluble surfactant, in the second-i step or the third-i step, the hydrophilic solvent which is a solvent as a continuous phase is absorbed in the droplets, and the hydrophilic solvent functions as a pore forming agent. Thus, by removing the solvent in the fourth step, composite particles including porous or hollow core particles can be obtained.

The oil-soluble surfactant is not particularly limited as long as the oil-soluble surfactant can be dissolved in the core material. The HLB (hidrophilic-lipophilic balance) is preferably 8 or less. The oil-soluble surfactant is preferably a nonionic surfactant, and examples of the nonionic surfactant include sorbitan fatty acid ester, glycerol fatty acid ester, polyoxyethylene alkylamine, and alkylalkanolamide. Of these, sorbitan fatty acid ester is preferable, and examples of the sorbitan fatty acid ester include sorbitan monooleate, sorbitan monostearate, sorbitan monopalmitate, and sorbitan trioleate.

The weight ratio between the core material and the oil-soluble surfactant that can be used in the second-i step is not particularly limited, but the concentration of the oil-soluble surfactant is preferably above the critical micelle concentration. When the concentration of the oil-soluble surfactant is above the critical micelle concentration, the hydrophilic solvent as a continuous phase is absorbed, and the hydrophilic solvent functions as a pore forming agent; thus, porous or hollow composite particles can be produced.

Furthermore, an additional component other than the polymerization initiator may be added in advance to the droplets (droplets containing the core material and the pore forming agent) to be dispersed in the dispersion liquid of the fine fibers in the second-i step.

Specific examples of the additional component include colorants, oil absorbing agents, light shielding agents (ultraviolet absorbers, ultraviolet scattering agents, etc.), antibacterial agents, antioxidants, antiperspirants, antifoaming agents, antistatic agents, binding agents, bleaching agents, chelating agents, deodorizing agents, aromatics, perfumes, dandruff preventing active substances, skin softeners, insect repellents, preservatives, natural extracts, beauty components, pH adjusting agents, vitamins, amino acids, hormones, oil-based raw materials such as fats, oils, and waxes, surfactants, inorganic particles (titanium oxide, silica, clay, etc.), and enzymes.

When an additional component other than the polymerization initiator is added in advance to the droplets containing the core material and the pore forming agent, the core particles of the formed composite particles can contain the additional component, enabling the composite particles to exhibit a function according to the application.

It is preferable for the additional component to easily dissolve or disperse in the droplets containing the core material and the pore forming agent and be difficult to dissolve or disperse in the hydrophilic solvent. When the additional component dissolves or disperses in the droplets, the droplets of the formed O/W emulsion are more likely to contain the additional component, and this makes it possible to efficiently obtain composite particles containing the additional component. The amount of additional component to be contained in the composite particles may be increased.

Furthermore, a polymerizable monomer, a dissolved polymer, and a molten polymer may be used in combination as a core material to form droplets containing a core material and a pore forming agent and emulsify the droplets. When a biodegradable polymer (resin) is selected as a polymer of the core particles of the composite particles, the resulting composite particles are composed of the core particles and the fine fibers that are made of a biodegradable polymer, thus achieving the composite particles that contain a biodegradable material and are highly environmentally friendly.

(Second-ii step)

The second-ii step is a step of dispersing droplets (hydrophilic droplets) containing a pore forming agent such as a hydrophilic solvent in a core material to produce a W/O emulsion liquid, dispersing droplets (hydrophobic droplets) containing the W/O emulsion liquid in the fine fiber dispersion liquid, and coating the surface of the droplets with the fine fibers to stabilize the droplets as a W/O/W emulsion liquid.

Specifically, a stabilizer is dissolved or dispersed in a core material, and a hydrophilic solvent such as water is added and dispersed as a pore forming agent in the core material to produce a W/O emulsion in which droplets (hydrophilic droplets) are dispersed. Next, the W/O emulsion liquid is added to the fine fiber dispersion liquid obtained in the first step and dispersed as droplets in the fine fiber dispersion liquid, and the surface of the droplets (hydrophobic droplets) is coated with the fine fibers to produce a W/O/W emulsion stabilized by the fine fibers.

In the second-ii step, the same materials and methods as in the second-i step are applied unless otherwise specified.

The method of producing a W/O emulsion is not particularly limited, and may be typical emulsification treatment, for example, various types of homogenizer treatment or mechanical stirring treatment. Specifically, the production method may be mechanical treatment using a high-pressure homogenizer, an ultrahigh-pressure homogenizer, a universal homogenizer, a ball mill, a roll mill, a cutter mill, a planetary mill, a jet mill, an attritor, a grinder, a blender, a homomixer, an ultrasonic homogenizer, a nanogenizer, aqueous counter collision, or a paint shaker. Two or more types of mechanical treatment may be used in combination.

For example, when an ultrasonic homogenizer is used, a hydrophilic solvent such as water is added as a pore forming agent to a core material-containing liquid containing a stabilizer to form a mixed solvent, and the tip of the ultrasonic homogenizer is inserted into the mixed solvent to perform ultrasonic treatment. The conditions for the ultrasonic homogenizer treatment are not particularly limited, but for example, the frequency is typically 20 kHz or more, and the output is typically 10 W/cm$^2$ or more. The treatment time is not particularly limited, but is typically approximately 10 seconds to 1 hour.

By the ultrasonic treatment, droplets containing the hydrophilic solvent as a pore forming agent are dispersed in the core material-containing liquid and emulsified, and the stabilizer is selectively adsorbed on the liquid-liquid interface between the droplets and the core material phase, thereby forming a stable structure as a W/O emulsion.

The hydrophilic solvent as a pore forming agent contained in the hydrophilic droplets may be a solvent that is difficult to be dissolved in the core material. For example, a solvent similar to the hydrophilic solvent used as a continuous phase may be used.

The stabilizer is used to disperse the droplets containing the hydrophilic solvent as a pore forming agent in the core material to produce a W/O emulsion liquid. The stabilizer is not particularly limited as long as the stabilizer can stabilize the droplets, and may be, for example, an oil-soluble surfactant or a solid material.

As described above, the oil-soluble surfactant may be a nonionic surfactant. Examples of the nonionic surfactant include sorbitan fatty acid ester, glycerol fatty acid ester, polyoxyethylene alkylamine, and alkylalkanolamide. Of these, sorbitan fatty acid ester is preferable, and examples of the sorbitan fatty acid ester include sorbitan monooleate, sorbitan monostearate, sorbitan monopalmitate, and sorbitan trioleate.

The solid material may be one that is able to stabilize the droplets, and examples of the solid material include clay, carbon black, silica particles, titanium oxide particles, and fine fibers. When fine fibers are used, it is preferable to introduce a hydrophobic functional group to the surface of the fibers. The method of introducing the hydrophobic functional group is not particularly limited, and the hydrophobic functional group can be introduced by an ionic bond, a covalent bond, or the like. When the fine fibers are fine fibers having an ionic functional group, the hydrophobic functional group can be introduced by an ionic bond using organic onium ions or the like.

As in the second-i step, the obtained W/O emulsion liquid is dispersed as droplets in the fine fiber dispersion liquid, and the surface of the droplets is coated with the fine fibers to stabilize the droplets as a W/O/W emulsion liquid.

By solidifying the core material in the W/O/W emulsion in the third-i step, it is possible to obtain core particles containing the droplets that are coated with the fine fibers and contain the hydrophilic solvent, and by removing the hydrophilic solvent, it is possible to obtain composite particles including porous or hollow core particles coated with the fine fibers.

(Third-i step)

The third-i step is a step of solidifying the core material in the droplets to obtain a dispersion liquid of particles in which the surface of the core particles is coated with the fine fibers.

The method of solidifying the core material is not particularly limited. When a polymerizable monomer is used as a core material, the core material can be solidified by polymerizing the polymerizable monomer to form a polymerized monomer. When a dissolved polymer is used as a core material, the core material can be solidified by removing the solvent from the dissolved polymer using a method in which the solvent in the droplets is dispersed in a hydrophilic solvent or a method in which the solvent is evaporated. When a molten polymer is used as a core material, the core material can be solidified by solidifying the molten polymer by cooling.

In the production method I, when the droplets contained in the O/W emulsion formed in the second-i step are composed of a liquid in which a hydrophobic non-polymerizable solvent is dissolved in the core material, the non-polymerizable solvent is deposited as oil droplets in the third-i step; thus, core particles containing the oil droplets are formed. When the core material contains an oil-soluble surfactant, the hydrophilic solvent as a continuous phase is absorbed by the droplets; thus, core particles containing the hydrophilic solvent are formed.

In the production method II, the core material in the droplets contained in the W/O/W emulsion formed in the second-ii step is solidified in the third-i step; thus, core particles containing the droplets containing the hydrophilic solvent are formed.

For example, by stirring and heating the O/W emulsion produced in the second-i step and the second-ii step in which the droplets containing the polymerizable monomer as a core material and the polymerization initiator are coated with the fine fibers and stabilized, the polymerizable monomer is polymerized, thereby solidifying the core material. The stirring method is not particularly limited, and may be a known method. Specifically, a disperser or a stirring bar may be used. Alternatively, the O/W emulsion may be only heated without being stirred.

The temperature for heating the O/W emulsion may be appropriately set according to the types of polymerizable monomer and polymerization initiator, but the temperature is preferably 20°C or more and 150°C or less. If the temperature is less than 20°C, the reaction rate of polymerization is reduced, which is not preferable. If the temperature exceeds 150°C, the fine fibers may be modified, which is not preferable. The time for the polymerization reaction may be appropriately set according to the types of polymerizable monomer and polymerization initiator, but is typically approximately 1 to 24 hours. The polymerization reaction may be performed by ultraviolet irradiation treatment, i.e., using a type of electromagnetic waves. Other than electromagnetic waves, particle beams such as electron beams may be used.

As the specific method of evaporating the solvent of the dissolved polymer, the solvent is evaporated to be removed by heating or/and drying under reduced pressure. When the boiling point of the organic solvent is lower than that of water, the organic solvent can be selectively removed. Although not particularly limited, the solvent can be efficiently removed by heating under reduced pressure. The heating temperature is preferably 20°C or more and 100°C or less, and the pressure is preferably 600 mHg or more and 750 mmHg or less.

As the specific method of dispersing the solvent of the dissolved polymer, the solvent in the droplets is dispersed by adding a solvent or salt to the O/W emulsion liquid. When the solvent having low solubility in the hydrophilic solvent is dispersed over time in the hydrophilic solvent phase, the dissolved polymer can be deposited and solidified as particles.

As the method of solidifying the molten polymer, the molten polymer is solidified by cooling the O/W emulsion liquid.

(Fourth step)

The fourth step is a step of collecting, from the dispersion liquid obtained in the third-i step, the core particles that contain the pore forming agent and whose surface is coated with the fine fibers, and removing the pore forming agent contained in the core particles to form pores in the core particles. As the collection method, for example, centrifugation may be used.

Immediately after the particles coated with the fine fibers are generated in the dispersion liquid obtained in the third-i step, the dispersion liquid contains a mixture of the particles coated with the fine fibers, a large amount of water, and free fine fibers that do not contribute to the formation of the coating layer. Thus, it is necessary to collect and wash the particles coated with the fine fibers from the dispersion liquid in this state, and the collection and washing are preferably performed by centrifugation washing or filtration washing.

The centrifugation washing may be performed by a known method. Specifically, the particles coated with the fine fibers can be collected by repeating the operation in which the particles coated with the fine fibers are precipitated by centrifugation to remove the supernatant and the particles are redispersed in a mixed solvent of water and methanol, and removing the residual solvent from the precipitate finally obtained by the centrifugation.

The filtration washing may also be performed by a known method. The composite particles can be collected, for example, by repeating suction filtration with water and methanol using a PTFE membrane filter with a pore diameter of 0.1 μm, and removing the residual solvent from the paste that finally remains on the membrane filter.

The method of removing the pore forming agent is not particularly limited, and the pore forming agent can be removed by drying under normal pressure or reduced pressure at normal temperature or during heating according to the solvent to be removed. The dry solid containing the composite particles obtained in this manner is not in the form of a film or an aggregate, and is obtained as a fine powder as described above.

If necessary, the composite particles may be dispersed in a solvent, and in the dispersion liquid of the composite particles, a functional material may be supported by the fine fibers on the surface of the composite particles.

The method of causing the composite particles to support a functional material is not particularly limited, and a known method may be used. For example, it is possible to use a method in which the composite particles are redispersed in a hydrophilic solvent such as water or alcohol to produce a dispersion liquid of the composite particles, and a functional material is added and mixed in the dispersion liquid of the composite particles to attach the functional material to the composite particles. Alternatively, a functional material may be synthesized in the dispersion liquid of the composite particles to cause the composite particles to support the functional material.

The method in which metal fine particles are supported as a functional material by composite particles coated with cellulose nanofibers having an anionic functional group as fine fibers is not particularly limited, and for example, the following method may be used.

A method including a step of preparing a dispersion liquid containing at least one type of the composite particles described above, a step of preparing a dispersion liquid containing at least one type of metal salt and composite particles, and a step of reducing metal ions in the dispersion liquid containing the metal salt and the composite particles to prepare a dispersion liquid of the composite particles supporting the metal fine particles.

If necessary, the composite particles supporting the functional material may be separated by filtration, centrifugation, or the like to remove free functional materials.

In the composite particles of the present embodiment, any of the functional components described above may be impregnated or contained in the pores. The composite particles containing a functional component can exhibit a function of the functional component.

A dry powder of the composite particles of the present embodiment can be used for light diffusion films, heat insulating materials, home care products, and personal care products. In particular, it is preferable to use the dry powder of the composite particles of the present embodiment as personal care particles that are used for personal care.

Personal care products are products such as cosmetics and toiletries for maintenance of cleanliness of human skin, grooming, and beautification. Personal care products are, for example, products related to hair care, oral care, odor care, nail care, body care, skin care, and makeup. Examples of such products include toothpastes, perfumes, nail lacquers, skin lotions, milky lotions, cleansing agents, eye shadows, eyeliners, foundations, blushes, hair gels, hair sprays, shampoos, conditioners, hair tonics, lotions, creams, and soaps.

The composite particles of the present embodiment can be mixed or dispersed in other cosmetic raw materials, and used for personal care products such as makeup products. Furthermore, the composite particles 1 of the present embodiment can be dispersed in a solvent containing a cosmetic raw material and the like, and used as a personal care composition.

Examples of cosmetic raw materials include colorants, oil absorbing agents, light shielding agents (ultraviolet absorbers, ultraviolet scattering agents, etc.), antibacterial agents, antioxidants, antiperspirants, antifoaming agents, antistatic agents, binding agents, bleaching agents, chelating agents, deodorizing components, aromatics, perfumes, dandruff preventing active substances, skin softeners, insect repellents, preservatives, natural extracts, beauty components, pH adjusting agents, vitamins, amino acids, hormones, oil-based raw materials such as fats, oils, and waxes, surfactants, and inorganic particles (titanium oxide, silica, clay, etc.).

(Advantageous effects of composite particles)

The composite particles of the first aspect of the present invention are composite particles having fine fibers, which are in a novel form that can be produced by a simple method, and solve the problems of the fine fibers, such as cellulose nanofibers or chitin nanofibers, having excessive solvent.

Since the composite particles of the first aspect of the present invention are coated with the fine fibers that have high safety, high hydrophilicity, high strength, and a large specific surface area, the composite particles have high strength even as porous particles, have good dispersion stability, exhibit various functions such as high moisturizing properties, skin pore correction effect, and soft-focus effect, have good skin compatibility, and can be produced by a simple method.

Even when the composite particles of the first aspect of the present invention are porous particles having a high porosity and a large specific surface area or hollow particles having a high porosity, the composite particles do not collapse during production, and are obtained as stable composite particles.

When a functional component is impregnated in the pores of the composite particles of the first aspect of the present invention, composite particles that exhibit various effects are obtained.

When in the composite particles of the first aspect of the present invention, the fine fibers such as cellulose nanofibers or chitin nanofibers are made of cellulose or chitin/chitosan which is a biodegradable polymer, and the core particle is also made of a material containing a biodegradable polymer, the composite particles are expected to reduce the environmental load and contribute to solving the problem of microplastics.

By the production method of the second and third aspects of the present invention, the composite particles of the first aspect of the present invention are produced.

The composite particles of the first aspect of the present invention can be used for personal care applications. Thus, the composite particles of the first aspect of the present invention can be contained in personal care products and personal care compositions.

The composite particles of the first aspect of the present invention can be used, for example, for products related to hair care, oral care, odor care, body care, skin care, and makeup. Specific examples of such products include toothpastes, perfumes, nail lacquers, beauty essences, skin lotions, milky lotions, cleansing agents, eye shadows, eyeliners, foundations, blushes, hair gels, hair sprays, shampoos, conditioners, hair tonics, lotions, creams, and soaps.

[Second embodiment]

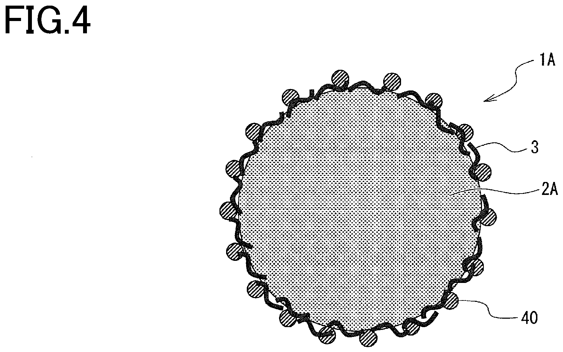

As shown in Fig. 4, a personal care particle 1A of the present embodiment is composed of a core particle 2A made of a material containing a polymer, the fine fibers 3 inseparably bonded to a surface of the core particle 2A, and a functional material 40 that exhibits a personal care function. The fine fibers 3 are either cellulose nanofibers or chitin nanofibers. The functional material 40 is supported by the fine fibers 3. The fine fibers 3 may contain both cellulose nanofibers and chitin nanofibers.

In the present embodiment, the fine fibers 3 are formed as a coating layer that coats the surface of the core particle 2A.

The method of producing the personal care particles 1A is not particularly limited, and may be a known method, and a chemical preparation method or a physicochemical preparation method can be used.

Examples of the chemical preparation method include polymerization granulation methods in which particles are formed from a polymerizable monomer in a polymerization process (an emulsion polymerization method, a suspension polymerization method, a seed polymerization method, a radiation polymerization method, etc.).

Examples of the physicochemical preparation method include dispersion granulation methods in which particles are formed from a polymer solution in the form of microdroplets (a spray drying method, an in-liquid curing method, a solvent evaporation method, a phase separation method, a solvent dispersion cooling method, etc.).

For example, by forming an O/W Pickering emulsion containing the fine fibers 3, and solidifying the core material in the droplets to produce particles in which the surface of the core particles 2A is coated with the fine fibers 3, it is possible to obtain the personal care particles 1A in which the core particles 2A and the fine fibers 3 are inseparably bonded to each other. The use of the fine fibers 3 makes it possible to form stable droplets without using an additive such as a surfactant, and obtain the personal care particles 1A that have high dispersibility, exhibit highly advantageous effects due to the functional material, and have high smoothness and good skin compatibility.

The core material may be one that forms the core particles 2A when solidified, and may be, for example, a polymerizable monomer, a molten polymer, or a dissolved polymer.

The method of solidifying the core material is not particularly limited, and the core material can be solidified by polymerizing a polymerizable monomer, solidifying a molten polymer, or removing a solvent from a dissolved polymer.

Personal care articles are products such as cosmetics and toiletries for maintenance of cleanliness of human skin, grooming, and beautification. Personal care articles are, for example, products related to hair care, oral care, odor care, nail care, body care, skin care, and makeup. Examples of such products include toothpastes, perfumes, nail lacquers, skin lotions, milky lotions, cleansing agents, eye shadows, eyeliners, foundations, blushes, hair gels, hair sprays, shampoos, conditioners, hair tonics, lotions, creams, and soaps.

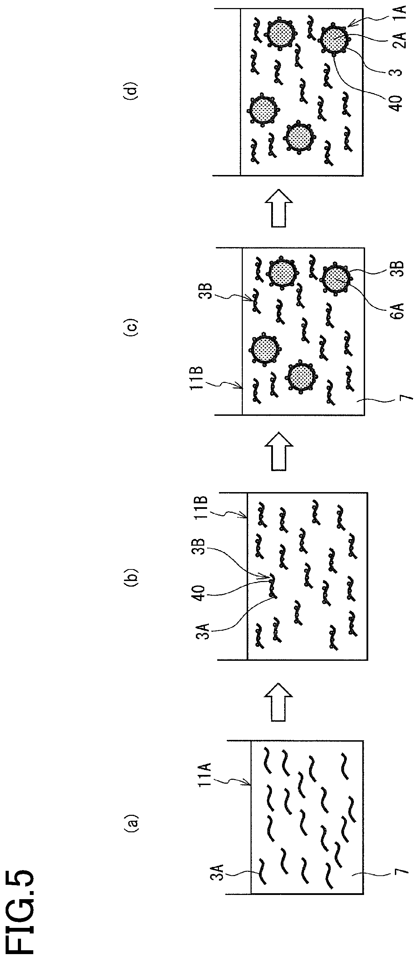

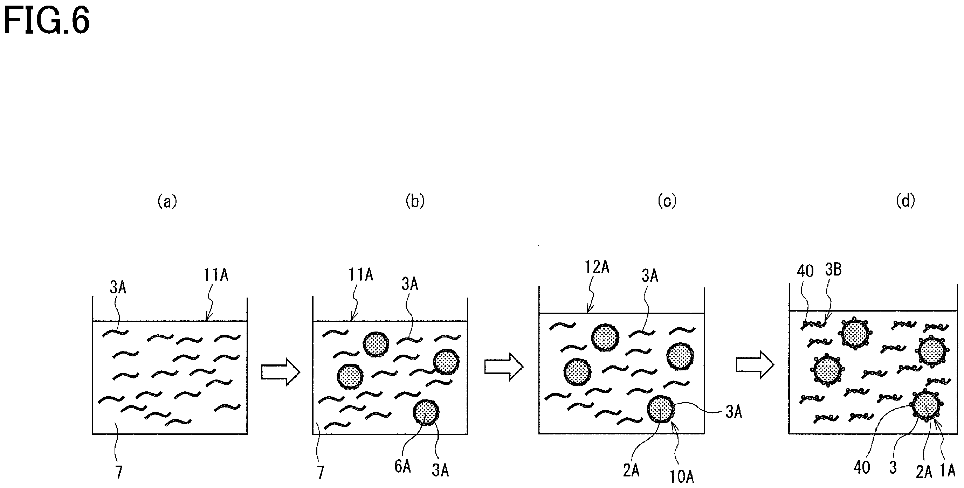

The personal care particles 1A of the present embodiment can be produced by production methods shown in Figs. 5 and 6.

The production method shown in the figure corresponds to the production method of the fifth aspect (production method IVI), and includes the first step, a second-iii step, a second-iv step, and a third-ii step.

As shown in Fig. 5 (a), the first step is a step of fibrillating a cellulose raw material or a chitin/chitosan raw material in the hydrophilic solvent 7 to obtain a dispersion liquid 11A of fine fibers 3A that are either cellulose nanofibers or chitin nanofibers supporting no material. The second-iii step is a step of causing the fine fibers 3A supporting no material in the dispersion liquid 11A of the fine fibers 3A to support the functional material 40 that exhibits a personal care function, thereby obtaining a dispersion liquid 11B of fine fibers 3B supporting the functional material 40, as shown in Fig. 5 (b).

As shown in Fig. 5 (c), the second-iv step is a step of dispersing droplets 6A containing a core material in the dispersion liquid 11B of the fine fibers 3B supporting the functional material, and coating the surface of the droplets 6A with the fine fibers 3B supporting the functional material.

The third-ii step is a step of solidifying the core material in the droplets 6A. In this step, as shown in Fig. 5 (d), the personal care particles (particles in which the surface of the core particles 2A is coated with the fine fibers 3 supporting the functional material 40) 1A are obtained.

Then, the personal care particles 1A are separated and collected from the dispersion liquid in the state shown in Fig. 5 (d).

Thus, in the production method III, the fine fibers 3B supporting the functional material are adsorbed on the interface of the droplets 6A dispersed in the hydrophilic solvent 7, thereby stabilizing the O/W Pickering emulsion. Then, while the stabilized state is maintained, the core material in the droplets 6A of the emulsion is solidified to obtain the personal care particles 1A.

The production method shown in Fig. 6 corresponds to the production method of the sixth aspect (production method IV), and includes the first step, the second-iv step, the third-ii step, and a fifth step.

As shown in Fig. 6 (a), the first step is a step of fibrillating a cellulose raw material or a chitin/chitosan raw material in the hydrophilic solvent 7 to obtain the dispersion liquid 11A of the fine fibers 3A that are either cellulose nanofibers or chitin nanofibers supporting no material.

As shown in Fig. 6 (b), the second-iv step is a step of dispersing the droplets 6A containing the core material in the dispersion liquid 11A of the fine fibers 3A supporting no material, and coating the surface of the droplets 6A with the fine fibers 3A supporting no material.

The third-ii step is a step of solidifying the core material in the droplets 6A to obtain a dispersion liquid 12A of particles 10A in which the surface of the core particles 2A is coated with the fine fibers 3A supporting no material as shown in Fig. 6 (c).

As shown in Fig. 6 (d), the fifth step is a step of causing the fine fibers 3A on the surface of the particles 10A in the dispersion liquid 12A to support the functional material 40 that exhibits a personal care function. In this step, the personal care particles (particles in which the surface of the core particles 2A is coated with the fine fibers 3 supporting the functional material 40) 1A are obtained. In this step, the functional material 40 is also supported by the fine fibers 3A supporting no material that are independently present in the dispersion liquid 12A to form the fine fibers 3B supporting the functional material.

Then, the personal care particles 1A are separated and collected from the dispersion liquid in the state shown in Fig. 6 (d).

Thus, in the production method IV, the fine fibers 3A are adsorbed on the interface of the droplets 6A that contain the core material and are dispersed in the hydrophilic solvent 7, thereby stabilizing the O/W Pickering emulsion. Then, while the stabilized state is maintained, the core material in the emulsion is solidified to obtain the particles 10A in which the surface of the core particles 2A is coated with the fine fibers 3A. Subsequently, the functional material 40 is supported by the fine fibers 3A on the surface of the particles 10 coated with the fine fibers to obtain the personal care particles 1A.

The term "inseparable" as used herein means that the core particles and the fine fibers are not separated from each other and the coating of the core particles with the fine fibers is maintained even after repetition of the operation of centrifuging a dispersion liquid containing the personal care particles to remove the supernatant, redispersing the personal care particles in a solvent, and collecting and washing the personal care particles, or the operation of washing the personal care particles with a solvent by filtration washing using a membrane filter. The coating state can be confirmed by observing the surface of the personal care particles with a scanning electron microscope.

The mechanism of bonding between the fine fibers and the core particles during production of the personal care particles of the present embodiment by the above production methods is not clear, but the following presumption can be made. The personal care particles are produced using, as a template, an O/W emulsion stabilized by the fine fibers. Accordingly, while the fine fibers are in contact with the core material in the droplets of the emulsion, the core material is solidified to form core particles. Thus, the fine fibers are physically fixed to the surface of the core particles, resulting in the core particles and the fine fibers being inseparable from each other.

The O/W emulsion is also referred to as an oil-in-water emulsion, in which oil is dispersed as oil droplets (oil particles) in water as a continuous phase.

Although not particularly limited, when personal care particles are produced using, as a template, an O/W emulsion stabilized by the fine fibers, due to the stabilized O/W emulsion, it is possible to obtain personal care particles having a spherical shape derived from the O/W emulsion. Specifically, it is preferable to form a coating layer that is composed of the fine fibers and has a relatively uniform thickness on the surface of the core particles having a spherical shape.

The particle size of the personal care particles can be confirmed by observation with an optical microscope. The average particle size of the particles can be calculated by measuring the diameter of the particles at 100 random locations, and taking the average value of the diameter of the particles. The average particle size is not particularly limited, but is preferably 0.1 $\mu$m or more and 1000 $\mu$m or less.

From the viewpoint of dispersion stability, the fine fibers are preferably formed as a coating layer on the surface of the core particles. The coating layer preferably coats the entire surface of the core particles, but may not necessarily coat the entire surface.

The thickness of the coating layer composed of the fine fibers is not particularly limited, but is preferably 3 nm or more and 1000 nm or less.

The average thickness of the coating layer can be calculated by cutting the personal care particles fixed with an embedding resin using a microtome, observing the personal care particles with a scanning electron microscope, measuring the thickness of the coating layer in the cross-sectional image of the personal care particles at 100 random locations in the image, and taking the average value of the thickness at the 100 locations.

The personal care particles are preferably uniformly coated with a coating layer having a relatively uniform thickness. When the particles are coated with a coating layer having a uniform thickness, the particles have high dispersion stability. Specifically, the coefficient of variation of the thickness of the coating layer is preferably 0.5 or less, and more preferably 0.4 or less.

The fine fibers constituting the personal care particles of the present embodiment are cellulose nanofibers and/or chitin nanofibers, as with the fine fibers constituting the composite particles of the first embodiment.

The fine fibers constituting the personal care particles of the present embodiment preferably have an ionic functional group on the crystal surface. When the fine fibers have an ionic functional group, aggregation of the personal care particles can be prevented, thus achieving personal care articles that effectively exhibit a function of the functional material and have good skin compatibility. When a functional material is supported by using the ionic functional group, it is possible to prevent or control removal of the functional material. For example, the fine fibers having an anionic functional group can easily support a cationic functional material, and the fine fibers having a cationic functional group can easily support an anionic functional material.

The type of ionic functional group is not particularly limited, but is preferably an anionic functional group. In the case where the fine fibers have an anionic functional group, for example, when metal fine particles are supported by the fine fibers, by coordinating metal ions to the anionic functional group of the fine fibers, and reducing and depositing the metal ions, the metal fine particles can be efficiently supported by the fine fibers. Furthermore, the fine fibers function as a dispersion stabilizer, thus enabling control of the shape and particle size of metal fine particles to be generated. Furthermore, it is possible to prevent removal of the metal fine particles from the fine fibers in the personal care particles. In general, when metal fine particles are dried, the metal fine particles are aggregated; thus, optical characteristics due to localized surface plasmon resonance (LSPR) (described later) cannot be exhibited. However, when the personal care particles are in the form of a dry powder, the metal fine particles do not become aggregated; thus, the personal care particles as a dry powder can exhibit the optical characteristics.

Examples of the anionic functional group include, but are not particularly limited to, a carboxyl group, a phosphate group, and a sulfo group. Of these, a carboxyl group and a phosphate group are preferable, and a carboxyl group is more preferable in terms of ease of selective introduction to the crystal surface of cellulose.

The content of ionic functional group is preferably 0.1 mmol or more and 5.0 mmol or less per 1 g of fine fiber raw material and/or fine fibers. If the content of ionic functional group is less than 0.1 mmol, the personal care particles may have poor dispersion stability, and if the content of ionic functional group exceeds 5.0 mmol, it may be difficult to stably support the functional material.

The fine fibers constituting the personal care particles of the present embodiment preferably have a fiber form derived from a microfibril structure. Specifically, the fine fibers preferably have a fibrous form, and have a number average minor axis diameter of 1 nm or more and 1000 nm or less, and a number average major axis diameter of 50 nm or more. The number average major axis diameter is preferably 5 times or more the number average minor axis diameter. The degree of crystallinity of the fine fibers is preferably 50% or more. The crystal structure of the cellulose nanofibers is preferably cellulose type I. The crystal structure of the chitin nanofibers may be alpha chitin or beta chitin, but is preferably alpha chitin.

In the personal care particles of the present embodiment, since the surface of the core particles is coated with the fine fibers supporting the functional material, the functional material has high dispersion stability; thus, the personal care particles can effectively exhibit a function of the functional material. The state in which a functional material is "supported" by fine fibers indicates that the functional material is physically or chemically and reversibly or irreversibly bonded to or adsorbed on the fine fibers. In the personal care particles of the present invention, the functional material can be supported by a known method.

In the personal care particles of the present embodiment, the functional material and the fine fibers are "inseparable" from each other. The state in which the fine fibers and the functional material are "inseparable" from each other indicates that the fine fibers and the functional material are not separated from each other even after repetition of the operation of centrifuging a dispersion liquid containing the personal care particles to remove the supernatant, redispersing the personal care particles in a solvent, and collecting and washing the personal care particles, or the operation of repeatedly washing the personal care particles with a solvent by filtration washing using a membrane filter.

The functional material constituting the personal care particles of the present embodiment is a material that exhibits its function in personal care articles, and may be a known functional material. Examples of the functional material are the functional materials exemplified in the first embodiment.

When colorants are used as a functional material, organic synthetic colorants and natural colorants are used to color hair coloring agents such as hair dyes, skin lotions, and beauty essences, and inorganic pigments and organic pigments are used for makeup products to control hue, saturation, and brightness of foundations and control the covering performance of foundations. Pigments and dyes may be contained in combination in lipsticks and nail enamels.

Dyes are substances that are dissolved in a solvent such as water, oil, or alcohol and are present in a dissolved state in a cosmetic base material and can color the material. Dyes include water-soluble dyes which are soluble in water, and oil-soluble dyes which are soluble in oil and alcohol. Examples of dyes include azo dyes, xanthene dyes, and quinoline dyes. Although not particularly limited, as a dye, it is preferable to use cationic dyes which have cationic properties or anionic dyes which have anionic properties. For example, cationic dyes can be adsorbed on fine fibers having an anionic functional group, and anionic dyes can be adsorbed on fine fibers having a cationic functional group.

Lakes are metal salts of water-soluble dyes.

Organic pigments are colored powders that have no soluble group in their structure and are not dissolved in water, oil, or the like. Examples of organic pigments include azo pigments, indigo pigments, and phthalocyanine pigments.

Natural colorants include colorants derived from animals and plants, and colorants derived from microorganisms. Examples of natural colorants include carotenoid colorants having a yellow to orange-red color from carrots, tomatoes, red salmon, and the like, and flavonoid colorants having a yellow to reddish-purple color from hibiscus, safflower, and the like.

Examples of inorganic pigments include extender pigments, colored pigments, white pigments, and pearlescent pigments.

Extender pigments are used to adjust the gloss and feeling in use. Examples of extender pigments include talc (hydrated magnesium silicate, talcum), kaolin (hydrous aluminum silicate, white clay), mica (mica, hydrous aluminum potassium silicate), silica (silicic anhydride), calcium carbonate ($CaCO_3$), and magnesium carbonate ($MgCO_3$).

Examples of colored pigments include iron oxide, ultramarine blue, Prussian blue, chromium oxide, chromium hydroxide, manganese violet, and carbon black.

White pigments are used not only for color adjustment but also for covering spots and freckles and protection from ultraviolet light. Examples of white pigments include titanium oxide and zinc oxide.

Examples of pearlescent pigments include titanium oxide-coated mica (titanated mica), and bismuth oxychloride. The thickness of the titanium oxide that coats the mica is varied to exhibit various interference colors.

Examples of polymer powders include alternately laminated polymers having different refractive indices, nylon powders, and polymethylmethacrylate powders.

Metal fine particles may be used as a colorant and a light shielding agent. Metal fine particles have properties that bulk metal does not have. For example, particles with a smaller size have a larger total surface area, and thus have higher catalyst performance. Nano-sized particles have a low melting point, and thus can be fired at a low temperature. Furthermore, depending on the size of particles, optical characteristics of the particles significantly vary. In the case where oxide particles having a small refractive index have a size of 1/10 or less of the visible light wavelength range, the oxide particles scatter a very small amount of light; thus, when the oxide particles are uniformly dispersed in a liquid or the like, the oxide particles appear transparent.

Nano-sized metal fine particles have properties of absorbing light having a specific wavelength according to their element, particle size, and shape, due to localized surface plasmon resonance. In some cases, free electrons on the surface of the metal fine particles collectively oscillate due to an external electric field such as light. Since electrons are electrically charged particles, when electrons oscillate, an electric field is generated around the electrons. The phenomenon in which an electric field generated by oscillation of free electrons resonates with an external electric field (light, etc.) is referred to as localized surface plasmon resonance (LSPR). LSPR allows the metal fine particles to absorb or reflect light in a specific wavelength range for light shielding.

When metal fine particles absorb or reflect light having a wavelength in the visible light range, the metal fine particles function as a colorant. The wavelength of light absorbed or scattered by metal fine particles varies depending on the type, particle size, and shape of metal constituting the metal fine particles. Metal fine particles are characterized to have high stability and stably absorb or scatter light in a specific wavelength range over a long period of time, as compared with organic pigments generally used as a colorant.

The metal element constituting the metal fine particles is not particularly limited, but the metal fine particles preferably contain at least one of rubidium (Ru), iron (Fe), cobalt (Co), rhodium (Rh), nickel (Ni), palladium (Pd), platinum (Pt), copper (Cu), silver (Ag), gold (Au), and zinc (Zn). The metal fine particles may be composed of a single element or a plurality of elements.

Of these, the metal fine particles preferably contain one or more types of metals or metal compounds selected from nickel (Ni), palladium (Pd), silver (Ag), and gold (Au), but the metal fine particles are not particularly limited to this. Metal fine particles containing silver (Ag) can impart antibacterial properties.

When a plurality of types of metals are used, for example, deposited silver (Ag) fine particles may be coated with a metal nobler than silver, a metal oxide such as silica, or the like to improve the stability of the silver (Ag) fine particles.

The metal fine particles may contain components other than metal.

The average particle size of the metal fine particles is not particularly limited, but is preferably 500 nm or less. If the average particle size exceeds 500 nm, it is difficult for the fine fibers to support the metal fine particles. The average particle size is more preferably 0.1 nm or more and 50 nm or less. Depending on the average particle size of the metal fine particles, the color tone of the metal fine particles may vary; thus, it is important to control the shape of the metal fine particles. The shape of the metal fine particles is not particularly limited. For example, the metal fine particles may have a spherical shape, a flat plate shape, or a rod shape.

In particular, in metal fine particles having an anisotropic shape such as flat plate-like metal fine particles having a flat plate shape and rod-like metal fine particles having a rod shape, depending on the aspect ratio of the metal fine particles, the absorption or scattering wavelength due to LSPR significantly varies. Among the metal fine particles having an anisotropic shape, in particular, applications of flat plate-like silver fine particles and rod-like gold fine particles are expected.

For example, it is known that spherical silver nanoparticles having a particle size of several nanometers to several tens of nanometers have absorption in the vicinity of a wavelength of 400 nm due to LSPR, and thus have a yellowish color. However, this does not apply to anisotropically grown silver fine particles, and for example, the absorption peak of flat plate-like silver fine particles is known to be red-shifted. It is confirmed that in this case, as the aspect ratio (i.e., particle size/particle thickness) of the flat plate-like silver fine particles is increased, the absorption/scattering peak is shifted toward the long wavelength side. Thus, the flat plate-like silver fine particles can be used as an optical material that absorbs/scatters an arbitrary wavelength. Furthermore, by controlling the absorption/scattering wavelength in the visible light range, it is possible to obtain flat plate-like silver fine particles having a vivid color tone such as red or blue, other than yellow, and the flat plate-like silver fine particles function as a colorant.

Depending on the aspect ratio of the flat plate-like silver fine particles, the absorption peak of the flat plate-like silver fine particles can be shifted to the near-infrared range, outside the visible light range, and the flat plate-like silver fine particles also function as a light shielding agent against near-infrared light.

The absorption/reflection peak of rod-like gold fine particles is shifted according to the aspect ratio (major axis/minor axis) of the rod-like gold fine particles.

In the present embodiment, visible light refers to electromagnetic waves in a wavelength range of approximately 400 nm to 700 nm, and near-infrared light refers to electromagnetic waves in a wavelength range (approximately 700 nm to 2500 nm) that is the infrared wavelength range near the visible light wavelength range.

In the present embodiment, "fine particles" of the metal fine particles refer to particles having a volume particle size of 10 $\mu$m or less. Furthermore, flat plate-like silver fine particles refer to silver fine particles having a flat plate shape, and rod-like gold fine particles refer to gold fine particles having a rod shape.

Assume that h is the thickness of the flat plate-like metal fine particles, d (equivalent circle diameter) is the diameter of the flat plate-like metal fine particles, and an aspect ratio (d/h) is the ratio of the diameter d to the thickness h of the flat plate-like metal fine particles. The "flat plate" shape of the flat plate-like metal fine particles indicates that the particles have a plate shape, and the plate shape of the particles indicates that the particles have, for example, an average aspect ratio (d/h) of 1.1 or more.

The shapes of the front and back surfaces of the flat plate-like metal fine particles are not particularly limited, but are typically a polygonal shape such as a hexagonal shape or a triangular shape. When the front and back surfaces have a polygonal shape, an elliptical shape, or the like, the diameter d (equivalent circle diameter) is calculated assuming that the diameter d is the diameter of a circle having an area equivalent to that of the front or back surface.

In the flat plate-like metal fine particles, the front surface may have a larger or smaller area than the back surface, and the front surface may not necessarily be parallel to the back surface.

From the viewpoint of controlling the optical characteristics, it is preferable for the flat plate-like metal fine particles to have a flat plate shape and have a particle size d, a thickness h, and an aspect ratio in the following ranges.

The average value of the particle size d of the flat plate-like metal fine particles is preferably 2 nm or more and 1000 nm or less, more preferably 20 nm or more and 500 nm or less, and still more preferably 20 nm or more and 400 nm or less.

The average value of the thickness h of the flat plate-like metal fine particles, i.e., the average value of the distance h between the front surface and the back surface of the flat plate-like metal fine particles, is preferably 1 nm or more and 100 nm or less, and more preferably 5 nm or more and 50 nm or less.

The above average values are obtained, for example, by measuring the values of 100 particles.

The average aspect ratio (average value of d/average value of h) of the flat plate-like metal fine particles is 1.1 or more, preferably 2.0 or more, more preferably 2.0 or more and 100 or less, and still more preferably 2.0 or more and 50 or less. The color tone of the flat plate-like metal fine particles can be changed by arbitrarily changing the average value of the particle size d to control the average aspect ratio of the flat plate-like metal fine particles.

The shape and size of the flat plate-like metal fine particles can be evaluated using a scanning electron microscope, a transmission electron microscope, or a scanning transmission electron microscope.

As the methods of measuring the particle size d and the thickness h of the flat plate-like metal fine particles and the method of calculating the aspect ratio of the flat plate-like metal fine particles, for example, the following methods may be used.

(1) Method of measuring particle size

A dry powder of the personal care particles is observed with a scanning transmission electron microscope to obtain a scanning transmission electron microscope image. In the scanning transmission electron microscope image, the diameter of a flat plate-like metal fine particle approximated by a circle is calculated as the particle size of the flat plate-like metal fine particle in the plane direction. The average value is obtained by measuring the particle size of 100 particles.

(2) Method of measuring average thickness

A dry powder of the personal care particles fixed with an embedding resin is cut in the cross-sectional direction using a microtome, and is observed with a transmission electron microscope for measurement. In the transmission electron microscope image, the thickness h of a flat plate-like metal fine particle is calculated as the thickness of the flat plate-like metal fine particle perpendicular to the plane direction. The average value of the thickness h (average thickness) is obtained by measuring the thickness of 100 particles.

(3) Method of calculating average aspect ratio

The ratio of the average value of the diameter d (equivalent circle particle diameter) of the flat plate-like metal fine particles to the average value of the thickness h of the flat plate-like metal fine particles obtained as described above is calculated as the average aspect ratio (average value of d/average value of h) of the flat plate-like metal fine particles.

The methods of measuring the particle size d and the thickness h of the flat plate-like metal fine particles and the method of calculating the average aspect ratio of the flat plate-like metal fine particles described above are merely examples, and the methods of measuring the particle size and the thickness of the flat plate-like metal fine particles and the method of calculating the aspect ratio of the flat plate-like metal fine particles are not limited to these methods.

The metal or metal compound constituting the flat plate-like metal fine particles is not particularly limited, and may be any metal or metal compound. Examples of the metal or metal compound constituting the flat plate-like metal fine particles include metals such as gold, silver, copper, aluminum, iron, platinum, zinc, palladium, ruthenium, iridium, rhodium, osmium, chromium, cobalt, nickel, manganese, vanadium, molybdenum, gallium, and aluminum, metal salts and metal complexes thereof, alloys thereof, and oxides and multiple oxides of these metals. Of these, the flat plate-like metal fine particles preferably contain at least one of gold, silver, and copper. In particular, the flat plate-like metal fine particles containing at least silver can shield light having a wavelength in the visible light range to the near-infrared range, and can impart antibacterial properties.

The ratio of the metal contained in the flat plate-like metal fine particles is not particularly limited.

In the method (production method III) shown in Fig. 5, first, the functional material 40 is supported by the fine fibers 3A supporting no material to produce the fine fibers 3B supporting the functional material. Next, the fine fibers 3B supporting the functional material are used to prepare an O/W Pickering emulsion having the droplets 6A containing the core material. Next, the core material in the droplets 6A of the emulsion is solidified to produce particles (personal care particles 1A) in which the core particles 2A are coated with the fine fibers 3B supporting the functional material.

In the method (production method IV) shown in Fig. 6, first, the fine fibers 3A supporting no material are used to produce an O/W Pickering emulsion having the droplets 6A containing the core material. Next, the core material in the droplets 6A of the emulsion is solidified to produce the particles 10A coated with the fine fibers 3A. Next, the functional material 40 is supported by the fine fibers 3A on the surface of the particles 10A to produce particles (personal care particles 1A) coated with the fine fibers 3B supporting the functional material 40.

The method of causing the fine fibers to support a functional material is not particularly limited, and for example, a commercially available or previously produced functional material may be added and mixed in a dispersion liquid of the fine fibers or a dispersion liquid of the particles coated with the fine fibers to attach the functional material to the fine fibers.

The preferable method of causing the fine fibers to support a functional material is a method of causing the fine fibers to support a functional material in a dispersion liquid of the fine fibers or a dispersion liquid of the particles coated with the fine fibers. When a functional material is supported by the fine fibers in a dispersion liquid of the fine fibers or a dispersion liquid of the particles coated with the fine fibers, the functional material is stably supported by the fine fibers, and thus aggregation of the functional material can be prevented.

The core particles constituting the personal care particles of the present embodiment are made of a material containing one or more types of polymers. The polymer may be a known polymer, and may be a polymer obtained by polymerizing a polymerizable monomer by a known method. That is, the material for forming the core particles of the personal care particles of the present embodiment may be any of the polymers exemplified in the first embodiment.

The core particles constituting the personal care particles of the present embodiment may include an additional component, such as a functional component, other than the polymer. Examples of the functional component include colorants, oil absorbing agents, light shielding agents (ultraviolet absorbers, ultraviolet scattering agents, etc.), antibacterial agents, antioxidants, antiperspirants, antifoaming agents, antistatic agents, binding agents, bleaching agents, chelating agents, deodorizing components, aromatics, perfumes, dandruff preventing active substances, skin softeners, insect repellents, preservatives, natural extracts, beauty components, pH adjusting agents, vitamins, amino acids, hormones, oil-based raw materials such as fats, oils, and waxes, surfactants, and inorganic particles (titanium oxide, silica, clay, etc.).

The functional component may be in the form of a solid, gas, or liquid. The content ratio of the functional component in the personal care particles is not particularly limited, but is preferably in a range in which the personal care particles can stably maintain their shape. The content ratio of the functional component is preferably 0.001 parts by mass or more and 80 parts by mass or less with respect to 100 parts by mass of the personal care particles.

<Method of producing personal care particles>

As described above, the personal care particles of the embodiment can be produced by the methods shown in Figs. 5 and 6 (production method III and production method IV).

In the production method III and the production method IV, a dispersion liquid containing the personal care particles is obtained. By removing the solvent from the dispersion liquid, the personal care particles are obtained as a dry solid. The method of removing the solvent is not particularly limited, and the personal care particles can be obtained as a dry solid, for example, by removing excess moisture by centrifugation or filtration, followed by heat drying in an oven. In this case, the obtained dry solid is not in the form of a film or an aggregate, and is obtained as a fine and smooth powder.

On the other hand, it is known that when the solvent is removed from the dispersion liquid of the fine fibers by a common method different from the production method III or the production method IV, the fine fibers are strongly aggregated or formed into a film.

In the dispersion liquid containing the personal care particles obtained by the production method III and the production method IV, the fine fibers are fixed to the surface of the personal care particles having a spherical shape; thus, even when the solvent is removed, the fine fibers do not become aggregated and the personal care particles are only in point contact with each other. This is presumably the reason why the dry solid is obtained as a fine powder. Accordingly, the personal care particles are not aggregated, thus achieving powder cosmetics having smoothness and good skin compatibility. Furthermore, aggregation of the functional material can be prevented, thus achieving uniform powder cosmetics.

The dry powder of the personal care particles can be a dry solid that contains almost no solvent and is redispersible in a solvent. Specifically, the dry powder of the personal care particles can have a solid content ratio of 80% or more, 90% or more, or 95% or more. The personal care particles from which the solvent is almost completely removed is preferable from the viewpoint of reduction of transportation costs, prevention of decay, improvement of the addition ratio, and improvement of the kneading efficiency with resin.

Due to the high hydrophilicity of the fine fibers on the surface of the personal care particles, the dry powder of the personal care particles has an advantage such as absorbing moisture such as sweat or exhibiting high moisturizing properties.

In addition, the personal care particles obtained as a dry powder are easily redispersed in a solvent again, and even after redispersion, the personal care particles exhibit dispersion stability derived from the fine fibers bonded to the surface of the personal care particles. Thus, the dry powder of the personal care particles can be contained in beauty essences, milky lotions, creams, and the like.

Each step will be described below in detail.

As described above, the production method III includes the first step, the second-iii step, the second-iv step, and the third-ii step, and the production method IV includes the first step, the second-iv step, the third-ii step, and the fifth step.

(First step)

The first step is a step of fibrillating a fine fiber raw material in a solvent to obtain a dispersion liquid of fine fibers. The fine fiber raw material is a cellulose raw material which is a raw material of cellulose nanofibers and/or a chitin/chitosan raw material which is a raw material of chitin nanofibers. That is, the first step of the present embodiment is the same as the first step of the first embodiment.

(Second-iii step)

The second-iii step is a step of causing the fine fibers in the dispersion liquid of the fine fibers to support a functional material to obtain a dispersion liquid of the fine fibers supporting the functional material.

The method of adding a functional material to the fine fibers is not particularly limited, and a known method may be used. For example, a functional material may be added and mixed in the dispersion liquid of the fine fibers to attach the functional material to the fine fibers. Alternatively, a functional material may be synthesized in the dispersion liquid of the fine fibers to cause the fine fibers to support the functional material.

As described above, when a functional material is supported by the fine fibers, it is preferable to introduce in advance an anionic functional group to the crystal surface of the fine fibers. When a cationic functional material is added and mixed in a dispersion liquid of the fine fibers having an anionic functional group, the functional material can be stably supported by the fine fibers. By adjusting the pH of the dispersion liquid, it is possible to control support or removal of the functional material.

The concentration of the fine fibers for supporting a functional material is not particularly limited, but is preferably 0.1 mass% or more and 20 mass% or less, and more preferably 0.5 mass% or more and 10 mass% or less. If the concentration of the fine fibers is less than 0.1%, it is difficult for the fine fibers to efficiently support the functional material. If the concentration of the fine fibers B exceeds 20 mass%, the fine fibers have a very high viscosity and cannot be sufficiently stirred, and this makes it difficult to achieve a uniform reaction.

The solvent of the dispersion liquid of the fine fibers is not particularly limited, but is preferably the hydrophilic solvent 7.

The hydrophilic solvent 7 is not particularly limited, but is preferably, water; an alcohol such as methanol, ethanol, or isopropanol; or a cyclic ether such as tetrahydrofuran.

In particular, the suspension preferably contains 50 mass% or more of water. If the ratio of water in the suspension is less than 50 mass%, the fine fibers may have poor dispersibility. A solvent other than water contained in the suspension is preferably any of the hydrophilic solvents described above.

If necessary, the pH of the suspension may be adjusted in order to improve the dispersibility of the fine fiber raw material and the fine fibers. Examples of an alkaline aqueous solution used for pH adjustment include organic alkali such as a sodium hydroxide aqueous solution, a lithium hydroxide aqueous solution, a potassium hydroxide aqueous solution, an ammonia aqueous solution, a tetramethylammonium hydroxide aqueous solution, a tetraethylammonium hydroxide aqueous solution, a tetrabutylammonium hydroxide aqueous solution, and a benzyltrimethylammonium hydroxide aqueous solution. A sodium hydroxide aqueous solution is preferable in terms of cost and the like.

The method in which metal fine particles are supported as a functional material by cellulose nanofibers having an anionic functional group is not particularly limited, and for example, the following method may be used. The method includes step ia, step ib, and step ic in this order.

Step ia: Cellulose nanofiber dispersion liquid preparing step of preparing a dispersion liquid containing at least one type of cellulose nanofibers.

Step ib: Metal salt/cellulose nanofiber-containing liquid preparing step of preparing a dispersion liquid containing at least one type of metal salt and at least one type of cellulose nanofibers.

Step ic: Metal fine particle-supporting cellulose nanofiber dispersion liquid preparation step of reducing metal ions in the metal salt/cellulose nanofiber-containing liquid to cause the cellulose nanofibers to support metal fine particles.

(Step ia: Cellulose nanofiber dispersion liquid preparing step)

In the method of causing the cellulose nanofibers to support metal fine particles, in step ia, a dispersion liquid of at least one type of cellulose nanofibers is prepared.

First, a cellulose nanofiber dispersion liquid is prepared. The solid concentration of the cellulose nanofiber dispersion liquid is not particularly limited, but is preferably 0.1 mass% or more and 20 mass% or less, and more preferably 0.5 mass% or more and 10 mass%.

If the solid concentration of the cellulose nanofiber dispersion liquid is less than 0.1 mass%, it is difficult to control the particle size of the metal fine particles. On the other hand, if the solid concentration of the cellulose nanofiber dispersion liquid exceeds 20 mass%, the cellulose nanofiber dispersion liquid has a high viscosity, and this makes it difficult to uniformly mix the metal salt with the cellulose nanofibers in step ib (metal salt/cellulose nanofiber-containing liquid preparing step), thus preventing the reduction reaction from proceeding uniformly in step ic (metal fine particle-supporting cellulose nanofiber dispersion liquid preparation step).

The solvent in which the cellulose nanofibers are dispersed is not particularly limited as long as the cellulose nanofibers sufficiently dissolve or disperse in the solvent. From the viewpoint of the environmental load, the solvent is preferably water.

From the viewpoint of the dispersibility of the metal fine particles and the cellulose nanofibers, the solvent is preferably water or a hydrophilic solvent. The hydrophilic solvent is not particularly limited, but is preferably an alcohol such as methanol, ethanol, or isopropanol.

The pH of the dispersion liquid of at least one type of cellulose nanofibers is not particularly limited, but is preferably pH 2 or more and pH 12 or less.

(Step ib: Metal salt/cellulose nanofiber-containing liquid preparing step)

In the method of causing the cellulose nanofibers to support metal fine particles, in step ib, a dispersion liquid containing at least one type of metal salt and at least one type of cellulose nanofibers is prepared to obtain a metal salt/cellulose nanofiber-containing liquid.

The method of preparing a solution or dispersion liquid containing at least one type of metal salt and at least one type of cellulose nanofibers is not particularly limited. For example, the solution or dispersion liquid may be prepared by preparing a dispersion liquid containing at least one type of cellulose nanofibers (cellulose nanofiber dispersion liquid) and a solution containing at least one type of metal salt (metal salt containing solution), and adding the metal salt containing solution to the cellulose nanofiber dispersion liquid while stirring the cellulose nanofiber dispersion liquid. Alternatively, a solid metal salt may be directly added to the cellulose nanofiber dispersion liquid, or the cellulose nanofiber dispersion liquid may be added to the metal salt containing solution.

The metal salt is preferably at least one selected from the group consisting of silver nitrate, silver chloride, silver oxide, silver sulfate, silver acetate, silver nitrite, silver chlorate, chloroauric acid, sodium chloroaurate, potassium chloroaurate, platinum chloride, platinum oxide, and platinum oxide. These metal salts may be used alone or in combination of two or more.

When the metal salt containing solution is prepared, the solvent used for the metal salt containing solution is not particularly limited as long as the metal salt is sufficiently dispersed or dissolved in the solvent.

From the viewpoint of the environmental load, the solvent is preferably water.

From the viewpoint of the solubility of the metal salt, the solvent is preferably water or a hydrophilic solvent. The hydrophilic solvent is not particularly limited, but is preferably an alcohol such as methanol, ethanol, or isopropanol.

The concentration of the metal salt in the metal salt containing solution is not particularly limited.

The concentration of the metal salt in the metal salt/cellulose nanofiber dispersion liquid is not particularly limited, but is preferably 0.002 mmol/L or more and 20.0 mmol/L or less. In particular, when cellulose nanofibers having an anionic functional group are used, metal ions are coordinated to the anionic functional group; thus, it is preferable to prepare the metal salt/cellulose nanofiber dispersion liquid so that the concentration of the metal salt (the concentration of the metal ions) is less than the amount of anionic functional group. If the concentration of the metal salt (the concentration of the metal ions) exceeds the amount of the anionic functional group of the cellulose nanofibers, the cellulose nanofibers may be aggregated.

The solvent used for the metal salt/cellulose nanofiber-containing liquid is not particularly limited as long as the cellulose nanofibers are sufficiently dispersed or dissolved in the solvent.

From the viewpoint of the environmental load, the solvent is preferably water.

From the viewpoint of the dispersibility of the cellulose nanofibers, the solvent is preferably water or a hydrophilic solvent. The hydrophilic solvent is not particularly limited, but is preferably an alcohol such as methanol, ethanol, or isopropanol.

The solid concentration of the cellulose nanofibers in the metal salt/cellulose nanofiber dispersion liquid is not particularly limited, but is preferably 0.1 mass% or more and 20 mass% or less, and more preferably 0.1 mass% or more and 50 mass% or less.

If the solid concentration of the cellulose nanofibers is less than 0.1 mass%, it is difficult to control the particle size of the metal fine particles. On the other hand, if the solid concentration of the cellulose nanofibers exceeds 20 mass%, the cellulose nanofiber dispersion liquid has a high viscosity, and this makes it difficult to uniformly mix the metal salt with the cellulose nanofibers in step ib, thus preventing the reduction reaction from proceeding uniformly in step ic.

The solvent of the metal salt/cellulose nanofiber-containing liquid is not particularly limited as long as the cellulose nanofibers sufficiently dissolve or disperse in the solvent.

From the viewpoint of the environmental load, the solvent is preferably water.

From the viewpoint of the dispersibility of the metal fine particles and the cellulose nanofibers, the solvent is preferably water or a hydrophilic solvent. The hydrophilic solvent is not particularly limited, but is preferably an alcohol such as methanol, ethanol, or isopropanol.

The pH of the metal salt/cellulose nanofiber-containing liquid is not particularly limited, but is preferably pH 2 or more and pH 12 or less.

The temperature of the metal salt/cellulose nanofiber-containing liquid is not particularly limited, but is preferably 4°C or more and 100°C or less when water is used as a solvent.

(Step ic: Metal fine particle-supporting cellulose nanofiber dispersion liquid preparation step)

In the method of causing the cellulose nanofibers to support metal fine particles, in step ic, metal ions in the metal salt/cellulose nanofiber-containing liquid are reduced and reacted to deposit metal fine particles and control the shape of the metal fine particles, thereby obtaining cellulose nanofibers supporting the metal fine particles having a desired shape.

The method of reducing the metal ions in the metal salt/cellulose nanofiber-containing liquid is not particularly limited, and a known method may be used. For example, the metal ions may be reduced by using a reducing agent, ultraviolet light, electron beams, in-liquid plasma, or the like. The reducing agent used to reduce the metal ions may be a known reducing agent.

Examples of the reducing agent include metal hydride reducing agents, borohydride reducing agents, borane reducing agents, silane reducing agents, hydrazine reducing agents, and hydrazide reducing agents. Examples of the reducing agent generally used in a liquid phase reduction method include sodium borohydride, dimethylamine borane, sodium citrate, ascorbic acid, ascorbic acid alkali metal salt, and hydrazine.

The amount of reducing agent added to the metal salt/cellulose nanofiber-containing liquid (the concentration of the reducing agent) is not particularly limited, but is preferably equal to or higher than the concentration of the metal salt in the metal salt/cellulose nanofiber-containing liquid, and more preferably 0.002 mmol/L or more and 2000 mmol/L or less.

If the concentration of the reducing agent in the metal salt/cellulose nanofiber-containing liquid is less than or equal to the concentration of the metal salt in the metal salt/cellulose nanofiber-containing liquid, unreduced metal ions remain in the metal salt/cellulose nanofiber-containing liquid.

When the reducing agent is used to reduce the metal ions in the metal salt/cellulose nanofiber-containing liquid, the method of adding the reducing agent is not particularly limited, and the reducing agent may be added by dissolving or dispersing the reducing agent in advance in a solvent such as water to obtain a solution or a dispersion liquid, and adding the solution or the dispersion liquid to the metal salt/cellulose nanofiber-containing liquid.

The rate of addition of the reducing agent to the metal salt/cellulose nanofiber-containing liquid is not particularly limited, but the reducing agent is preferably added in such a manner that the reduction reaction proceeds uniformly.

The method of causing the cellulose nanofibers to support metal fine particles is not particularly limited, but preferably includes at least step ia, step ib, and step ic described above. Another step may be performed between these steps.

If necessary, at least part of the surface of the metal fine particles may be coated with a semiconductor, metal, or a metal oxide thereof. Examples of the semiconductor, metal, and metal oxide thereof used for coating include, but are not particularly limited to, metals such as gold, silver, copper, aluminum, iron, platinum, zinc, palladium, ruthenium, iridium, rhodium, osmium, chromium, cobalt, nickel, manganese, vanadium, molybdenum, gallium, and aluminum, metal salts and metal complexes thereof, alloys thereof, oxides and multiple oxides of these metals, and silica. In particular, from the viewpoint of stability and versatility, it is preferable to use gold or silica for coating.

If necessary, the functional material-supporting cellulose nanofibers may be washed by filtration, centrifugation, or the like to remove free functional materials.

(Second-iv step)

The second-iv step is a step of coating, with the fine fibers (the fine fibers 3B supporting the functional material 40 as shown in Fig. 5 in the production method III, the fine fibers 3A supporting no functional material 40 as shown in Fig. 6 in the production method IV), the surface of the droplets containing the core material in the dispersion liquid of the fine fibers to stabilize the droplets as an emulsion.

Specifically, in the second-iv step, the core material-containing liquid is added to the dispersion liquid of the fine fibers 3B supporting the functional material (dispersion liquid 11B in Fig. 5) obtained in the second-iii step in the production method III, and the core material-containing liquid is added to the dispersion liquid of the fine fibers 3A (dispersion liquid 11A in Fig. 6) obtained in the first step in the production method IV, and then the core material is dispersed as the droplets 6A in the dispersion liquid, and the surface of the droplets 6A is coated with the fine fibers to produce an O/W emulsion stabilized by the fine fibers. Hereinafter, the O/W emulsion stabilized by the fine fibers may be simply referred to as an "emulsion liquid".

The method of producing an O/W emulsion is not particularly limited, and may be any of the methods exemplified in the first embodiment.

By ultrasonic treatment, droplets containing a core material are dispersed in the dispersion liquid of the fine fibers and emulsified, and the fine fibers are selectively adsorbed on the liquid-liquid interface between the droplets and the fine fiber dispersion liquid, and thus the droplets are coated with the fine fibers to form a stable structure as an O/W emulsion. Such an emulsion that is stabilized by the adsorption of a solid material on the liquid-liquid interface is academically called a "Pickering emulsion".

Although, as described above, the mechanism of how the fine fibers form a Pickering emulsion is not clear, presumably, since cellulose has a hydrophilic site derived from a hydroxyl group and a hydrophobic site derived from a hydrocarbon group in its molecular structure and thus exhibits amphipathic properties, due to the amphipathic properties, the fine fibers are adsorbed on the liquid-liquid interface between the hydrophobic monomer and the hydrophilic solvent.

The O/W emulsion structure can be confirmed by observation with an optical microscope. The particle size of the O/W emulsion is not particularly limited, but the average particle size is preferably 0.1 $\mu$m or more and 1000 $\mu$m or less. The average particle size can be calculated by measuring the diameter of 100 randomly selected emulsions, and taking the average value of the diameter of the emulsions.

In the O/W emulsion structure, the thickness of the coating layer of the fine fibers formed on the surface layer of the droplets containing the core material is not particularly limited, but is preferably 3 nm or more and 1000 nm or less. Although not particularly limited, the particle size of the emulsion structure is approximately the same as the particle size of the personal care particles obtained in the third-ii step. The thickness of the coating layer can be measured, for example, by using a cryo-TEM.

The weight ratio between the fine fiber dispersion liquid and the core material used in the second-iv step is not particularly limited, but the weight ratio of the polymerizable monomer is preferably 1 part by mass or more and 50 parts by mass or less with respect to 100 parts by mass of the fine fibers. If the weight ratio of the polymerizable monomer is 1 part by mass or less, the yield of personal care particles is reduced, which is not preferable. If the weight ratio of the polymerizable monomer exceeds 50 parts by mass, it is difficult to uniformly coat the droplets with the fine fibers, which is not preferable.

The core material-containing liquid to be used may be one that contains a core material and is able to form an O/W emulsion, and is preferably hydrophobic in order to stably form an O/W emulsion. The core material is a material that is solidified by a chemical or physicochemical change to form the core particles 2A. The core material is not particularly limited as long as the core material can stably form droplets. The core material may be, for example, a polymerizable monomer (monomer), a molten polymer, or a dissolved polymer.

The polymerizable monomer used in the second-iv step is not particularly limited, and may be any of the polymerizable monomers exemplified as the polymerizable monomer used in the second-i step of the first embodiment.

Furthermore, any of the polymerization initiators exemplified in the first embodiment may be added to the polymerizable monomer.

In the case where a core material-containing liquid containing a polymerizable monomer and a polymerization initiator is used in the second-iv step, the polymerization initiator is contained in the droplets of the O/W emulsion in the third-ii step (described later), thus facilitating a polymerization reaction when the monomer in the droplets of the emulsion is polymerized in the third-ii step (described later).

The weight ratio between the polymerizable monomer and the polymerization initiator used in the second-iv step is not particularly limited, but in general, the weight ratio of the polymerization initiator is preferably 0.1 parts by mass or more with respect to 100 parts by mass of the polymerizable monomer. If the weight ratio of the polymerizable monomer is less than 0.1 parts by mass, the polymerization reaction does not sufficiently proceed, resulting in a lower yield of personal care particles, which is not preferable.

The core material-containing liquid may contain a solvent. Although not particularly limited, it is preferable to use an organic solvent in order to stabilize the emulsion in the second-iv step. Examples of the organic solvent include toluene, xylene, ethyl acetate, butyl acetate, methyl ethyl ketone (MEK), methyl isobutyl ketone (MIBK), isophorone, cellosolve acetate, isophorone, Solvesso 100, trichlene, hexane, chloroform, dichloromethane, dichloroethane, isooctane, and nonane.

The weight ratio between the polymerizable monomer and the solvent that can be used in the second-iv step is not particularly limited, but the weight ratio of the solvent is preferably 80 parts by mass or less with respect to 100 parts by mass of the polymerizable monomer.

The polymer used to obtain a dissolved polymer may be any of the polymers exemplified in the first embodiment.

The method in which the polymer is melted to obtain a molten polymer may be any of the methods exemplified in the first embodiment. The polymer used for the molten polymer may be any of the polymers exemplified in the first embodiment.

A functional component other than the polymerization initiator may be added in advance to the droplets containing the core material. Specific examples of the functional component include colorants, oil absorbing agents, light shielding agents (ultraviolet absorbers, ultraviolet scattering agents, etc.), antibacterial agents, antioxidants, antiperspirants, antifoaming agents, antistatic agents, binding agents, bleaching agents, chelating agents, deodorizing agents, aromatics, perfumes, dandruff preventing active substances, skin softeners, insect repellents, preservatives, natural extracts, beauty components, pH adjusting agents, vitamins, amino acids, hormones, oil-based raw materials such as fats, oils, and waxes, surfactants, inorganic particles (titanium oxide, silica, clay, etc.), and enzymes.

When a functional component other than the polymerization initiator is added in advance to the polymerizable monomer, the core particles of the formed personal care particles can contain the functional component, enabling the personal care particles to exhibit a function according to the application.

It is preferable for the functional component to easily dissolve or disperse in the droplets and be difficult to dissolve or disperse in the hydrophilic solvent. When the functional component dissolves or disperses in the droplets, the droplets of the formed O/W emulsion are more likely to contain the functional component, and this makes it possible to efficiently obtain personal care particles containing the functional component. The amount of functional component to be contained in the personal care particles may be increased.

Furthermore, a polymerizable monomer, a dissolved polymer, and a molten polymer may be used in combination as a core material to form droplets and emulsify the droplets. When a biodegradable polymer (resin) is selected as a polymer of the core particles of the personal care particles, the resulting personal care particles are composed of the core particles and the fine fibers that are made of a biodegradable polymer, thus achieving the personal care particles that contain a biodegradable material and are highly environmentally friendly.

(Third-ii step)

The third-ii step is a step of solidifying the core material in the droplets to obtain a dispersion liquid of particles in which the surface of the core particles is coated with the fine fibers (the fine fibers 3B supporting the functional material 40 as shown in Fig. 5 in the production method III, the fine fibers 3A supporting no functional material 40 as shown in Fig. 6 in the production method IV).

The method of solidifying the core material is not particularly limited. When a polymerizable monomer is used as a core material, the core material can be solidified by polymerizing the polymerizable monomer to form a polymerized monomer. When a dissolved polymer is used as a core material, the polymer can be solidified by removing the solvent using a method in which the solvent in the droplets is dispersed in a hydrophilic solvent or a method in which the solvent is evaporated. When a molten polymer is used as a core material, the core material can be solidified by solidifying the molten polymer by cooling.

For example, by stirring and heating the O/W emulsion produced in the second-iv step in which the droplets containing the polymerizable monomer as a core material and the polymerization initiator are coated with the fine fibers and stabilized, the polymerizable monomer is polymerized, thereby solidifying the core material. The stirring method is not particularly limited, and may be a known method. Specifically, a disperser or a stirring bar may be used. Alternatively, the O/W emulsion may be only heated without being stirred.

The temperature for heating the O/W emulsion may be appropriately set according to the types of polymerizable monomer and polymerization initiator, but the temperature is preferably 20°C or more and 150°C or less. If the temperature is less than 20°C, the reaction rate of polymerization is reduced, which is not preferable. If the temperature exceeds 150°C, the fine fibers may be modified, which is not preferable. The time for the polymerization reaction may be appropriately set according to the types of polymerizable monomer and polymerization initiator, but is typically approximately 1 to 24 hours. The polymerization reaction may be performed by ultraviolet irradiation treatment, i.e., using a type of electromagnetic waves. Other than electromagnetic waves, particle beams such as electron beams may be used.

Specific examples of the method of evaporating the solvent of the dissolved polymer include a method in which the solvent is evaporated to be removed by heating or/and drying under reduced pressure. When an organic solvent having a boiling point lower than that of water is used, the organic solvent can be selectively removed. Although not particularly limited, the solvent can be efficiently removed by heating under reduced pressure. The heating temperature is preferably 20°C or more and 100°C or less, and the pressure is preferably 600 mHg or more and 750 mmHg or less.

Specific examples of the method of dispersing the solvent of the dissolved polymer include a method in which the solvent in the droplets is dispersed by adding a solvent or salt to the O/W emulsion liquid. When the solvent having low solubility in the hydrophilic solvent is dispersed over time in the hydrophilic solvent phase, the dissolved polymer can be deposited and solidified as particles.

Examples of the method of solidifying the molten polymer include a method in which the molten polymer is solidified by cooling the O/W emulsion liquid.

Through the steps described above, the dispersion liquid of the particles in which the core particles are coated with the fine fibers (the personal care particles 1A shown in Fig. 5 (d) in the production method III, the particles 10A coated with the fine fibers shown in Fig. 6 (c) in the production method IV) is obtained.

In the production method III, immediately after the personal care particles 1A are generated, the dispersion liquid of the personal care particles 1A contains a mixture of a large amount of water and free fine fibers that do not contribute to the formation of the coating layer. Thus, the personal care particles 1A need to be collected and washed. In the production method IV, immediately after the particles 10A coated with the fine fibers are generated, the dispersion liquid of the particles 10A contains a mixture of a large amount of water and free fine fibers that do not contribute to the formation of the coating layer. Thus, the particles 10A need to be collected and washed.

The collection and washing are preferably performed by centrifugation washing or filtration washing. Furthermore, the residual solvent may be removed.

The centrifugation washing may be performed by a known method. Specifically, the personal care particles 1A or the particles 10A can be collected by repeating the operation in which the personal care particles 1A or the particles 10A are precipitated by centrifugation to remove the supernatant and the particles are redispersed in a mixed solvent of water and methanol, and removing the residual solvent from the precipitate finally obtained by the centrifugation. The filtration washing may also be performed by a known method. The personal care particles 1A or the particles 10A can be collected, for example, by repeating suction filtration with water and methanol using a PTFE membrane filter with a pore diameter of 0.1 μm, and removing the residual solvent from the paste that finally remains on the membrane filter.

The method of removing the residual solvent is not particularly limited, and can be performed by air drying or heat drying in an oven. The dry solid containing the personal care particles 1A obtained in this manner is not in the form of a film or an aggregate, and is obtained as a fine powder as described above.

(Fifth step)

The fifth step is a step of causing the fine fibers 3A on the surface of the particles 10A coated with the fine fibers 3A in the dispersion liquid of the particles 10A shown in Fig. 6 (c) to support the functional material 40 in the production method IV.

The method of causing the fine fibers 3A on the surface of the particles 10A to support the functional material is not particularly limited, and a known method may be used. For example, it is possible to use a method in which the particles coated with the fine fibers are redispersed in a hydrophilic solvent such as water or alcohol to produce a dispersion liquid of the particles, and a functional material is added and mixed in the dispersion liquid to attach the functional material to the particles. Alternatively, a functional material may be synthesized in the dispersion liquid of the particles coated with the fine fibers to cause the particles coated with the fine fibers to support the functional material.

As described above, when a functional material is supported by the particles coated with the fine fibers, it is preferable to introduce in advance an anionic functional group to the crystalline surface of the fine fibers. When a cationic functional material is added and mixed in a dispersion liquid of the particles coated with the fine fibers having an anionic functional group, the functional material can be stably supported by the fine fibers on the particles coated with the fine fibers. By adjusting the pH of the dispersion liquid of the particles coated with the fine fibers, it is possible to control support or removal of the functional material.

The concentration of the particles coated with the fine fibers in the dispersion liquid used to support the functional material is not particularly limited, but is preferably 0.1 mass% or more and 50 mass% or less, and more preferably 0.5 mass% or more and 20 mass% or less. If the concentration is less than 0.1%, it is difficult to efficiently support the functional material. If the concentration exceeds 50 mass%, the particles coated with the fine fibers have poor dispersibility, and this makes it difficult to achieve a uniform reaction.

The solvent of the dispersion liquid of the particles coated with the fine fibers is not particularly limited, but is preferably a hydrophilic solvent.

The hydrophilic solvent is not particularly limited, but is preferably, water; an alcohol such as methanol, ethanol, or isopropanol; or a cyclic ether such as tetrahydrofuran.

In particular, the suspension preferably contains 50 mass% or more of water. If the ratio of water in the suspension is less than 50 mass%, the particles coated with the fine fibers may have poor dispersibility. A solvent other than water contained in the suspension is preferably any of the hydrophilic solvents described above.

If necessary, the pH of the suspension may be adjusted in order to improve the dispersibility of the particles coated with the fine fibers. Examples of an alkaline aqueous solution used for pH adjustment include organic alkali such as a sodium hydroxide aqueous solution, a lithium hydroxide aqueous solution, a potassium hydroxide aqueous solution, an ammonia aqueous solution, a tetramethylammonium hydroxide aqueous solution, a tetraethylammonium hydroxide aqueous solution, a tetrabutylammonium hydroxide aqueous solution, and a benzyltrimethylammonium hydroxide aqueous solution. A sodium hydroxide aqueous solution is preferable in terms of cost and the like.

The method in which metal fine particles are supported as a functional material by particles coated with cellulose nanofibers having an anionic functional group as fine fibers is not particularly limited, and for example, the following method may be used. The method includes step iia, step iib, and step iic in this order.

Step iia: Cellulose nanofiber coated particle dispersion liquid preparing step of preparing a dispersion liquid containing at least one type of cellulose nanofiber coated particles.

Step iib: Metal salt/cellulose nanofiber coated particle-containing liquid preparing step of preparing a dispersion liquid containing at least one type of metal salt and at least one type of cellulose nanofiber coated particles.

Step iic: Metal fine particle-supporting cellulose nanofiber coated particle dispersion liquid preparation step of reducing metal ions in the metal salt/cellulose nanofiber coated particle-containing liquid to prepare a reaction liquid.

(Step iia: Cellulose nanofiber coated particle dispersion liquid preparing step)

In the method of causing the cellulose nanofiber coated particles to support metal fine particles, in step iia, a dispersion liquid of at least one type of cellulose nanofiber coated particles is prepared.

First, a cellulose nanofiber coated particle dispersion liquid is prepared. The solid concentration of the cellulose nanofiber coated particle dispersion liquid is not particularly limited, but is preferably 0.1 mass% or more and 50 mass% or less, and more preferably 0.5 mass% or more and 20 mass%.

If the solid concentration of the cellulose nanofiber coated particle dispersion liquid is less than 0.01 mass%, it is difficult to control the particle size of the metal fine particles. On the other hand, if the solid concentration of the cellulose nanofiber coated particle dispersion liquid exceeds 50 mass%, the cellulose nanofiber coated particles have poor dispersibility, and this makes it difficult to uniformly mix the metal salt with the cellulose nanofiber coated particles in step iib (metal salt/cellulose nanofiber-containing liquid preparing step), thus preventing the reduction reaction from proceeding uniformly in step iic (metal fine particle-supporting cellulose nanofiber coated particle dispersion liquid preparation step).

The solvent in which the cellulose nanofiber coated particles are dispersed and the pH of the dispersion liquid are the same as those in step ia.

(Step iib: Metal salt/cellulose nanofiber coated particle-containing liquid preparing step)

In the method of causing the cellulose nanofiber coated particles to support metal fine particles, in step iib, a dispersion liquid containing at least one type of metal salt and at least one type of cellulose nanofiber coated particles is prepared.

The method of preparing the dispersion liquid containing at least one type of metal salt and at least one type of cellulose nanofiber coated particles is not particularly limited. For example, the dispersion liquid may be prepared by preparing a dispersion liquid containing at least one type of cellulose nanofiber coated particles and a solution containing at least one type of metal salt (metal salt containing solution), and adding the metal salt containing solution to the cellulose nanofiber coated particle dispersion liquid while stirring the cellulose nanofiber coated particle dispersion liquid. Alternatively, a solid metal salt may be directly added to the cellulose nanofiber coated particle dispersion liquid, or the cellulose nanofiber coated particle dispersion liquid may be added to the metal salt containing solution.

The metal salt and the metal salt-containing liquid can be prepared in the same manner as in step ib.

The concentration of the metal salt in the metal salt/cellulose nanofiber coated particle dispersion liquid is not particularly limited, but is preferably 0.002 mmol/L or more and 20.0 mmol/L or less. In particular, when cellulose nanofiber coated particles having an anionic functional group are used, metal ions are coordinated to the anionic functional group; thus, it is preferable to prepare the metal salt/cellulose nanofiber coated particle dispersion liquid so that the concentration of the metal salt (the concentration of the metal ions) is less than the amount of anionic functional group. If the concentration of the metal salt (the concentration of the metal ions) exceeds the amount of anionic functional group of the cellulose nanofiber coated particles, the cellulose nanofiber coated particles may be aggregated.

The solvent used for the metal salt/cellulose nanofiber coated particle-containing liquid may be the same as the solvent used in the second-iii step.

The solid concentration of the cellulose nanofiber coated particles in the metal salt/cellulose nanofiber coated particle dispersion liquid is not particularly limited, but is preferably 0.1 mass% or more and 50 mass% or less, and more preferably 0.1 mass% or more and 20 mass% or less.

If the solid concentration of the cellulose nanofiber coated particles is less than 0.1 mass%, it is difficult to control the particle size of the metal fine particles. On the other hand, if the solid concentration of the cellulose nanofibers exceeds 50 mass%, the cellulose nanofiber coated particles have poor dispersibility, and this makes it difficult to uniformly mix the metal salt with the cellulose nanofiber coated particles in step iib, thus preventing the reduction reaction from proceeding uniformly in step iic.

The solvent of the metal salt/cellulose nanofiber coated particle-containing liquid may be the same as the solvent used in step ib. The pH and the temperature of the liquid may be the same as those in the second-iii step.

(Step iic: Metal fine particle-supporting cellulose nanofiber coated particle dispersion liquid preparation step)

In the method of causing the cellulose nanofiber coated particles to support metal fine particles, in step iic, metal ions in the metal salt/cellulose nanofiber coated particle-containing liquid are reduced and reacted to deposit metal fine particles and control the shape of the metal fine particles, thereby obtaining cellulose nanofiber coated particles supporting the metal fine particles.

The method of reducing the metal ions in the metal salt/cellulose nanofiber coated particle-containing liquid may be the same as the method used in step ic, and the same reducing agent as that used in step ic may be used.

The amount of reducing agent added to the metal salt/cellulose nanofiber coated particle-containing liquid (the concentration of the reducing agent) is not particularly limited, but is preferably equal to or higher than the concentration of the metal salt in the metal salt/cellulose nanofiber coated particle-containing liquid, and more preferably 0.002 mmol/L or more and 2000 mmol/L or less.

If the concentration of the reducing agent in the metal salt/cellulose nanofiber coated particle-containing liquid is less than or equal to the concentration of the metal salt in the metal salt/cellulose nanofiber coated particle-containing liquid, unreduced metal ions remain in the metal salt/cellulose nanofiber coated particle-containing liquid.

When the reducing agent is used to reduce the metal ions in the metal salt/cellulose nanofiber coated particle-containing liquid, the method of adding the reducing agent is not particularly limited, and the reducing agent may be added by dissolving or dispersing the reducing agent in advance in a solvent such as water to obtain a solution or a dispersion liquid, and adding the solution or the dispersion liquid to the metal salt/cellulose nanofiber coated particle-containing liquid.

The rate of addition of the reducing agent to the metal salt/cellulose nanofiber coated particle-containing liquid is not particularly limited, but the reducing agent is preferably added in such a manner that the reduction reaction proceeds uniformly.

The method of causing the cellulose nanofiber coated particles to support metal fine particles is not particularly limited, but preferably includes at least step iia, step iib, and step iic described above. Another step may be performed between these steps.

If necessary, at least part of the surface of the metal fine particles may be coated with a semiconductor, metal, or a metal oxide thereof. Examples of the semiconductor, metal, and metal oxide thereof used for coating include, but are not particularly limited to, metals such as gold, silver, copper, aluminum, iron, platinum, zinc, palladium, ruthenium, iridium, rhodium, osmium, chromium, cobalt, nickel, manganese, vanadium, molybdenum, gallium, and aluminum, metal salts and metal complexes thereof, alloys thereof, oxides and multiple oxides of these metals, and silica. In particular, from the viewpoint of stability and versatility, it is preferable to use gold or silica for coating.

If necessary, the functional material-supporting cellulose nanofiber coated particles may be washed by filtration, centrifugation, or the like to remove free functional materials.

In the fifth step, the cellulose nanofiber coated particles may be used instead of the cellulose nanofibers in the second-iii step, and the same conditions may be applied unless otherwise specified.

A dry powder of the personal care particles of the present embodiment can be mixed or dispersed in other cosmetic raw materials, and used for personal care articles such as makeup products. Furthermore, the personal care particles can be dispersed in a solvent containing a cosmetic raw material and the like, and used as a personal care composition.

Examples of cosmetic raw materials include colorants, oil absorbing agents, light shielding agents (ultraviolet absorbers, ultraviolet scattering agents, etc.), antibacterial agents, antioxidants, antiperspirants, antifoaming agents, antistatic agents, binding agents, bleaching agents, chelating agents, deodorizing components, aromatics, perfumes, dandruff preventing active substances, skin softeners, insect repellents, preservatives, natural extracts, beauty components, pH adjusting agents, vitamins, amino acids, hormones, oil-based raw materials such as fats, oils, and waxes, surfactants, and inorganic particles (titanium oxide, silica, clay, etc.).

(Advantageous effects of personal care particles)

An aspect of the personal care particles of the present invention provides personal care particles having fine fibers, which are in a novel form that can be produced by a simple method, and solve the problems of the fine fibers, such as cellulose nanofibers or chitin nanofibers, having excessive solvent.

Furthermore, an aspect of the personal care particles of the present invention provides personal care particles that do not become aggregated, and have high smoothness, good skin compatibility, and good moisturizing properties, due to being coated with fine fibers having high safety, high hydrophilicity, and a high specific surface area.

Furthermore, an aspect of the personal care particles of the present invention provides personal care particles that due to a functional material being supported by fine fibers having a high specific surface area, maintain dispersion stability of the functional material, and effectively exhibit a function of the functional material such as ultraviolet light blocking (absorption and/or scattering), color development, or an antibacterial effect.

Cellulose nanofibers and chitin nanofibers are respectively made of cellulose and chitin/chitosan which are biodegradable polymers. Thus, the use of a material containing a biodegradable polymer to form core particles provides personal care particles that reduce the environmental load.

Embodiments of the present invention have been described in detail above. However, the specific configuration should not be limited to these embodiments, but should include design changes within the scope not departing from the spirit of the present invention. Furthermore, the components described in the above embodiments can be combined as appropriate. For example, the core particles may contain other components in addition to the polymer and the functional component.

[Examples]

The present invention will be described in detail below by way of examples, but the technical scope of the present invention is not limited to these examples. In the following examples, "%" indicates mass% (w/w%) unless otherwise noted.

[Examples 1 to 11, Comparative Examples 1 to 6]

<Example 1>

(First step: Step of obtaining cellulose nanofiber dispersion liquid)

{TEMPO oxidation of wood cellulose}

First, 70 g of softwood kraft pulp was suspended in 3500 g of distilled water, followed by adding a solution obtained by dissolving 0.7 g of TEMPO and 7 g of sodium bromide in 350 g of distilled water, followed by cooling to 20°C. To the resultant solution, 450 g of sodium hypochlorite aqueous solution with a concentration of 2 mol/L and a density of 1.15 g/mL was added dropwise to start an oxidation reaction. The temperature in the system was kept constant at 20°C. The pH during the reaction was maintained at pH 10 and prevented from being further reduced by adding a 0.5 N sodium hydroxide aqueous solution. When the total amount of added sodium hydroxide reached 3.0 mmol/g with respect to the weight of cellulose, approximately 100 mL of ethanol was added to stop the reaction. Then, filtration washing with distilled water was repeated by using a glass filter to obtain oxidized pulp (oxidized cellulose).

{Measurement of amount of carboxyl group of oxidized cellulose}

A solid content weight of 0.1 g of oxidized pulp and reoxidized pulp obtained by the TEMPO oxidation was weighed and dispersed in water at a concentration of 1%, followed by adding hydrochloric acid to adjust the pH to 2.5. Then, the amount of carboxyl group (mmol/g) was determined by conductometric titration using a 0.5 M sodium hydroxide aqueous solution.

{Fibrillation treatment of oxidized cellulose, preparation of water dispersion liquid}

First, 0.5 g of oxidized cellulose obtained by the TEMPO oxidation was dispersed in 99.5 g of distilled water, and finely ground by using a blender for 30 minutes to obtain a cellulose nanofiber water dispersion liquid with a concentration of 0.5%. This cellulose nanofiber water dispersion liquid was used as a continuous phase.

{Evaluation of cellulose nanofibers}

For the obtained oxidized cellulose and cellulose nanofibers, the amount of carboxyl group, the degree of crystallinity, the number average major axis diameter, the light transmittance, and the rheological properties were measured or calculated in the following manner.

{Measurement of amount of carboxyl group}

The amount of carboxyl group of the oxidized cellulose before dispersion treatment was calculated in the following manner.
First, 0.2 g of oxidized cellulose on a dry weight basis was placed in a beaker, and 80 ml of ion-exchanged water was added. To the resultant solution, 5 mL of 0.01 mol/L sodium chloride aqueous solution was added, and then 0.1 mol/L hydrochloric acid was added while stirring to adjust the pH of the entire solution to 2.8. To the resultant solution, a 0.1 mol/L sodium hydroxide aqueous solution was added at 0.05 mL/30 sec using an automatic titrator (trade name: AUT-701, manufactured by DKK-TOA Corporation), and the conductivity and the pH value were measured every 30 seconds. The measurement was continued until the pH reached 11. The titer of sodium hydroxide was determined from the obtained conductivity curve, and the content of carboxyl group was calculated. The results are shown in Table 1.

{Calculation of degree of crystallinity}

The degree of crystallinity of the TEMPO-oxidized cellulose was calculated in the following manner.
The X-ray diffraction pattern of the TEMPO-oxidized cellulose was measured using a multipurpose X-ray diffraction system equipped with a horizontal sample stage (trade name: Ultima III, manufactured by Rigaku Corporation) in the range of $5° \leq 2\theta \leq 35°$ at an X-ray output of 40 kV and 40 mA. The obtained X diffraction pattern was derived from the cellulose type I crystal structure; thus, the degree of crystallinity of the TEMPO-oxidized cellulose was calculated by the method described below using the following formula (2). The results are shown in Table 1.

$$\text{Degree of crystallinity (\%)} = [(I22.6 - I18.5)/I22.6] \times 100 \dots (2)$$

In the formula, I22.6 represents the diffraction intensity of a lattice plane (002 plane) (diffraction angle $2\theta$ = 22.6°) in the X-ray diffraction, and I18.5 represents the diffraction intensity of an amorphous part (diffraction angle $2\theta$ = 18.5°).

{Calculation of number average major axis diameter and number average minor axis diameter of cellulose nanofibers}

The number average major axis diameter and the number average minor axis diameter of the cellulose nanofibers were calculated in the following manner using an atomic force microscope.

First, a cellulose nanofiber water dispersion liquid with a concentration of 0.5% was diluted to 0.001%, and 20 $\mu$L portions of the dispersion liquid were then cast on a mica plate, followed by air-drying. After drying, the shape of the cellulose nanofibers was observed using an atomic force microscope (trade name: AFM5400L, manufactured by Hitachi High-Technologies Corporation) in the DFM mode.

The number average major axis diameter of the cellulose nanofibers was determined as the average value of the major axis diameter (maximum diameter) of 100 fibers measured from an image observed using the atomic force microscope. The number average minor axis diameter of the cellulose nanofibers was determined as the average value of the minor axis diameter (minimum diameter) of 100 fibers measured from an image observed using the atomic force microscope. The results are shown in Table 1.

{Measurement of light transmittance of cellulose nanofiber water dispersion liquid}

The light transmittance of a cellulose nanofiber water dispersion liquid with a concentration of 0.5 mass% was measured in the following manner.

Water was placed as a reference in one of sample cells made of quartz, and the cellulose nanofiber water dispersion liquid was placed in the other sample cell so that no air bubbles were formed. The light transmittance at a wavelength of 220 nm to 800 nm with an optical path length of 1 cm was measured using a spectrophotometer (trade name: NRS-1000, manufactured by JASCO Corporation). Fig. 4 shows the results.

{Measurement of rheological properties}

The rheological properties of a cellulose nanofiber water dispersion liquid with a concentration of 0.5 mass% were measured by steady-state viscoelasticity measurement using a rheometer. Specifically, the rheological properties were measured using a rheometer (trade name: AR2000ex, manufactured by TA Instruments) with a cone plate having an inclination angle of 1°. The temperature of the measurement site was adjusted to 25°C, and the shear viscosity was measured continuously at a shear rate of 0.01 $s^{-1}$ to 1000 $s^{-1}$. Fig. 5 shows the results. The shear viscosities at a shear rate of 10 $s^{-1}$ and 100 $s^{-1}$ are shown in Table 1.

[Table 1]

| Amount of carboxyl group (mmol/g) | Degree of crystallinity (%) | Number average minor axis diameter (nm) | Number average major axis diameter (nm) | Transmittance (%) | Viscosity (mPa·s) | |
|---|---|---|---|---|---|---|
| | | | | | [10s$^{-1}$] | [100s$^{-1}$] |
| 1.44 | 83 | 3 | 831 | 99.1 | 446.4 | 106.8 |

As shown in Table 1, the cellulose nanofibers (TEMPO-oxidized CNF) contained in the cellulose nanofiber water dispersion liquid had a number average minor axis diameter of 3 nm and a number average major axis diameter of 831 nm.

As is clear from Fig. 4, the cellulose nanofiber water dispersion liquid exhibited a high transparency. As is clear from Fig. 5, the cellulose nanofiber dispersion liquid was thixotropic.

(Second-i step: Step of producing O/W emulsion)

Next, 5 g of n-heptane which was a non-polymerizable solvent as a pore forming agent and 1 g of 2,2-azobis-2,4-dimethylvaleronitrile (hereinafter also referred to as ADVN) as a polymerization initiator were dissolved in 5 g of divinylbenzene (hereinafter also referred to as DVB) as a polymerizable monomer to prepare a polymerizable monomer mixed solution. This polymerizable monomer mixed solution was used as a dispersed phase. In the polymerizable monomer mixed solution, the content ratio of the non-polymerizable solvent as a pore forming agent to the total mass of the polymerizable monomer (DVB) as a core material and the non-polymerizable solvent as a pore forming agent was 50 mass%.

When the total amount of polymerizable monomer mixed solution obtained was added to 40 g of cellulose nanofiber water dispersion liquid obtained in the first step, the polymerizable monomer mixed solution and the cellulose nanofiber water dispersion liquid were separated into different phases (two phases).

Next, the shaft of an ultrasonic homogenizer was inserted from the liquid surface of the upper phase of the mixed solution in the two-phase separated state, and ultrasonic homogenizer treatment was performed for 3 minutes at a frequency of 24 kHz and an output of 400 W. The mixed solution after ultrasonic homogenizer treatment had a cloudy emulsion-like appearance. When a drop of the mixed solution was placed on a glass slide, covered with a cover glass, and observed using an optical microscope, it was confirmed that numerous emulsion droplets having a size of approximately several micrometers were produced and that an O/W emulsion containing a cellulose nanofiber water dispersion liquid as a continuous phase and a polymerizable monomer mixed solution as a dispersed phase was dispersed and stabilized.

(Third-i step: Step of solidifying core material)

The O/W emulsion dispersion liquid was placed in hot water at 70°C by using a water bath and stirred with a stirring bar for 8 hours to perform a polymerization reaction. After the 8-hour treatment, the dispersion liquid was cooled to room temperature. The dispersion liquid showed no change in appearance before and after the polymerization reaction.

(Fourth step: Collection, washing, and pore forming agent removal step)

The obtained dispersion liquid was centrifuged at a centrifugal force of 75,000 g for 5 minutes to obtain a precipitate. The supernatant was removed by decantation to collect the precipitate. Then, the precipitate was washed twice alternately with pure water and methanol using a PTFE membrane filter with a pore size of 0.1 $\mu$m.

The washed and collected material obtained in this manner was redispersed at a concentration of 1%, and the particle size of the material was evaluated by using a particle size distribution analyzer (NANOTRAC UPA-EX150, Nikkiso Co., Ltd.). Next, the washed and collected material was air dried, followed by vacuum drying treatment at a room temperature of 25°C for 24 hours, thereby obtaining a fine dry powder.

<Example 2>

A dry powder was obtained in the same manner as in Example 1, except that 7 g of DVB and 3 g of n-heptane were used in the second-i step.

<Example 3>

A dry powder was obtained in the same manner as in Example 1, except that 3 g of DVB and 7 g of n-heptane were used in the second-i step.

<Example 4>

A dry powder was obtained in the same manner as in Example 1, except that n-hexane was used instead of n-heptane in the second-i step.

<Example 5>

A dry powder was obtained in the same manner as in Example 1, except that n-dodecane was used instead of n-heptane in the second-i step.

<Example 6>

Unlike Example 1, in the second-i step, 7 g of DVB was used, and instead of n-heptane, 3 g of sorbitan monooleate (Span 80) as an oil-soluble surfactant was used, and distilled water which was a solvent as a continuous phase was absorbed as a pore forming agent in the emulsion droplets. Except for the above, a dry powder was obtained in the same manner as in Example 1.

The amount of distilled water absorbed in the emulsion droplets was unknown; thus, the content ratio of the distilled water as a pore forming agent to the total mass of the polymerizable monomer (DVB) as a core material and the distilled water as a pore forming agent was unknown.

<Example 7>

In the second-i step, 1 mass% acetylated CNF was dispersed in 7 g of DVB as a polymerizable monomer, and then 1 g of 2,2-azobis-2,4-dimethylvaleronitrile (hereinafter also referred to as ADVN) as a polymerization initiator was dissolved to prepare a polymerizable monomer mixed solution.

When 3 g of pure water as a pore forming agent was added to the obtained polymerizable monomer mixed solution, the polymerizable monomer mixed solution and the distilled water were separated into different phases (two phases). Next, ultrasonic homogenizer treatment was performed to produce a W/O emulsion liquid. The ratio (content ratio) of the pore forming agent to the total mass of the polymerizable monomer (DVB) as a core material and the distilled water as a pore forming agent was 30 mass%.

The total amount of W/O emulsion liquid obtained was added to 40 g of cellulose nanofiber water dispersion liquid.

Except for the above, a dry powder was obtained in the same manner as in Example 1.

<Example 8>

Unlike Example 1, a chitin nanofiber (also referred to as chitin NF) dispersion liquid produced according to Patent Literature 6 listed in the citation list was used instead of the TEMPO-oxidized CNF. Except for the above, a dry powder was produced in the same manner as in Example 1.

<Example 9>

Unlike Example 1, a carboxymethylated CNF dispersion liquid obtained by performing carboxymethylation according to Patent Literature 2 listed in the citation list, instead of the TEMPO oxidation, was used. Except for the above, a dry powder was produced in the same manner as in Example 1.

<Example 10>

Unlike Example 1, a phosphoric acid esterified CNF dispersion liquid obtained by performing phosphoric acid esterification according to Non Patent Literature 1 listed in the citation list, instead of the TEMPO oxidation, was used. Except for the above, a dry powder was produced in the same manner as in Example 1.

<Example 11>

Unlike Example 1, in the second-i step, 10 g of molten polymer was used instead of DVB as a polymerizable monomer. The molten polymer was obtained by heating and melting 5 g of poly-ε-caprolactone (PCL, manufactured by Wako Pure Chemical Industries, Ltd.). Except for the above, a dry powder was produced in the same manner as in Example 1.

<Comparative Example 1>

Unlike Example 1, particles were produced by spray drying without performing the second-i step and the third-i step. Except for the above, the steps were performed in the same manner as in Example 1.

<Comparative Example 2>

Unlike Example 1, in the second-i step, n-heptane as a non-polymerizable solvent was not added to 10 g of DVB as a polymerizable monomer. Except for the above, the steps were performed in the same manner as in Example 1.

<Comparative Example 3>

Unlike Example 1, a carboxymethyl cellulose (hereinafter also referred to as CMC) aqueous solution was used instead of the TEMPO-oxidized CNF dispersion liquid. Except for the above, the steps were performed in the same manner as in Example 1.

<Comparative Example 4>

Unlike Example 11, in the second-i step, 8 parts by mass of polyvinyl alcohol (PVA) and 0.5 parts by mass of polyglyceryl-10 laurate (PGLE ML10) were dissolved in 500 g of pure water to obtain an aqueous solution, and then instead of the cellulose nanofiber water dispersion liquid, the aqueous solution was added to the polymerizable monomer mixed solution to produce an emulsion dispersion liquid. Except for the above, the steps were performed in the same manner as in Example 11.

<Comparative Example 5>

Unlike Example 11, in the second-i step, instead of the TEMPO-oxidized CNF dispersion liquid, a CMC aqueous solution was added to the polymerizable monomer mixed solution to produce an emulsion dispersion liquid. Except for the above, the steps were performed in the same manner as in Example 11.

<Comparative Example 6>

As a conventional example, commercially available styrene-divinylbenzene copolymer microbeads (with a particle size of 4.5 $\mu$m, Techno Chemical Corporation) were prepared.

<Evaluation method>

(Evaluation of particle structure)

The obtained dry powders of the examples and Comparative Examples 1 to 5 and the prepared dry powder of Comparative Example 6 were observed using a scanning electron microscope. Furthermore, each of the obtained dry powders was embedded in resin, and the cross section was observed by SEM. The particle structure of the dry powder was evaluated by observation. The results were evaluated according to the following criteria: "Good" and "Poor".
Good: Pores were formed in the particles, and the surface of the particles was coated with fine fibers (cellulose nanofibers or chitin nanofibers).
Poor: No such particles were obtained.

(Evaluation of sphericity)

The dry powder was placed in a particle shape image analyzer "PITA-04" from Seishin Enterprise Co., Ltd., and the average circularity was calculated from the average value of the particle circularity of 1000 or more particles, and the sphericity was evaluated according to the following criteria. The circularity was calculated by the formula "circularity = $4\pi S/L^2$", where S represents the area of a particle, and L represents the perimeter of the particle in the image.
Good: Average circularity was 0.6 or more.
Poor: Average circularity was less than 0.6.

(Evaluation of amount of oil absorption)

The amount of oil absorption was measured by the method in accordance with JK 5101-13-1. The powder was placed on a measuring plate, and 4 or 5 droplets at a time of purified linseed oil were gradually added to the powder through a burette. Each time the purified linseed oil was added, the purified linseed oil was kneaded into the sample with a palette knife. This action was repeated, and the purified linseed oil was continuously dropped until a lump of purified linseed oil and sample was formed. After that, a single droplet at a time of purified linseed oil was added, and kneading was repeated so that the purified linseed oil was completely mixed with the sample. The process was ended when the paste had a smooth consistency.
The amount of oil absorption was evaluated according to the following criteria: "Good", "Fair", and "Poor".
Good: The amount of oil absorption was 30 ml/100 g or more.
Fair: The amount of oil absorption was 20 ml/100 g or more and less than 30 ml/100 g.
Poor: The amount of oil absorption was less than 20 ml/100 g.

(Evaluation of light diffusing properties)

The light diffusing properties were evaluated in the following manner using a variable angle photometer GC5000 manufactured by Nippon Denshoku Industries Co., Ltd. First, a glass plate on which the dry powder was placed was inserted in the GC5000, and a spectral reflectance R1 and a spectral reflectance R2 were measured at a wavelength of 400 nm to 700 nm. The spectral reflectance R1 was a spectral reflectance when incident light at an angle of -45° was received at an angle of 0°, and the spectral reflectance R2 was a spectral reflectance when incident light at an angle of -45° was received at an angle of 45°. Next, from the measurement results, the ratio of R1 to R2 at a wavelength of 600 nm was calculated as a light diffusion index. A light diffusion index closer to 1 indicates a lower angle dependency and a higher soft-focus effect. The light diffusing properties were evaluated according to the following criteria: "Good", "Fair", and "Poor".

Good: The light diffusion index was 0.9 or more.

Fair: The light diffusion index was 0.6 or more and less than 0.9.

Poor: The light diffusion index was less than 0.6.

(Evaluation of dispersibility)

The dry powder was added to pure water at a concentration of 1 mass% and stirred with a stirring bar for 24 hours to be dispersed. Then, the dry powder was visually observed for the presence of aggregation. The results were evaluated according to the following criteria: "Good" and "Poor".

Good: No aggregates were visually observed.

Poor: Aggregates were visually observed.

(Evaluation of feeling in use)

In a room at a temperature of 20°C and a humidity of 40% RH, each of the dry powders of the examples and the comparative examples was applied to an inner part of the lower arm of a subject (woman in her thirties). Specifically, 0.1 g of dry powder was applied to the skin and spread with a finger into a circle with a diameter of approximately 5 cm. Then, the feeling in use of the dry powder when applied to the skin was evaluated according to the following criteria: "Good" and "Poor".

Good: Highly compatible with the skin, and causing little or no irritation to the skin.

Poor: Poorly compatible with the skin, and causing irritation to the skin.

(Evaluation of moisturizing properties)

In a room at a temperature of 20°C and a humidity of 40% RH, each of the dry powders of the examples and the comparative examples was applied to an inner part of the lower arm of a subject (woman in her thirties). Specifically, 0.1 g of dry powder was applied to the skin and spread with a finger into a circle with a diameter of approximately 5 cm. When 30 minutes had elapsed after the application, at the part at which the dry powder was applied, the amount of skin moisture was measured by using a Moisture checker MY-707S (Scalar Corporation). The results were evaluated according to the following criteria: "Good" and "Poor".

Good: The amount of moisture was 60% or more.

Poor: The amount of moisture was less than 60%.

Table 2 below shows all the evaluation results of Examples 1 to 11 and Comparative Examples 1 to 6. The emulsion stabilizer in Table 2 indicates cellulose nanofibers, or an emulsion stabilizer used instead of the cellulose nanofibers. Furthermore, Table 2 shows the polymer constituting the core particles, the pore forming agent used in the production method, and the content ratio of the pore forming agent (the ratio of the pore forming agent to the total mass of the core material and the pore forming agent). In Examples 1 to 11, the production method I shown in Fig. 2 was used.

[Table 2]

| | Emulsion stabilizer | Core particles | Pore forming agent | | Particle structure | Sphericity | Amount of oil absorption | Light diffus-ing proper-ties | Dispersibility | Feeling in use | Moisturizing properties |
|---|---|---|---|---|---|---|---|---|---|---|---|
| | | | Type | Content ratio (mass%) | | | | | | | |
| Example 1 | TEMPO-oxidized CNF | DVB polymer | n-heptane | 50 | Good | Good | Good | Good | Good | Good | Good |
| Example 2 | TEMPO-oxidized CNF | DVB polymer | n-heptane | 30 | Good | Good | Good | Good | Good | Good | Good |
| Example 3 | TEMPO-oxidized CNF | DVB polymer | n-heptane | 70 | Good | Good | Good | Good | Good | Good | Good |
| Example 4 | TEMPO-oxidized CNF | DVB polymer | n-hexane | 50 | Good | Good | Good | Good | Good | Good | Good |
| Example 5 | TEMPO-oxidized CNF | DVB polymer | n-dodecane | 50 | Good | Good | Fair | Good | Good | Good | Good |
| Example 6 | TEMPO-oxidized CNF | PLA | Solvent as continuous phase (distilled water) | Unknown | Good | Good | Good | Good | Good | Good | Good |
| Example 7 | TEMPO-oxidized CNF | PCL | Distilled water | 300 | Good | Good | Good | Good | Good | Good | Good |
| Example 8 | Chitin NF | DVB polymer | n-heptane | 50 | Good | Good | Good | Good | Good | Good | Good |
| Example 9 | Carboxymethylated CNF | DVB polymer | n-heptane | 50 | Good | Good | Good | Good | Good | Good | Good |
| Example 10 | Phosphoric acid esterified CNF | DVB polymer | n-heptane | 50 | Good | Good | Good | Good | Good | Good | Good |
| Example 11 | TEMPO-oxidized CNF | PCL | n-heptane | 50 | Good | Good | Good | Good | Good | Good | Good |
| Comparative Example 1 | TEMPO-oxidized CNF | - | - | 0 | Poor | Poor | Poor | Poor | Poor | Poor | Good |
| Comparative Example 2 | TEMPO-oxidized CNF | DVB polymer | - | 0 | Poor | Good | Poor | Fair | Good | Good | Good |

| | Emulsion stabilizer | Core particles | Pore forming agent | | Particle structure | Sphericity | Amount of oil absorption | Light diffusing properties | Dispersibility | Feeling in use | Moisturizing properties |
|---|---|---|---|---|---|---|---|---|---|---|---|
| | | | Type | Content ratio (mass%) | | | | | | | |
| Comparative Example 3 | CMC | DVB polymer | n-heptane | 50 | Poor | Poor | Good | Good | Poor | Poor | Poor |
| Comparative Example 4 | PVA/PGLE ML10 | PCL | n-heptane | 50 | Poor | Poor | Good | Good | Poor | Poor | Poor |
| Comparative Example 5 | CMC | PCL | n-heptane | 50 | Poor | Poor | Good | Good | Poor | Poor | Poor |
| Comparative Example 6 | - | - | - | - | Poor | Good | Poor | Poor | Poor | Good | Poor |

When the dry powder obtained in Example 1 was observed using a scanning electron microscope (SEM), it was confirmed that due to the polymerization reaction performed using the O/W emulsion droplets as a template, numerous particles having a spherical shape derived from the shape of the emulsion droplets were present and that the surface of the particles was uniformly coated with cellulose nanofibers having a width of several nanometers. Despite repeated washing by filtration washing, the surface of the particles was evenly and uniformly coated with the cellulose nanofibers, indicating that the core particles and the cellulose nanofibers were inseparably bonded to each other. Furthermore, it was confirmed that fine pores were formed on the surface of the particles.

When the dry powder obtained in Example 1 was embedded in resin, and the cross section was observed with the SEM, it was confirmed that a large number of fine pores were present in the dry powder. That is, it was confirmed that the dry powder obtained in Example 1 was the composite particles of the first embodiment.

When the dry powder obtained in Example 1 was added to pure water at a concentration of 1 mass% and stirred with a stirring bar for 24 hours to be redispersed, the dry powder was easily redispersed, and no aggregation was visually observed. When the particle size of the particles was evaluated by using a particle size distribution analyzer, the average particle size was approximately the same as before drying, and the data obtained using the particle size distribution analyzer did not show any signal indicating aggregation.

These results showed that even though the surface of the dry powder obtained in Example 1 (the composite particles of the first embodiment) was coated with the cellulose nanofibers, the composite particles were not in the form of a film when dried and were obtained as a powder, and had good redispersibility.

Similarly, when the surface and cross section of the dry powders obtained in Examples 2 to 11 were observed using the SEM, it was confirmed that the particles constituting the dry powders were spherical composite particles in which pores were formed and whose surface was coated with fine fibers.

Thus, it was confirmed that by using the production method I, regardless of the type of fine fibers (TEMPO-oxidized CNF, carboxymethylated CNF, phosphoric acid esterified CNF, chitin NF), spherical composite particles were produced in which pores were formed in core particles made of various polymers such as a polymer of various monomers or a biodegradable polymer and whose surface was coated with fine fibers. Furthermore, it was confirmed that regardless of the type of pore forming agent or the amount of pore forming agent, spherical composite particles were produced in which pores were formed in core particles and whose surface was coated with fine fibers.

It was confirmed that as in Example 1, in the dry powders obtained in Examples 2 to 4, Example 6, and Examples 8 to 11, the surface of the particles had a portion coated with fine fibers and a portion having pores. That is, it was confirmed that porous composite particles were obtained in Examples 1 to 4, Example 6, and Examples 8 to 11. The dry powders obtained in these examples had very high oil absorbency and exhibited good light diffusing properties.

In the dry powders obtained in Examples 5 and 7, no pores were observed on the surface of the particles, but it was confirmed by observation of the cross section that the particles were hollow composite particles. The dry powders obtained in these examples had relatively high oil absorbency and exhibited good light diffusing properties.

All the dry powders obtained in Examples 1 to 11 had good redispersibility, and exhibited good skin compatibility and good moisturizing properties.

Furthermore, by impregnating the dry powder (composite particles) obtained in Example 1 with a vitamin C derivative as a functional component, personal care particles containing a skin-whitening component were obtained.

On the other hand, the dry powders of Comparative Example 1, 2, and 6 were neither porous particles nor hollow particles, and were poor in oil absorbency and light diffusing properties. The dry powders of Comparative Examples 3 to 5 were porous particles and had good oil absorbency, but did not have a spherical shape (had a low sphericity) and had a poor feeling in use. Furthermore, since the particles were not coated with fine fibers, the particles were poor in dispersibility and moisturizing properties.

[Examples 21 to 30, Comparative Examples 21 to 29]

<Example 21>

(First step: Step of obtaining cellulose nanofiber dispersion liquid)

A cellulose nanofiber water dispersion liquid with a concentration of 0.5 mass% was obtained in the same manner as in the first step of Example 1.

(Second-iii step: Step of supporting functional material)

{Step ia: Preparation of cellulose nanofiber dispersion liquid}

First, 50 g of 0.5 mass% cellulose nanofiber water dispersion liquid was prepared.

{Step ib: Preparation of silver nitrate aqueous solution/cellulose nanofiber-containing liquid}

A 100 mM silver nitrate (I) aqueous solution was prepared. Then, 1.0 g of 100 mM silver nitrate aqueous solution was added to 50 g of 0.5 mass% cellulose nanofiber water dispersion liquid while the temperature was kept constant (25°C) and the liquid was stirred with a stirring bar, and the resultant solution was continuously stirred for 30 minutes.

{Step ic: Reduction and deposition}

Sodium borohydride was dissolved in distilled water to prepare a 100 mM sodium borohydride aqueous solution. Then, 1.0 g of 100 mM sodium borohydride aqueous solution was added to the silver nitrate aqueous solution/cellulose nanofiber-containing liquid while the temperature was kept constant (25°C) and the liquid was stirred with a stirring bar, and the resultant solution was continuously stirred for 60 minutes to produce a dispersion liquid of silver fine particle-supporting cellulose nanofibers. The obtained dispersion liquid had a yellow color derived from the silver fine particles, indicating the generation of silver fine particles.

(Second-iv step: Step of producing O/W emulsion)

Next, 1 g of 2,2-azobis-2,4-dimethylvaleronitrile (hereinafter also referred to as ADVN) as a polymerization initiator was dissolved in 10 g of divinylbenzene (hereinafter also referred to as DVB) as a polymerizable monomer. When the total amount of polymerizable monomer mixed solution obtained was added to 40 g of silver fine particle-supporting cellulose nanofiber dispersion liquid, the polymerizable monomer mixed solution and the cellulose nanofiber dispersion liquid were separated into different phases (two phases).
Next, the shaft of an ultrasonic homogenizer was inserted from the liquid surface of the upper phase of the mixed solution in the two-phase separated state, and ultrasonic homogenizer treatment was performed for 3 minutes at a frequency of 24 kHz and an output of 400 W. The mixed solution after ultrasonic homogenizer treatment had a cloudy emulsion-like appearance. When a drop of the mixed solution was placed on a glass slide, covered with a cover glass, and observed using an optical microscope, it was confirmed that numerous emulsion droplets having a size of approximately several micrometers were produced and that the droplets were dispersed and stabilized as an O/W emulsion.

(Third-ii step: Step of solidifying core material)

The O/W emulsion dispersion liquid was placed in hot water at 70°C by using a water bath and stirred with a stirring bar for 8 hours to perform a polymerization reaction. After the 8-hour treatment, the dispersion liquid was cooled to room temperature. The dispersion liquid showed no change in appearance before and after the polymerization reaction.

(Washing and drying step)

The obtained dispersion liquid was centrifuged at a centrifugal force of 75,000 g for 5 minutes to obtain a precipitate. The supernatant was removed by decantation to collect the precipitate. Then, the precipitate was washed twice alternately with pure water and methanol using a PTFE membrane filter with a pore size of 0.1 μm.
The washed and collected material obtained in this manner was redispersed at a concentration of 1%, and the particle size of the material was evaluated by using a particle size distribution analyzer (NANOTRAC UPA-EX150, Nikkiso Co., Ltd.). Next, the washed and collected material was air dried, followed by vacuum drying treatment at a room temperature of 25°C for 24 hours, thereby obtaining a fine dry powder having a yellow color derived from the LSPR of spherical silver fine particles.

(Observation of shape using scanning electron microscope)

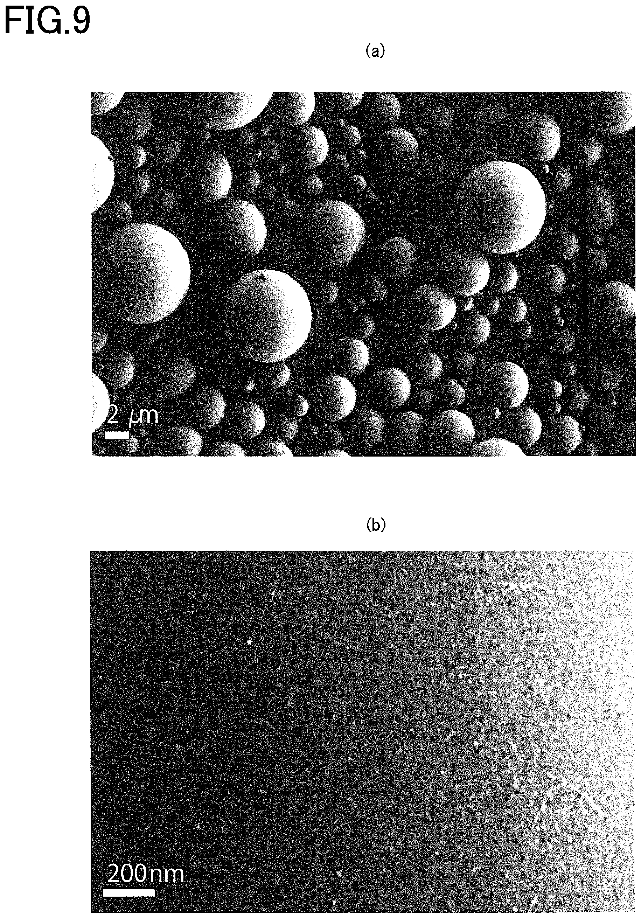

The obtained dry powder was observed using a scanning electron microscope (SEM). Fig. 9 shows the SEM image. It was confirmed that due to the polymerization reaction performed using the O/W emulsion droplets as a template, numerous particles having a spherical shape derived from the shape of the emulsion droplets were formed and that the surface of the particles was uniformly coated with cellulose nanofibers having a width of several nanometers. Despite repeated washing by filtration washing, the surface of the particles was evenly and uniformly coated with the cellulose nanofibers, indicating that the core particles and the cellulose nanofibers were inseparably bonded to each other.
Despite the repeated washing by filtration washing, spherical silver fine particles having a size of approximately a few to 10 nm were present on the surface of the cellulose nanofibers, and the spherical silver fine particles were supported as a functional material by the cellulose nanofibers on the surface of the particles. This suggested that the cellulose nanofibers and the spherical silver fine particles were inseparable from each other.

When the obtained dry powder was embedded in resin and cut in cross section using a microtome, and was observed with a scanning electron microscope, it was confirmed that a uniform coating layer was formed on the surface of the core particles.

Thus, despite repeated filtration washing, the surface of the personal care particles was evenly and uniformly coated with the cellulose nanofibers, and the spherical silver fine particles were supported as a functional material on the surface of the cellulose nanofibers. Therefore, it was confirmed that the obtained dry powder was the personal care particles of the second embodiment.

(Evaluation of dispersibility)

When the dry powder was added to pure water at a concentration of 1 mass% and stirred with a stirring bar for 24 hours to be redispersed, the dry powder was easily redispersed, and no aggregation was visually observed. When the particle size of the particles was evaluated by using a particle size distribution analyzer, the average particle size was approximately the same as before drying, and the data obtained using the particle size distribution analyzer did not show any signal indicating aggregation. These results showed that even though the surface of the personal care particles was coated with the cellulose nanofibers, the personal care particles were not in the form of a film when dried and were obtained as a powder, and had good redispersibility.

<Example 22>

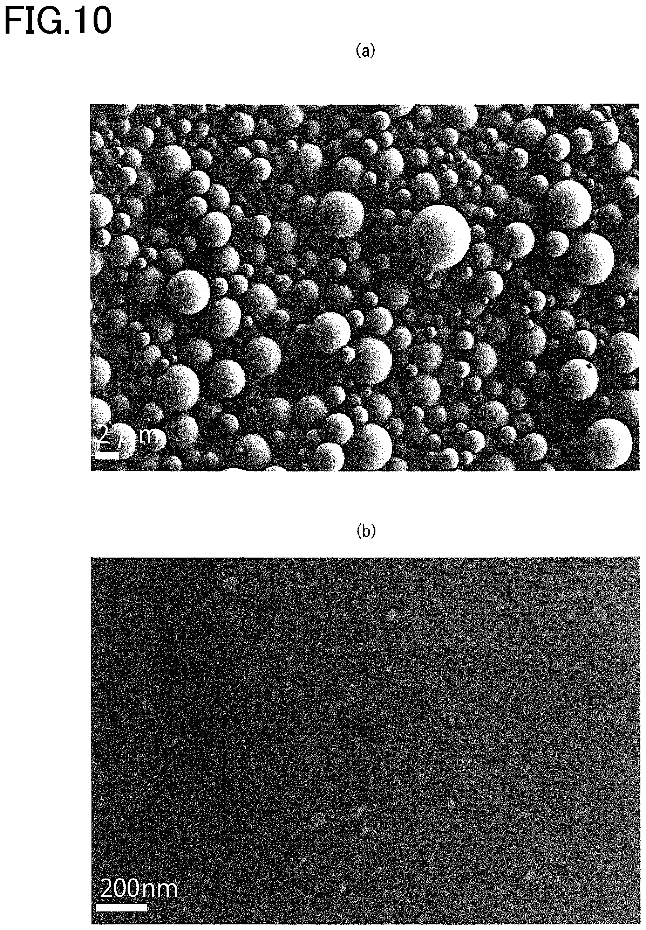

Personal care particles were obtained under the same conditions as in Example 21, except that oxygen gas was bubbled in step ic of the second-iii step, unlike Example 21. A dry powder of the obtained personal care particles was observed using a scanning electron microscope (SEM). Fig. 10 shows the SEM image.

<Example 23>

Unlike Example 1, a chitin nanofiber (also referred to as "chitin NF") dispersion liquid produced according to Patent Literature 6 listed in the citation list was used instead of the TEMPO-oxidized CNF. Except for the above, personal care particles were produced under the same conditions as in Example 21.

<Example 24>

Unlike Example 21, a carboxymethylated CNF dispersion liquid obtained by performing carboxymethylation according to Patent Literature 2 listed in the citation list, instead of the TEMPO oxidation, was used. Except for the above, personal care particles were produced under the same conditions as in Example 21.

<Example 25>

Unlike Example 21, a phosphoric acid esterified CNF dispersion liquid obtained by performing phosphoric acid esterification according to Non Patent Literature 1 listed in the citation list, instead of the TEMPO oxidation, was used. Except for the above, personal care particles were produced under the same conditions as in Example 21.

<Example 26>

(First step: Step of obtaining cellulose nanofiber dispersion liquid)

A cellulose nanofiber dispersion liquid was obtained under the same conditions as in Example 21.

(Second-iii step: Step of supporting functional material)

A spherical silver fine particle-supporting cellulose nanofiber dispersion liquid was obtained under the same conditions as in Example 21.

(Second-iv step: Step of producing O/W emulsion)

Next, 10 g of polylactic acid (PLA) was dissolved and mixed in 100 g of dichloroethane to prepare a dissolved polymer.

When the total amount of dissolved polymer was added to 500 g of 0.5 mass% spherical silver fine particle-supporting cellulose nanofiber dispersion liquid, the dissolved polymer and the spherical silver fine particle-supporting cellulose nanofiber dispersion liquid were separated into different phases (two phases).

Next, from the liquid surface of the upper phase of the mixed solution in the two-phase separated state, ultrasonic homogenizer treatment was performed using an ultrasonic homogenizer in the same manner as in the second-iv step of Example 21. It was confirmed with an optical microscope that numerous emulsion droplets having a size of approximately one to several tens of micrometers were produced and that the droplets were dispersed and stabilized as an O/W emulsion.

(Third-ii step: Step of solidifying core material)

The O/W emulsion liquid was dried under reduced pressure of 700 mgHg at 40°C for 3 hours to completely evaporate the dichloroethane. The dispersion liquid showed no change in appearance before and after the evaporation of the dichloroethane.

When the obtained dispersion liquid was separated and washed under the same conditions as in Example 21, personal care particles having a particle size of approximately one to several tens of micrometers were obtained. When the collected material was dried under the same conditions as in Example 21, a fine dry powder having a yellow color derived from the LSPR of spherical silver fine particles was obtained.

<Example 27>

First, spherical silver fine particle-supporting cellulose nanofibers were prepared by performing the first step and the second-iii step in the same manner as in Example 26. Next, 10 g of poly-ε-caprolactone (PCL, manufactured by Wako Pure Chemical Industries, Ltd.) was dissolved in 200 g of ethyl acetate to prepare a dissolved polymer. Except for the above, an O/W emulsion liquid was prepared in the same manner as in the second-iv step of Example 26. Next, the obtained O/W emulsion liquid was dried under reduced pressure of 700 mgHg at 40°C for 5 hours to completely evaporate the ethyl acetate (third-ii step).

When the obtained dispersion liquid was separated and washed under the same conditions as in Example 21, personal care particles having a particle size of approximately one to several tens of micrometers were obtained. When the collected material was dried under the same conditions as in Example 21, a fine dry powder having a yellow color derived from the LSPR of spherical silver fine particles was obtained.

<Example 28>

Cellulose nanofiber coated particles were obtained by performing the first step under the same conditions as in Example 21, and then without performing the second-iii step, performing the second-iv step and the third-ii step under the same conditions as in Example 21, followed by the washing and drying step. Next, the fifth step described below was performed using the obtained cellulose nanofiber coated particles, followed by washing and drying performed as described below.

(Fifth step: Step of supporting functional material)

{Step iia: Preparation of cellulose nanofiber coated particle dispersion liquid}

The cellulose nanofiber coated particles were dispersed in pure water to prepare 50 g of 0.5 mass% cellulose nanofiber coated particle dispersion liquid.

{Step iib: Preparation of silver nitrate aqueous solution/cellulose nanofiber coated particle-containing liquid}

A 100 mM silver nitrate (I) aqueous solution was prepared. Then, 1.0 g of 100 mM silver nitrate aqueous solution was added to 50 g of 0.5 mass% cellulose nanofiber coated particle dispersion liquid while the temperature was kept constant (25°C) and the liquid was stirred with a stirring bar, and the resultant solution was continuously stirred for 30 minutes.

{Step iic: Reduction and deposition}

Sodium borohydride was dissolved in distilled water to prepare a 100 mM sodium borohydride aqueous solution. Then, 1.0 g of 100 mM sodium borohydride aqueous solution was added to the silver nitrate aqueous solution/cellulose nanofiber coated particle-containing liquid while the temperature was kept constant (25°C) and the liquid was stirred with a stirring bar, and the resultant solution was continuously stirred for 60 minutes to produce a dispersion liquid of silver fine particle-supporting cellulose nanofiber coated spherical particles. The obtained dispersion liquid had a yellow color derived from the spherical silver fine particles, indicating the generation of spherical silver fine particles.

(Washing and drying step)

The obtained dispersion liquid was centrifuged at a centrifugal force of 75,000 g for 5 minutes to obtain a precipitate. The supernatant was removed by decantation to collect the precipitate. Then, the precipitate was washed 4 times with pure water using a PTFE membrane filter with a pore size of 0.1 μm. The washed and collected material obtained in this manner was redispersed at a concentration of 1%, and the particle size of the material was evaluated by using a particle size distribution analyzer (NANOTRAC UPA-EX150, Nikkiso Co., Ltd.).
Next, the washed and collected material was air dried, followed by vacuum drying treatment at a room temperature of 25°C for 24 hours, thereby obtaining a fine dry powder having a yellow color derived from spherical silver fine particles.

<Example 29>

Unlike Example 21, in the second-iii step, an aqueous solution of sodium tetrachloroaurate(III) was used instead of the silver nitrate aqueous solution. Except for the above, the steps were performed in the same manner as in Example 21 to obtain dry particles. The obtained dry powder was observed using a scanning electron microscope (SEM). Fig. 13 shows the SEM image.

<Example 30>

Unlike Example 21, cellulose nanofiber coated particles were obtained by performing the first step, and then without performing the second-iii step, performing the second-iv step, the third-ii step, and the washing and drying step. Next, the fifth step described below was performed using the obtained cellulose nanofiber coated particles, followed by washing and drying performed as described below.

(Fifth step: Step of supporting functional material)

The cellulose nanofiber coated particles were dispersed in pure water to prepare 10 g of 5 mass% cellulose nanofiber coated particle dispersion liquid. Subsequently, rhodamine B as a cationic dye was dissolved in pure water to prepare 10 g of 5 mass% rhodamine B aqueous solution. While stirring the rhodamine B aqueous solution, the cellulose nanofiber coated particle dispersion liquid was added to the rhodamine B aqueous solution and stirred for a while. Thus, a dispersion liquid of rhodamine B-supporting cellulose nanofiber coated particles was produced.

(Washing and drying step)

The obtained dispersion liquid was centrifuged at a centrifugal force of 75,000 g for 5 minutes to obtain a precipitate. The supernatant was removed by decantation to collect the precipitate. Then, the precipitate was washed 4 times with pure water using a PTFE membrane filter with a pore size of 0.1 μm. The washed and collected material obtained in this manner was redispersed at a concentration of 1%, and the particle size of the material was evaluated by using a particle size distribution analyzer (NANOTRAC UPA-EX150, Nikkiso Co., Ltd.).
Next, the washed and collected material was air dried, followed by vacuum drying treatment at a room temperature of 25°C for 24 hours, thereby obtaining a fine dry powder (personal care particles) having a red color derived from rhodamine B.

<Comparative Example 21>

Unlike Example 21, the second-iv step and the third-ii step were not performed. Except for the above, the steps were performed in the same manner as in Example 21.

<Comparative Example 22>

Unlike Example 21, pure water was used instead of the TEMPO-oxidized CNF dispersion liquid. Except for the above, the steps were performed in the same manner as in Example 21.

<Comparative Example 23>

Unlike Example 21, a carboxymethyl cellulose (hereinafter also referred to as CMC) aqueous solution was used instead of the TEMPO-oxidized CNF dispersion liquid. Except for the above, the steps were performed in the same manner as in Example 21.

<Comparative Example 24>

First, 8 parts by mass of polyvinyl alcohol (PVA) and 0.5 parts by mass of polyglyceryl-10 laurate (PGLE ML10) were dissolved in 500 g of pure water to produce an aqueous solution. By using the produced solution instead of the cellulose nanofiber dispersion liquid, a dispersion liquid of "PVA/PGLE ML10" supporting silver fine particles was produced in the same manner as in the second-iii step of Example 21. Next, by using the produced dispersion liquid instead of the silver fine particle-supporting cellulose nanofiber dispersion liquid, an O/W emulsion liquid was prepared in the same manner as in the second-iv step of Example 26. Next, the obtained O/W emulsion liquid was spray-dried at a drying temperature of 100°C using a spray dryer.

<Comparative Example 25>

Unlike Comparative Example 24, polyglyceryl-10 laurate (PGLE ML10) was not added. Except for the above, the steps were performed in the same manner as in Comparative Example 24.

<Comparative Example 26>

Unlike Example 28, a carboxymethyl cellulose (hereinafter also referred to as CMC) aqueous solution was used instead of the TEMPO-oxidized CNF dispersion liquid. Except for the above, the steps were performed in the same manner as in Example 28.

<Comparative Example 27>

Unlike Comparative Example 21, an aqueous solution of sodium tetrachloroaurate(III) was used instead of the silver nitrate aqueous solution. Except for the above, the steps were performed in the same manner as in Example 21.

<Comparative Example 28>

Unlike Comparative Example 21, in step ic of the second-iii step, oxygen gas was bubbled. Except for the above, the steps were performed in the same manner as in Example 21.

<Comparative Example 29>

As a conventional example, a dry powder was prepared in which commercially available styrene-divinylbenzene copolymer microbeads (particle size 4.5 $\mu$m, Techno Chemical Corporation) were mixed with commercially available spherical silver fine particles (particle size 10 nm, Sigma-Aldrich).

<Evaluation method>

(Evaluation of particle structure)

The obtained dry powders of the examples and Comparative Examples 21 to 28 and the prepared dry powder of Comparative Example 29 were observed using a scanning electron microscope, and the particle structure was evaluated. The results were evaluated according to the following criteria: "Good" and "Poor".
Good: Spherical particles were obtained, and the surface of the particles was coated with fine fibers (cellulose nanofibers or chitin nanofibers), and a functional material was supported by the fine fibers.
Poor: No such particles were obtained.

(Evaluation of collection)

The dispersion liquid obtained in the third-ii step or the fifth step was centrifuged at a centrifugal force of 75,000 g for 5 minutes. The supernatant was removed by decantation to collect the precipitate. Then, the precipitate was washed twice alternately with pure water and methanol using a PTFE membrane filter with a pore size of 0.1 μm. The results were evaluated according to the following criteria: "Good" and "Poor".

Good: Precipitate was collected by centrifugation, and sample was collected on the membrane filter.

Poor: No precipitate was collected by centrifugation. Or no sample was collected on the membrane filter.

(Evaluation of dispersibility)

Each of the dry powders of the examples and the comparative examples was added to pure water at a concentration of 1 mass% and stirred with a stirring bar for 24 hours to be dispersed. Then, the dry powder was visually observed for the presence of aggregation. The results were evaluated according to the following criteria: "Good" and "Poor".

Good: No aggregates were visually observed.

Poor: Aggregates were visually observed.

(Evaluation of feeling in use)

In a room at a temperature of 20°C and a humidity of 40% RH, each of the dry powders of the examples and the comparative examples was applied to an inner part of the lower arm of a subject (woman in her thirties). Specifically, 0.1 g of dry powder was applied to the skin and spread with a finger into a circle with a diameter of approximately 5 cm. Then, the feeling in use of the dry powder when applied to the skin was evaluated according to the following criteria: "Good" and "Poor".

Good: Highly compatible with the skin, and causing little or no irritation to the skin.

Poor: Poorly compatible with the skin, and causing irritation to the skin.

(Evaluation of moisturizing properties)

In a room at a temperature of 20°C and a humidity of 40% RH, each of the dry powders of the examples and the comparative examples was applied to an inner part of the lower arm of a subject (woman in her thirties). Specifically, 0.1 g of dry powder was applied to the skin and spread with a finger into a circle with a diameter of approximately 5 cm. When 30 minutes had elapsed after the application, at the part at which the dry powder was applied, the amount of skin moisture was measured by using a Moisture checker MY-707S (Scalar Corporation). The results were evaluated according to the following criteria: "Good" and "Poor".

Good: The amount of moisture was 60% or more.

Poor: The amount of moisture was less than 60%.

(Evaluation of colorability)

In a room at a temperature of 20°C and a humidity of 40% RH, each of the dry powders of the examples and the comparative examples was applied to an inner part of the lower arm of a subject (woman in her thirties). Specifically, 0.1 g of dry powder was applied to the skin and spread with a finger into a circle with a diameter of approximately 5 cm. The results were evaluated according to the following criteria: "Good" and "Poor".

Good: Uniform coloration was visually observed.

Poor: Non-uniform coloration was visually observed.

In addition, the color of the dry powder was visually observed.

Table 3 below shows all the evaluation results of Examples 21 to 30 and Comparative Examples 21 to 29. The emulsion stabilizer in Table 3 indicates cellulose nanofibers, or an emulsion stabilizer used instead of the cellulose nanofibers. Furthermore, Table 3 shows the polymer constituting the core particles, and the functional component (supported material) contained in the core particles. Table 3 also shows which of the production method III shown in Fig. 5 and the production method IV shown in Fig. 6 was used as the production method in each of the examples.

[Table 3]

| | Emulsion stabilizer | Core particles | Supported material/ Production method | Particle structure | Collection | Dispersibility | Feeling in use | Moisturizing properties | Colorability determination/ Color |
|---|---|---|---|---|---|---|---|---|---|
| Example 21 | TEMPO-oxidized CNF | DVB polymer | Spherical silver fine particles/I | Good | Good | Good | Good | Good | Good/Yellow |
| Example 22 | TEMPO-oxidized CNF | DVB polymer | Flat plate-like silver fine particles/I | Good | Good | Good | Good | Good | Good/Blue |
| Example 23 | Chitin NF | DVB polymer | Spherical silver fine particles/I | Good | Good | Good | Good | Good | Good/Yellow |
| Example 24 | Carboxymethylated CNF | DVB polymer | Spherical silver fine particles/I | Good | Good | Good | Good | Good | Good/Yellow |
| Example 25 | Phosphoric acid esterified CNF | DVB polymer | Spherical silver fine particles/I | Good | Good | Good | Good | Good | Good/Yellow |
| Example 26 | TEMPO-oxidized CNF | PLA | Spherical silver fine particles/I | Good | Good | Good | Good | Good | Good/Yellow |
| Example 27 | TEMPO-oxidized CNF | PCL | Spherical silver fine particles/I | Good | Good | Good | Good | Good | Good/Yellow |
| Example 28 | TEMPO-oxidized CNF | DVB polymer | Spherical silver fine particles/II | Good | Good | Good | Good | Good | Good/Yellow |
| Example 29 | TEMPO-oxidized CNF | DVB polymer | Spherical gold fine particles/I | Good | Good | Good | Good | Good | Good/Red |
| Example 30 | TEMPO-oxidized CNF | DVB polymer | Rhodamine B/II | Good | Good | Good | Good | Good | Good/Red |
| Comparative Example 21 | TEMPO-oxidized CNF | - | Spherical silver fine p article s/- | - | Poor | Poor | Poor | Good | Good/Yellow |
| Comparative Example 22 | None (pure water only) | DVB polymer | Spherical silver fine particles/I | Poor | Good | Poor | Poor | Poor | Poor/Yellow |
| Comparative Example 23 | CMC | DVB polymer | Spherical silver fine particles/I | Poor | Good | Poor | Poor | Poor | Poor/Yellow |
| Comparative Example 24 | PVA/PGLE ML10 | PLA | Spherical silver fine particles/I | Poor | Good | Poor | Poor | Poor | Poor/Yellow |

(continued)

| | Emulsion stabilizer | Core particles | Supported material/ Production method | Particle structure | Collection | Dispersibility | Feeling in use | Moisturizing properties | Colorability determination/ Color |
|---|---|---|---|---|---|---|---|---|---|
| Comparative Example 25 | PVA | PLA | Spherical silver fine particles/I | Poor | Good | Poor | Poor | Poor | Poor/Yellow |
| Comparative Example 26 | CMC | DVB polymer | Spherical silver fine particles/II | Poor | Good | Poor | Poor | Poor | Poor/Yellow |
| Comparative Example 27 | TEMPO-oxidized CNF | - | Spherical gold fine particles/- | - | Poor | Poor | Poor | Good | Good/Red |
| Comparative Example 28 | TEMPO-oxidized CNF | - | Spherical flat plate-like silver fine particles/- | - | Poor | Poor | Poor | Good | Good/Blue |
| Comparative Example 29 | - | - | - | - | - | Poor | Good | Poor | Poor/Yellow |

The symbol "-" in Table 3 indicates that the evaluation was not performed or that the evaluation was unable to be completed and was not performed.

As is clear from the evaluation results of Examples 21 to 30 shown in Table 3, it was confirmed that by using the production method III or the production method IV, regardless of the type of fine fibers (TEMPO-oxidized CNF, carboxymethylated CNF, phosphoric acid esterified CNF, chitin NF), personal care particles including core particles containing a polymer of various monomers or a biodegradable polymers were produced. Furthermore, it was confirmed that personal care particles supporting silver or gold fine particles having various shapes or a dye were produced.

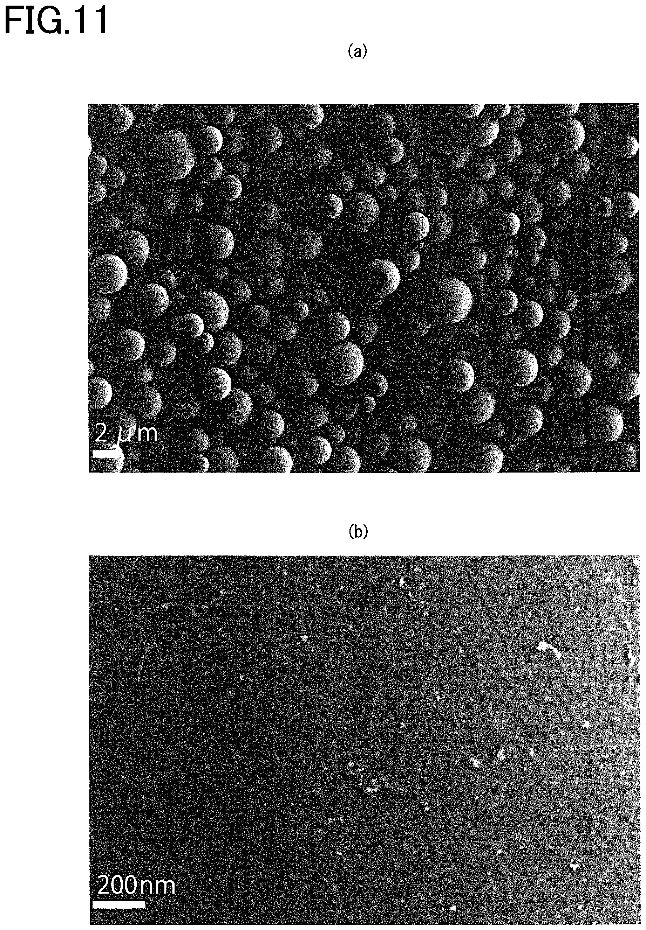

As shown in Fig. 9, it was confirmed that in Example 21, spherical silver fine particles were supported on the surface of the cellulose nanofiber coated particles. As shown in Fig. 10, it was confirmed that in Example 22, flat plate-like fine particles were supported on the surface of the cellulose nanofiber coated particles. As shown in Fig. 11, it was confirmed that in Example 29, spherical gold fine particles were supported on the surface of the cellulose nanofiber coated particles.

When the personal care particles (dry powder) were observed, the personal care particles exhibited optical characteristics derived from the LSPR of various metal fine particles, and had a yellow color in Example 21 and Examples 23 to 28, a blue color in Example 22, and a red color in Example 29. In Example 30, the personal care particles had a red color derived from rhodamine B.

The personal care particles obtained in Examples 21 to 30 were able to be washed by filtration, and even after drying, the personal care particles had good redispersibility, and exhibited good skin compatibility, good moisturizing properties, and uniform colorability.

On the other hand, in Comparative Examples 21, 27, and 28, a dispersion liquid of metal fine particle-supporting cellulose nanofibers was produced by causing cellulose nanofibers to support metal fine particles of various shapes and metals.

In Comparative Examples 21, 27, and 28, centrifugation washing of the obtained metal fine particle-supporting cellulose nanofiber dispersion liquid was attempted but failed. Thus, the obtained dispersion liquid was freeze-dried and freeze-ground to obtain a powder, and the powder was evaluated in the same manner. The results showed that the obtained powder was good in moisturizing properties and colorability, but poor in redispersibility and skin compatibility.

In Comparative Examples 22 and 25, the second-iv step was unable to be completed. Specifically, when ultrasonic homogenizer treatment was performed, the monomer phase or the dissolved polymer phase and the cellulose nanofiber dispersion liquid phase remained in the two-phase separated state, or even when an emulsion was formed, the emulsion immediately collapsed; thus, no stable O/W emulsion was produced.

In Comparative Examples 23, 24, and 26, an O/W emulsion was formed in the second-iv step. This is presumably because CMC and PGLE ML10 had amphipathic properties as with the cellulose nanofibers and thus functioned as an emulsion stabilizer. However, when the core material was solidified by polymerizing the polymerizable monomer or removing the solvent from the dissolved polymer in the subsequent third-ii step, the emulsion collapsed, and personal care particles to be produced using the O/W emulsion as a template were not obtained. The reason for this is not clear, but CMC and PGLE ML10 were water soluble and were thus highly likely to be fragile as a coating layer for maintaining the emulsion form during solidification of the core material. This is presumably the reason why the emulsion collapsed during solidification of the core material.

In Comparative Examples 22 to 26, samples were collected by filtration washing. However, in the evaluation of redispersibility of the dry powders of all Comparative Examples 22 to 26, aggregates were visually observed, and the dry powders had poor dispersibility.

In Comparative Examples 22 to 26, the results of the evaluation of feeling in use, moisturizing properties, and colorability of the dry powders showed that the dry powders had poor skin compatibility and a small moisture retention amount, and non-uniform coloration was visually observed. Presumably, since the dry powders of Comparative Examples 22 to 26 contained no cellulose nanofibers, due to the lack of the moisturizing effect and the dispersion effect of the cellulose nanofibers, the dry powders were poor in feeling in use and colorability.

The dry powder of Comparative Example 29 had a good feeling in use, but was poor in dispersibility in water, moisturizing properties, and colorability.

[Industrial Applicability]

The composite particles according to the first aspect of the present invention can be used for coating materials, coating material additives such as matting agents, films such as light diffusion films, additives to molded products such as automobiles and construction materials, paper such as recording paper, personal care products such as cosmetics, toiletry products, home care products, medical care products, and the like.

When the composite particles according to the first aspect of the present invention are applied to personal care products, the composite particles can be used, for example, for products related to hair care, oral care, odor care, body care, skin care, and makeup. Specific examples of such products include toothpastes, perfumes, nail lacquers, beauty essences, skin lotions, milky lotions, cleansing agents, eye shadows, eyeliners, foundations, blushes, hair gels, hair sprays, shampoos, conditioners, hair tonics, lotions, creams, and soaps.

The fourth aspect of the present invention provides personal care particles having fine fibers, which are in a novel form that can be produced by a simple method, and solve the problems of the fine fibers, such as cellulose nanofibers or chitin nanofibers, having excessive solvent.

Furthermore, the fourth aspect of the present invention provides personal care particles having good texture and good moisturizing properties, due to being coated with fine fibers having high safety, high hydrophilicity, and a high specific surface area.

Furthermore, the fourth aspect of the present invention provides personal care particles that due to a functional material being supported by fine fibers having a high specific surface area, maintain dispersion stability of the functional material, and effectively exhibit a function of the functional material such as ultraviolet light shielding (absorption and/or scattering), color development, an antibacterial effect, or deodorization.

Fine fibers such as cellulose nanofibers and chitin nanofibers are respectively made of cellulose and chitin/chitosan which are biodegradable polymers. Thus, the use of a material containing a biodegradable polymer to form core particles provides personal care particles that reduce the environmental load and solve the problem of microplastics.

The personal care particles of the fourth aspect can be contained in personal care articles and personal care compositions. Thus, the personal care particles of the fourth aspect can be used, for example, for products related to hair care, oral care, odor care, body care, skin care, and makeup. Specific examples of such products include toothpastes, perfumes, nail lacquers, beauty essences, skin lotions, milky lotions, cleansing agents, eye shadows, eyeliners, foundations, blushes, hair gels, hair sprays, shampoos, conditioners, hair tonics, lotions, creams, and soaps.

[Reference Signs List]

1 ... Composite particle
1A ... Personal care particle
2A ... Core particle
2 ... Core particle
3 ... Fine fiber (fine fiber which is at least either cellulose nanofiber or chitin nanofiber) 3A ... Fine fiber supporting no material
3B ... Fine fiber supporting functional material
4 ... Pore
6 ... Droplet containing core material and pore forming agent (droplet of W/O emulsion liquid, hydrophobic droplet)
6A ... Droplet containing core material
7 ... Hydrophilic solvent
8 ... Stabilizer
9 ... Droplet containing pore forming agent (hydrophilic droplet)
10 ... Particle (containing pore forming agent) coated with fine fibers
10A ... Particle (containing no pore forming agent) coated with fine fibers
11A ... Dispersion liquid of fine fibers supporting no material
11B ... Dispersion liquid of fine fibers supporting functional material
12 ... Dispersion liquid of core particles that contain pore forming agent and whose surface is coated with fine fibers
12A ... Dispersion liquid of core particles (containing no pore forming agent) whose surface is coated with fine fibers
13 ... W/O emulsion liquid
14 ... W/O/W emulsion liquid
40 ... Functional material

**Claims**

1. A composite particle comprising:

   a core particle having a pore; and
   fine fibers made of at least either cellulose nanofibers or chitin nanofibers, the fine fibers being inseparably bonded to a surface of the core particle.

2. The composite particle according to claim 1, wherein the core particle is made of a material containing a polymer.

3. The composite particle according to claim 2, wherein the polymer includes a biodegradable polymer.

4. The composite particle according to any one of claims 1 to 3, wherein the core particle is a porous particle or a hollow particle.

5. The composite particle according to any one of claims 1 to 4, wherein the composite particle has a light diffusion index of 0.6 or more.

6. The composite particle according to any one of claims 1 to 5, wherein the pore contains at least one of an ultraviolet absorber, an ultraviolet scattering agent, a perfume, a deodorizing component, a skin-whitening component, an anti-inflammatory component, a peeling agent, and a colorant.

7. The composite particle according to any one of claims 1 to 6, wherein the fine fibers have an ionic functional group.

8. A method of producing a composite particle, the method comprising:

   fibrillating one or both of a cellulose raw material and a chitin/chitosan raw material in a solvent to obtain a dispersion liquid of fine fibers that are at least either cellulose nanofibers or chitin nanofibers;
   dispersing a droplet containing a pore forming agent and a core material in the dispersion liquid, and coating a surface of the droplet with the fine fibers, the pore forming agent forming a pore when removed, the core material forming a core particle when solidified;
   solidifying the core material in the droplet to obtain a dispersion liquid of a core particle that contains the pore forming agent and whose surface is coated with the fine fibers; and
   collecting the core particle from the dispersion liquid of the core particle, and removing the pore forming agent from the collected core particle to form a pore in the core particle.

9. A method of producing a composite particle, the method comprising:

   fibrillating one or both of a cellulose raw material and a chitin/chitosan raw material in a solvent to obtain a dispersion liquid of fine fibers that are at least either cellulose nanofibers or chitin nanofibers;
   producing a W/O emulsion liquid in which a droplet containing a pore forming agent that forms a pore when removed is dispersed in a core material that forms a core particle when solidified, dispersing a droplet containing the W/O emulsion liquid in the dispersion liquid of the fine fibers, and coating a surface of the droplet containing the W/O emulsion liquid with the fine fibers to produce a W/O/W emulsion liquid;
   solidifying the core material in a droplet of the W/O/W emulsion liquid to obtain a dispersion liquid of a core particle that contains the droplet containing the pore forming agent and whose surface is coated with the fine fibers; and
   collecting the core particle from the dispersion liquid of the core particle, and removing the pore forming agent from the collected core particle to form a pore in the core particle.

10. The production method according to claim 8 or 9, wherein the core material is at least one of a polymerizable monomer, a dissolved polymer, and a molten polymer.

11. A personal care product comprising the composite particle according to any one of claims 1 to 7.

12. A personal care particle comprising:

   a core particle made of a material containing a polymer;
   fine fibers made of at least either cellulose nanofibers or chitin nanofibers, the fine fibers being inseparably bonded to a surface of the core particle; and
   a functional material that is supported by the fine fibers and exhibits a personal care function.

13. The personal care particle according to claim 12, wherein the functional material is at least one of an ultraviolet absorber, an ultraviolet scattering agent, a perfume, a deodorizing component, a skin-whitening component, an anti-inflammatory component, a peeling agent, and a colorant.

14. The personal care particle according to claim 13, wherein the colorant is at least one of an organic synthetic colorant, a natural colorant, an inorganic pigment, and metal fine particles.

**15.** The personal care particle according to claim 14, wherein the metal fine particles are made of a material containing at least one of Ru, Fe, Co, Rh, Ni, Pd, Pt, Cu, Ag, Au, and Zn.

**16.** The personal care particle according to claim 14 or 15, wherein the metal fine particles have an average particle size of 2 nm or more and 1000 nm or less, and have a flat plate shape with an average thickness of 1 nm or more and 100 nm or less.

**17.** The personal care particle according to any one of claims 12 to 16, wherein the cellulose nanofibers or the chitin nanofibers have an ionic functional group.

**18.** The personal care particle according to any one of claims 12 to 17, wherein the polymer includes a biodegradable polymer.

**19.** A method of producing a personal care particle, the method comprising:

fibrillating one or both of a cellulose raw material and a chitin/chitosan raw material in a solvent to obtain a dispersion liquid of fine fibers that are at least either cellulose nanofibers or chitin nanofibers;
causing the fine fibers in the dispersion liquid of the fine fibers to support a functional material that exhibits a personal care function, thereby obtaining a dispersion liquid of the fine fibers supporting the functional material;
dispersing a droplet containing a core material in the dispersion liquid of the fine fibers supporting the functional material, and coating a surface of the droplet with the fine fibers supporting the functional material; and
solidifying the core material in the droplet coated with the fine fibers supporting the functional material.

**20.** A method of producing a personal care particle, the method comprising:

fibrillating one or both of a cellulose raw material and a chitin/chitosan raw material in a solvent to obtain a dispersion liquid of fine fibers that are at least either cellulose nanofibers or chitin nanofibers;
dispersing a droplet containing a core material in the dispersion liquid, and coating a surface of the droplet with the fine fibers;
solidifying the core material in the droplet to obtain a dispersion liquid of a particle in which a surface of a core particle is coated with the fine fibers; and
causing, in the dispersion liquid of the particle coated with the fine fibers, the fine fibers on the surface of the particle coated with the fine fibers to support a functional material that exhibits a personal care function.

**21.** The production method according to claim 19 or 20, wherein the core material is at least one of a polymerizable monomer, a dissolved polymer, and a molten polymer.

**22.** A personal care article comprising the personal care particle according to any one of claims 12 to 18.

**23.** A personal care composition comprising the personal care particle according to any one of claims 12 to 18.

FIG.1A

FIG.1B

FIG.1C

# FIG.2

(a)  (b)  (c)

# FIG.3

(a)

(c)

(e)

(b)

(d)

EP 3 981 796 A1

FIG.4

FIG.5

(a)       (b)       (c)       (d)

# FIG.6

(a)  (b)  (c)  (d)

## FIG.7

## FIG.8

# FIG.9

(a)

(b)

# FIG.10

(a)

(b)

# FIG.11

(a)

2 μm

(b)

200nm

# INTERNATIONAL SEARCH REPORT

| International application No. |
| --- |
| PCT/JP2020/021982 |

## A. CLASSIFICATION OF SUBJECT MATTER

C08E 15/02(2006.01)i; A61Q 1/00(2006.01)i; A61Q 3/00(2006.01)i; A61Q 11/00(2006.01)i; A61Q 15/00(2006.01)i; A61Q 19/00(2006.01)i; A61Q 5/00(2006.01)i; A61K 8/06(2006.01)i; A61K 8/73(2006.01)i; A61K 8/81(2006.01)i; A61K 8/85(2006.01)i; C08E 37/08(2006.01)i
FI:      A61K8/73; A61K8/85; A61Q19/00; A61Q1/00; C08B37/08 A; A61K8/81; A61Q5/00; A61Q11/OO; A61Q15/00; A61Q3/00; A61K8/06; C08B15/02

According to International Patent Classification (IPC) or to both national classification and IPC

## B. FIELDS SEARCHED

Minimum documentation searched (classification system followed by classification symbols)
A61K8/0-8/99; A61Q1/00-90/00; C08B15/02; C08B37/08

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

## C. DOCUMENTS CONSIDERED TO BE RELEVANT

| Category* | Citation of document, with indication, where appropriate, of the relevant passages | Relevant to claim No. |
| --- | --- | --- |
| X | JP 2017-186187 A (JGC CATALYSTS AND CHEMICALS LTD.) 12.10.2017 (2017-10-12) claims, paragraphs [0016], [0046]-[0051], [0069]-[0073] | 1, 4, 6-7, 11 |
| P, Y | WO 2019/135384 A1 (TOPPAN PRINTING CO., LTD.) 11.07.2019 (2019-07-11) paragraphs [0014], [0032]-[0097], [0276], fig. 1 | 1-11 |
| Y | JP 2018-140962 A (ENEX CO., LTD.) 13.09.2018 (2018-09-13) claims, paragraphs [0012], [0038]-[0041] | 1-11 |
| Y | WO 1999/036470 A1 (QUEST INTERNATIONAL B. V.) 22.07.1999 (1999-07-22) column 3, lines 10-18, column 7, lines 26-32 | 1-11 |

☒ Further documents are listed in the continuation of Box C.      ☒ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 25 June 2020 (25.06.2020) | 18 August 2020 (18.08.2020) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2020/021982

C (Continuation).   DOCUMENTS CONSIDERED TO BE RELEVANT

| Category* | Citation of document, with indication, where appropriate, of the relevant passages | Relevant to claim No. |
|---|---|---|
| P, Y | WO 2019/151486 A1 (NISSAN CHEMICAL CORPORATION) 08.08.2019 (2019-08-08) paragraphs [0086]-[0090] | 5 |
| Y | JP 2005-281470 A (SEKISUI PLASTICS CO., LTD.) 13.10.2005 (2005-10-13) paragraph [0004] | 8-10 |
| Y | JP 2017-192897 A (HITACHI CHEMICAL CO., LTD.) 26.10.2017 (2017-10-26) paragraphs [0021]-[0023] | 8-10 |
| Y | WO 2017/056908 A1 (SEKISUI PLASTICS CO., LTD.) 06.04.2017 (2017-04-06) paragraphs [0037]-[0045] | 3, 5 |

Form PCT/ISA/210 (continuation of second sheet) (January 2015)

73

**INTERNATIONAL SEARCH REPORT**

International application No.

PCT/JP2020/021982

**Box No. II**      **Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III**      **Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.: 1-11

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

## INTERNATIONAL SEARCH REPORT
Information on patent family members

| International application No. |
| --- |
| PCT/JP2020/021982 |

| Patent Documents referred in the Report | Publication Date | Patent Family | Publication Date |
| --- | --- | --- | --- |
| JP 2017-186187 A | 12 Oct. 2017 | (Family: none) | |
| WO 2019/135384 A1 | 11 Jul. 2019 | (Family: none) | |
| JP 2018-140962 A | 13 Sep. 2018 | (Family: none) | |
| WO 1999/036470 A1 | 22 Jul. 1999 | EP 930334 A1 | |
| WO 2019/151486 A1 | 08 Aug. 2019 | (Family: none) | |
| JP 2005-281470 A | 13 Oct. 2005 | (Family: none) | |
| JP 2017-192897 A | 26 Oct. 2017 | (Family: none) | |
| WO 2017/056908 A1 | 06 Apr. 2017 | US 2018/0265663 A1 paragraphs [0078]-[0093] EP 3357955 A1 CN 108137822 A KR 10-2018-0063076 A | |

Form PCT/ISA/210 (patent family annex) (January 2015)

**INTERNATIONAL SEARCH REPORT**

International application No.

PCT/JP2020/021982

<Continuation of Box No. III>

Document 1: JP 2017-186187 A (JGC CATALYSTS AND CHEMICALS LTD.) 12.10.2017 (2017-10-12) claims, paragraphs [0016], [0046]-[0051] (Family: none)

Claims are classified into the two inventions below.
(Invention 1) Claims 1-11
Claims, paragraphs [0016] and [0046]-[0051] of document 1 disclose porous silica particles containing cellulose nanofibers, and also set forth that cleansing is repeatedly performed with pure water when a cake-type material is obtained by adding, in a slurry obtained by mixing a silica sol and a silicic acid solution, a dispersion solution of cellulose nanofibers. Thus, it can be said that the cellulose nanofibers are present at the surfaces of the porous silica particles and are bonded with a strength in which the cellulose nanofibers are not missed by cleansing with pure water. Therefore, document 1 is recognized to disclose "composite particles having core particles with pores and microfibers which are formed of at least one of cellulose nanofibers or chitin nanofibers and are inseparably bonded to the surfaces of the core particles." However, claim 2 dependent on claim 1 has the special technical feature in that the "core particles are formed of a material containing a polymer," and claims 4-7 also have the same special technical feature as claim 2. Thus, claims 1-7 are classified as invention 1.
Claims 8-11 are not dependent on claim 1 and are inventively associated with claim 1, and are thus classified as invention 1.

(Invention 2) Claims 12-23
Claim 12 cannot be said to have a special technical feature identical or corresponding to that of claim 2 classified as invention 1.
In addition, claims 12-23 are not dependent on claim 1. In addition, claims 12-23 are not substantially identical or equivalent to any of the claims classified as invention 1.
Therefore, claims 12-23 cannot be classified as invention 1, and are thus classified as invention 2.

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010216021 A
- WO 2014088072 A
- JP 2008001728 A
- WO 2013042654 A
- WO 2010095574 A
- JP 2010180309 A

**Non-patent literature cited in the description**

- **NOGUCHI Y ; HOMMA I ; MATSUBARA Y.** Complete nanofibrillation of cellulose prepared by phosphorylation. *Cellulose,* 2017, vol. 24, 1295